# EUROPEAN PATENT APPLICATION

(11) **EP 2 505 655 A2**
(43) Date of publication of application: **03.10.2012**
(21) Application number: 12173782.9
(22) Date of filing: 21.11.2007
(51) Int. Cl.: C12P 7/10, C12N 9/24, C12R 1/645

(54) **Recombinat yeast strains expressing tethered cellulase enzymes**

(30) Priority: 22.11.2006 US 867018 P
(62) Divisional of application: 07871554.7
(71) Applicant: The Trustees of Dartmouth College, Hanover, NH 03755-1404 (US)
(72) Inventor: McBride, John E.E., Hanover, NH 03755 (US); Deleault, Kristen M., Canaan, NH 03741 (US); Lynd, Lee R., Meriden, NH 03770 (US); Pronk, Jack T., 2636 DD Schipluiden (NL)
(74) Representative: Kudlek & Grunert Patentanwälte

(57) **Abstract**

Recombinant yeast strains that saccharify, ferment and grow on insoluble and crystalline forms of cellulose are disclosed herein. The yeast strains express tethered cellulases including cellobiohydrolase, endoglucanase and β-glucosidase. The recombinant organisms are particularly suited for consolidated bioprocessing.

## Description

### RELATED APPLICATIONS

This application claims the benefit of priority to U.S. Provisional Patent Application Serial No. 60/867,018, filed November 22, 2006, which is hereby incorporated by reference in its entirety.

### GOVERNMENT RIGHTS

The U.S. government has certain rights in this invention as provided for by the terms of Grant No. 60NANB1D0064, awarded by the National Institute of Standards and Technology.

### BACKGROUND

### 1. FIELD OF THE INVENTION

The present invention pertains to the field of biomass processing to produce ethanol and other products. In particular, recombinant organisms that hydrolyze, ferment and grow on soluble and insoluble cellulose are disclosed, as well as methods for the production and use of the organisms.

### 2. DESCRIPTION OF THE RELATED ART

Biomass represents an inexpensive and readily available cellulosic feedstock from which sugars may be produced. These sugars may be recovered or fermented to produce alcohols and/or other products. Among bioconversion products, interest in ethanol is high because it may be used as a renewable domestic fuel.

Significant research has been performed in the areas of reactor design, pretreatment protocols and separation technologies, so that bioconversion processes are becoming economically competitive with petroleum fuel technologies. Further, it has been observed that large cost savings may be obtained when two or more process steps are combined. For example, simultaneous saccharification and fermentation (SSF) and simultaneous saccharification and co-fermentation (SSCF) processes combine enzymatically-mediated saccharification with fermentation in a single reactor or continuous process apparatus.

In addition to savings associated with shorter reaction times and reduced capital costs, co-fermentation processes may also provide improved product yields because certain compounds that would otherwise accrue at levels that inhibit metabolysis or hydrolysis are consumed by the co-fermenting organisms. In one such example, β-glucosidase ceases to hydrolyze cellobiose in the presence of glucose and, in turn, the build-up of cellobiose impedes cellulose degradation. An SSCF process involving co-fermentation of cellulose and hemicellulose hydrolysis products may alleviate this problem by converting the glucose into one or more products that do not inhibit the hydrolytic activity of β-glucosidase.

The ultimate combination of biomass processing steps is referred to as consolidated bioprocessing (CBP). CBP involves four biologically-mediated events: (1) enzyme production, (2) substrate hydrolysis, (3) hexose fermentation and (4) pentose fermentation. These events may be performed in a single step by a microorganism that degrades and utilizes both cellulose and hemicellulose. Development of CBP organisms could potentially result in very large cost reductions as compared to the more conventional approach of producing saccharolytic enzymes in a dedicated process step. CBP processes that utilize more than one organism to accomplish the four biologically-mediated events are referred to as consolidated bioprocessing co-culture fermentations.

### Consolidated Bioprocessing Organisms

Numerous attempts have been made to create recombinant organisms for CBP. For example, various cellulase genes have been expressed in *Saccharomyces cerevisiae* with the aim of direct ethanol production from cellulose. While short-lived fermentations have been observed using recombinant organisms, sustainable growth of the organisms on cellulose has not been achieved. This is, at least, partially due to the fact that heterologous cellulase enzymes are usually produced by recombinant organisms in such low concentrations that the amount of saccharified substrate available is unable to sustain growth of the organisms. This concentration deficiency is exacerbated when enzymes are secreted into media, where they are further diluted.

In an attempt to alleviate enzyme concentration deficiencies, yeast strains displaying cell surface proteins have recently been developed. Fujita, Y.; Takahashi, S.; Ueda, M.; Tanaka, A.; Okada, H.; Morikawa, Y.; Kawaguchi, T.; Arai, M.; Fukuda, H.; Kondo, A. "Direct and Efficient Production of Ethanol from Cellulosic Material with a Yeast Strain Displaying Cellulolytic Enzymes" Applied and Environmental Microbiology, 68(1), 5136-5141, (2002) describes an *S*. *cerevisiae* strain expressing tethered β-glucosidase I (BGLI) and endoglucanase II (EGII). The strain is able to grow on barley β-glucan, which is a linear, soluble polysaccharide. To date, however, there have been no reports of yeast strains expressing cell-surface tethered enzymes that are able to grow on insoluble cellulose, nor have there been reports of any yeast strains able to grow on crystalline cellulose.

As reported by Fan et al. in PCT/US05/018430, expression of cell-surface tethered enzymes may provide an advantage for cell growth, where saccharified substrate is unable to diffuse away from the cell before being metabolized. Further, a portion of a population of cells expressing tethered enzymes may exhibit enhanced expression of the one or more tethered enzymes relative to the overall population. This portion may exhibit enhanced binding to the substrate and improved growth characteristics. As such, observation of these traits may be a useful criteria for organism selection.

### SUMMARY

The present instrumentalities advance the art and overcome the problems outlined above by providing recombinant yeast strains that express tethered cellulase enzymes and have the ability to saccharify insoluble cellulose. Methods for using the recombinant organisms to produce ethanol are also disclosed.

In an embodiment, a transformed yeast cell expresses a plurality of genes, wherein the genes code for expression of tethered enzymes including endoglucanase, cellobiohydrolase and β-glucosidase.

In an embodiment, a transformed organism includes a yeast that in a native state lacks the ability to saccharify cellulose, wherein the yeast is transformed with heterologous polynucleotides that express a plurality of enzymes that confer upon the yeast the ability to saccharify crystalline cellulose.

In an embodiment, an isolated polynucleotide includes (a) a polynucleotide sequence of SEQ ID NO: 11; (b) a polynucleotide sequence of SEQ ID NO: 12; (c) a polynucleotide sequence of SEQ ID NO: 28; (d) a polynucleotide sequence of SEQ ID NO: 29; and (e) a polynucleotide sequence of SEQ ID NO: 30; or (f) a polynucleotide sequence having at least about 90% sequence identity with the polynucleotide sequences of (a)-(e).

A yeast host according to any of the aforementioned embodiments may be utilized in a method for producing ethanol, which includes producing a transformed yeast host and culturing the transformed yeast host in medium that contains cellulose under suitable conditions for a period sufficient to allow saccharification and fermentation of the cellulose to ethanol.

A yeast host according to any of the aforementioned embodiments may be utilized in a method for selecting a transformed yeast cell with enhanced binding affinity for insoluble cellulose. The method includes producing a transformed yeast host, culturing the transformed yeast host under suitable conditions for a period sufficient to allow growth and replication of the transformed yeast host, exposing a sample of transformed yeast host from the culture to the insoluble cellulose and selecting the sample of transformed yeast host that provides at least a two fold reduction in supernatant optical density relative to a similarly cultured and exposed sample of the native organism.

### BRIEF DESCRIPTION OF THE DRAWINGS

FIG. 1 is a schematic of an exemplary δ-integration vector having two cellulase enzymes and a kanamycin marker.

FIG. 2 shows a comparison of recombinant Y294 and CEN.PK yeast transformed to express β-glucosidase I, endoglucanase I, cellobiohydrolase I and cellobiohydrolase II enzymes and untransformed Y294 and CEN.PK yeast growth on phosphoric acid swollen cellulose (PASC), according to an embodiment.

FIG. 3 shows a comparison of recombinant CEN.PK yeast transformed to express β-glucosidase I, endoglucanase I, cellobiohydrolase I and cellobiohydrolase II enzymes and untransformed CEN.PK yeast growth on bacterial microcrystalline cellulose (BMCC), according to an embodiment.

FIG. 4 shows a comparison of recombinant Y294 yeast transformed to express β-glucosidase I and endoglucanase I enzymes; Y294 yeast transformed to express β-glucosidase I, endoglucanase I, cellobiohydrolase I and cellobiohydrolase II enzymes and untransformed Y294 yeast growth on bacterial microcrystalline cellulose (BMCC), according to an embodiment.

FIG. 5 shows a comparison of recombinant yeast transformed to express β-glucosidase I and endoglucanase I enzymes and untransformed yeast cell binding on cellulose particles, according to an embodiment.

FIG. 6 shows cell concentration and viable cell counts for semi-continuous cultures of transformed and untransformed strains of CEN.PK growing on Avicell as a carbon source, according to an embodiment.

### DETAILED DESCRIPTION

There will now be shown and described methods for engineering and utilizing recombinant yeast in the conversion of biomass to ethanol. The disclosed yeast strains express tethered cellulase enzymes, which impart upon the yeast an ability to grow on insoluble non-crystalline and crystalline forms of cellulose.

As used herein, an organism is in "a native state" if it is has not been genetically engineered or otherwise manipulated by the hand of man in a manner that intentionally alters the genetic and/or phenotypic constitution of the organism. For example, wild-type organisms may be considered to be in a native state.

As used herein, a protein is "tethered" to an organism's cell surface if at least one terminus of the protein is covalently and/or electrostatically bound to the cell membrane, or cell wall. It will be appreciated that a tethered protein may include one or more enzymatic regions that may be joined to one or more other types of regions (e.g., a promoter, a terminator, an anchoring domain, a linker, a signaling region, etc.). While the one or more enzymatic regions may not be directly bound to the cell membrane (e.g., such as when binding occurs via an anchoring domain), this protein may nonetheless be considered a "tethered enzyme" according to the present specification.

Tethering may, for example, be accomplished by incorporation of an anchoring domain into a recombinant protein that is heterologously expressed by a cell, e.g., a fatty acid linkage, glycosyl phosphatidyl inositol anchor or other suitable molecular anchor which may bind the tethered protein to the cell membrane of the host cell. In addition, tethering may be accomplished by prenylation, which is the attachment of a hydrophobic chain to a protein to faciliate interaction between the modified protein and the hydrophobic region of the lipid bilayer.

Although the results reported herein are for *Saccharomyces cerevisiae,* the methods and materials also apply to other types of yeast including *Schizosaccharomyces pombe, Candida albicans, Kluyveromyces lactis, Pichia pastoris, Pichia stipitis, Yarrowia lipolytica, Hansenula polymorpha, Phaffia rhodozyma, Candida utilis, Arxula adeninivorans, Debaryomyces hansenii, Debaryomyces polymorphus, Kluyveromyces marxianus, Issatchenkia orientalis* and *Schwanniomyces occidentalis.* The disclosed methods and materials are useful generally in the field of engineered yeast.

The disclosed recombinant yeast strains have the potential to contribute significant savings in the lignocellulosic biomass to ethanol conversion. For example, recombinant yeast strains may be suitable for a consolidated bioprocessing co-culture fermentation where they would convert cellulose to ethanol, and hemicellulose would be degraded by a pentose-utilizing organism, such as *Saccharomyces cerevisiae* RWB218, disclosed by Kuyper, M.; Hartog, M.M.P.; Toirkens, M.J.; Almering, M.J.H.; Winkler, A.A.; van Dijken, J.P.; Pronk, J.T. "Metabolic engineering of a xylose-isomerase-expressing Saccharomyces cerevisiae strain for rapid anaerobic xylose fermentation", FEMS Yeast Research, 5: 399-409, (2005).

It will be appreciated that suitable lignocellulosic material may be any feedstock that contains soluble or insoluble cellulose, where the insoluble cellulose may be in a crystalline or non-crystalline form. In various embodiments, the lignocellulosic biomass comprises wood, corn, corn stover, sawdust, bark, leaves, agricultural and forestry residues, grasses such as switchgrass, ruminant digestion products, municipal wastes, paper mill effluent, newspaper, cardboard or combinations thereof.

In some embodiments, endoglucanase, cellobiohydrolase and β-glucosidase can be any suitable endoglucanase, cellobiohydrolase and/or β-glucosidase derived from, for example, a fungal or bacterial source.

In certain embodiments, endoglucanase(s) can be an endoglucanase I and/or an endoglucanase II isoform, paralogue or orthologue. In another embodiment, endoglucanase expressed by the host cells can be recombinant endo-1,4-β-glucanase. In some embodiments, endoglucanase is an endoglucanase I from *Trichoderma reesei.* In another embodiment, endoglucanase is encoded by the polynucleotide sequence of SEQ ID NO: 28.

In certain embodiments, β-glucosidase is derived from *Saccharomycopsis fibuligera.* In some embodiments, β-glucosidase can be a β-glucosidase I and/or a β-glucosidase II isoform, paralogue or orthologue. In another embodiment, β-glucosidase expressed by the host cells can be recombinant β-glucanase I from a *Saccharomycopsis fibuligera* source.

In certain embodiments, cellobiohydrolase(s) can be a cellobiohydrolase I and/or a cellobiohydrolase II isoform, paralogue or orthologue. In some embodiments, cellobiohydrolases are cellobiohydrolase I and/or cellobiohydrolase II from *Trichoderma reesei.* In another embodiment, cellobiohydrolases are encoded by the polynucleotide sequences of SEQ ID NOS: 29 and/or 30.

Cellulase catalytic domain genes that are suitable for use in the disclosed recombinant organisms include, for example, those shown in Table 1. Cellulase genes suitable for incorporation into yeast according to the present instrumentalities (e.g., BGLI, EGI, CBHI, CBHII, Endo-1, EG19, glycoside hydrolase, Cel3AC, gghA and BGLA) may be synthesized or isolated from various organisms. Such cellulase genes, and methods for synthesizing and/or isolating the genes, are known in the art. For example, many cellulase catalytic domains can be located in the online ExPASy database (http://www.expasy.org/) under E.C. # 3.2.1.4 (endo-1,4, beta-D-glucanase), E.C.# 3.2.1.91 (cellulose 1,4-beta-cellobiosidase) and E.C.# 3.2.1.21 (beta-glucosidase) [retrieved November 14, 2007]. Retrieved from the Internet: <URL: www.expasy.org/>.

**Table 1. Cellulase Catalytic Domains**

| **Name** | **Cellulase Catalytic Domain Amino Acid Sequence** | **Originating Organism** | **SEQ ID NO** |
|---|---|---|---|
| BGLI | mvsftsllagvaaisgvlaapaaevepvavekrea eaeamlmivqllvfalglavavpiqnytqspsqrd essqwvsphyyptpqggrlqdvwqeayarakai vgqmtivekvnlttgtgwqldpcvgntgsvprfgi pnlclqdgplgvrfadfvtgypsglatgatfnkdlfl qrgqalghefnskgvhialgpavgplgvkarggrn feafgsdpylqgtaaaatikglqennvmacvkhfi gneqekyrqpddinpatnqttkeaisanipdram halylwpfadsvragvgsvmcsynrvnntyace nsymmnhllkeelgfqgfvvsdwgaqlsgvysa isgldmsmpgevyggwntgtsfwgqnltkaiyn etvpierlddmatrilaalyatnsfptedhlpnfssw ttkeygnkyyadntteivkvnynvdpsndftedta lkvaeesivllknenntlpispekakrlllsgiaagp | *Saccharomycopsis fibuliga* | 36 |
| | dpigyqcedqsctngalfqgwgsgsvgspkyqvt pfeeisylarknkmqfdyiresydlaqvtkvasda hlsivvvsaasgegyitvdgnqgdrknltlwnngd klietvaencantvvvvtstgqinfegfadhpnvta ivwagplgdrsgtaianilfgkanpsghlpftiaktd ddyipietyspssgepednhlvendllvdyryfee kniepryafgyglsyneyevsnakvsaakkvdee lpepatylsefsyqnakdsknpsdafapadlnrvn eylypyldsnvtlkdgnyeypdgysteqrttpnqp ggglggndalwevaynstdkfvpqgnstdkfvp qlylkhpedgkfetpiqlrgfekvelspgekktvdl rllrrdlsvwdttrqswivesgtyealigvavndik- tsvlfti | | |
| EGI | mnifyiflfllsfvqgslnctlrdsqqkslvmsgpye lkasldkreaeaeaqqpgtstpevhpklttykctks ggcvaqdtsvvldwnyrwmhdanynsctvngg vnttlcpdeatcgkncfiegvdyaasgvttsgsslt mnqympsssggyssvsprlylldsdgeyvmlkl ngqelsfdvdlsalpcgengslylsqmdenggan qyntaganygsgycdaqcpvqtwrngtlntshqg fccnemdilegnsranaltphsctatacdsagcgfn pygsgyksyygpgdtvdtsktftiitqfntdngsps gnlvsitrkyqqngvdipsaqpggdtisscpsasa ygglatmgkalssgmvlvfsiwndnsqymnwl dsgnagpcsstegnpsnilannpnthvvfsnirwg digsttnstappppppasstfsttrrssttssspsctqth wgqcggigysgcktctsgttcqysndyysqcl | *Trichoderma reesei* | 37 |
| CBHI | mnifyiflfllsfvqgslnctlrdsqqkslvmsgpye lkasldkreaeaeaqsactlqsethppltwqkcssg gtctqqtgsvvidanwrwthatnsstncydgntws stlcpdnetcaknccldgaayastygvttsgnslsig fvtqsaqknvgarlylmasdttyqeftllgnefsfdv dvsqlpcglngalyfvsmdadggvskyptntaga kygtgycdsqcprdlkfingqanvegwepssnna ntgigghgsccsemdiweansisealtphpcttvg qeicegdgcggtysdnryggtcdpdgcdwnpyrl gntsfygpgssftldttkkltvvtqfetsgainryyvq ngvtfqqpnaelgsysgnelnddyctaeeaefggs sfsdkggltqfkkatsggmvlvmslwddyyanm lwldstyptnetsstpgavrgscstssgvpaqvesq spnakvtfsnikfgpigstgnpsggnppggnrgttt trrpatttgsspgptqshygqcggigysgptvcas- gttcqvlnpyysqcl | *Trichoderma reesei* | 38 |
| CBHII | mvsftsllagvaaisgvlaapaaevepvavekrea eaeavpleerqacssvwgqcggqnwsgptccas gstcvysndyysqClpgaassssstraasttsrvspt tsrsssatpppgstttrvppvgsgtatysgnpfvgvt pwanayyasevsslaipsltgamataaAavakvp | *Trichoderma reesei* | 39 |
| | sfmwldtldktplmeqtladirtanknggnyagqf vvydlpdrdcaalasngeysiadggvakyknyid tirqivveYsdirtllviepdslanlvtnlgtpkcana qsaylecinyavtqlnlpnvamyldaghagwlg wpanqdpaaqlfanvyknassprAlrglatnvan yngwnitsppsytqgnavyneklyihaigpllanh gwsnaffitdqgrsgkqptgqqqwgdwcnvigt gfgirpsantgdslldsfvwvkpggecdgtsdssa prfdshcalpdalqpapqagaw- fqayfvqlltnanpsfl | | |
| Endo-1 | mrlvnslgrrkillilavivafstvllfaklwgrktsstl devgskthgdltaenknggylpeeeipdqppatg afnygealqkaiffyecqrsgkldpstlrlnwrgds glddgkdagidltggwydagdhvkfnlpmsysa amlgwavyeyedafkqsgqynhilnnikwacd yfikchpekdvyyyqvgdghadhawwgpaev mpmerpsykvdrsspgstvvaetsaalaiasiifk kvdgeyskeclkhakelfefadttksddgytaang fynswsgfydelswaavwlylatndssyldkaes ysdkwgyepqtnipkykwaqcwddvtygtylll ariknd ngkykeaierhldwwttgyngeritytpk glawldqwgslryatttaflacvysdwengdkek aktylefarsqadyalgstgrsfvvgfgenppkrph hrtahgswadsqmeppehrhvlygalvggpdst dnytddisnytcnevacdynagfvgllakmykly ggspdpkfngieevpedeifveagvnasgnnfiei kaivnnksgwparvcenlsfryfinieeivnagks asdlqvsssynqgaklsdvkhykdniyyvevdls gtkiypggqsaykkevqfrisapegtvfnpendys yqglsagtvvkseyipvydagvlvfgrepgsasks tskdnglskatptvktesqptakhtqnpasdfktpa nqnsvkkdqgikgevvlqyangnagatsnsinpr fkiinngtkainlsdvkiryyytkeggasqnfwcd wssagnsnvtgnffnlsspkegadtclevgfgsga gtldpggsvevqirfskedwsnynqsndys- fkqaclrqrtliylyatwlr | *Clostridium thermocellum* | 40 |
| EG19 | mgsrttisilvvlllglvqlaisghdykqalsksilffe aqrsghlppnqrvswrshsglydgkssgvdlvgg yydagdnvkfglpmaftvttmcwsiieyggqles ngelghaidavkwgtdyfikahpepnvlygevg dgksdhycwqrpeemttdrraykidrnnpgsdla getaaamaaasivfrrsdpsysaellrhahqlfefad kyrgkydssitvaqkyyrsvsgyndellwaaawl yqatndkyyldylgkngdsmggtgwsmtefgw dvkyagvqtlvakvlmqgkggehtavferyqqk aeqfmcsllgkstknikktpgglifrqswnnmqfv tsasflatvysdylsyskrdllcsqgnispsqllefsk sqvdyilgdnpratsymvgygenyprqvhhrgss | *Arabidopsis thaliana* | 41 |
| | ivsfnvdqkfvtcrggyatwfsrkgsdpnvltgalv ggpdaydnfadqrdnyeqtepatynnapllgvlar lisgstgfdqllpgvsptpspviikpapvpqrkptk ppasspspitisqkmtnswknegkvyyrystiltn rstktlkilkisitklygpiwgvtktgnsfsfpswmq slpsgksmefvyihsaspadvlvsnysle | | |
| EGI | mkafhllaalagaavaqqaqlcdqyatytggvyti nnnlwgkdagsgsqcttvnsassagtswstkwn wsggensvksyansgltfnkklvsqisqipttarw sydntgiradvaydlftaadinhvtwsgdyelmi wlaryggvqpigsqiatatvdgqtwelwygangs qktysfvaptpitsfqgdvndffkyltqnhgfpass qylitlqfgtepftggpatlsvsnwsasvq | *Aspergillus aculeauts* | 42 |
| Glycoside hydrolase | mnfrrmlcaaivltivlsimlpstvfaledkspklp dykndllyertfdeglcfpwhtcedsggkcdfavv dvpgepgnkafrltvidkgqnkwsvqmrhrgitl eqghtytvrftiwsdkscrvyakigqmgepytey wnnnwnpfnltpgqkltveqnftmnyptddtcef tfhlggelaagtpyyvylddvslydprfvkpveyv lpqpdvrvnqvgylpfakkyatvvssstsplkwql lnsanqvvlegntipkgldkdsqdyvhwidfsnf ktegkgyyfklptvnsdtnyshpfdisadiyskmk fdalaffyhkrsgipiempyaggeqwtrpaghig vapnkgdtnvptwpqddeyagrpqkyytkdvtg gwydagdhgkyvvnggiavwtlmnmyerakir gianqgaykdggmnipernngypdildearwei effkkmqvtekedpsiagmvhhkihdfrwtalg mlphedpqprylrpvstaatlnfaatlaqsarlwkd ydptfaadclekaeiawqaalkhpdiyaeytpgsg gpgggpynddyvgdefywaacelyvttgkdeyk nylmnsphylempakmgenggangednglwg cftwgttqglgtitlalvenglpsadiqkarnniaka adkwlenieeqgyrlpikqaederggypwgsnsf ilnqmivmgyaydftgnskyldgmqdgmsyll grngldqsyvtgygerplqnphdrfwtpqtskkfp apppgiiaggpnsrfedptitaavkkdtppqkcyi dhtdswstneitinwnapfawvtayldeidlitppg gvdpeepeviygdcngdgkvnstdavalkryilrs gisintdnadvnadgrvnstdlailkryilkeidvl- phk | *Clostridium thermocellum* | 43 |
| Cel3AC | mfkfaallalaslvpgfvqaqspvwgqcggngwt gpttcasgstcvkqndfysqclpnnqappstttqpg ttppatttsggtgptsgagnpytgktvwlspfyade vaqaaadisnpslatkaasvakiptfvwfdtvakv pdlggyladarsknqlvqivvydlpdrdcaalasn gefslandglnkyknyvdqiaaqikqfpdvsvva viepdslanlvtnlnvqkcanaqsaykegviyavq klnavgvtmyidaghagwlgwpanlspaaqlfa | *Agaricus bisporus* | 44 |
| | qiyrdagsprnlrgiatnvanfnalrasspdpitqgn snydeihyiealapmlsnagfpahfivdqgrsgvq nirdqwgdwcnvkgagfgqrpttntgsslidaiv wvkpggecdgtsdnssprfdshcslsdahqpa- peagtwfqayfetlvananpal | | |
| CBHI | mfrtatllaftmaamvfgqqvgtntaenhrtltsqk ctksggcsnlntkivldanwrwlhstsgytncytg nqwdatlcpdgktcaancaldgadytgtygitasg sslklqfvtgsnvgsrvylmaddthyqmfqllnqe ftfdvdmsnlpcglngalylsamdadggmakyp tnkagakygtgycdsqcprdikfingeanvegwn atsanagtgnygtcctemdiweanndaaaytphp cttnaqtrcsgsdctrdtglcdadgcdfnsfrmgdq tflgkgltvdtskpftvvtqfitndgtsagtlteirrlyv qngkviqnssvkipgidpvnsitdnfcsqqktafg dtnyfaqhgglkqvgealrtgmvlalsiwddyaa nmlwldsnyptnkdpstpgvargtcattsgvpaqi eaqspnayvvfsnikfgdlnttytgtvssssvssshs ststssshssssstpptqptgvtvpqwgqcggigyt- gsttcaspytchvlnpyysqcy | *Phanerochaete chrysosporium* | 45 |
| gghA | mkkfpegflwgvatasyqiegspladgagmsiw htfshtpgnvkngdtgdvacdhynrwkedieiie kigakayrfsiswprilpegtgkvnqkgldfynrii dtlleknitpfitiyhwdlpfslqlkggwanrdiad wfaeysrvlfenfgdrvkhwitlnepwvvaivgh lygvhapgmkdiyvafhtvhnllrahaksvkvfr etvkdgkigivfnngyfepasereediraarfmhq fnnyplflnpiyrgeypdlvlefareylprnyedd meeikqeidfvglnyysghmvkydpnsparvsf vernlpktamgweivpegiywilkgvkeeynpq evyitengaafddvvseggkvhdqnridylrahie qvwraiqdgvplkgyfvwslldnfewaegyskrf givyvdyntqkriikdsgywysngiknngltd | *Thermotoga neapolitana* | 46 |
| BGLA | mdmsfpkgflwgaatasyqiegawnedgkgesi wdrfthqkrnilyghngdvacdhyhrfeedvslm kelglkayrfsiawtrifpdgfgtvnqkglefydrli nklvengiepvvtlyhwdlpqklqdiggwanpei vnyyfdyamlvinrykdkvkkwitfnepyciafl gyfhgihapgikdfkvamdvvhslmlshfkvvk avkennidvevgitlnltpvylqterlgykvseiere mvslssqldnqlfldpvlkgsypqklldylvqkdll dsqkalsmqqevkenfifpdflginyytravrlyd ensswifpirwehpageytemgwevfpqglfdll iwikesypqipiyitengaayndivtedgkvhdsk rieylkqhfeaarkaiengvdlrgyfvwslmdnfe wamgytkrfgiiyvdyetqkrikkdsfyfyqqy- ikens | *Caldocellum saccharolyticum* | 47 |

### EXAMPLES

### Materials

Strain Y294 was obtained from Dr. W. H. Emile van Zyl, University of Stellenbosch, South Africa. BGLI from *Saccharomycopsis fibuligera* was derived from a plasmid supplied by Dr. van Zyl. CEN.PK 113-11C was obtained from Dr. Peter Kötter, Universität Frankfurt, Germany. The KanMX4 marker used in the integrating vector was derived by PCR from Plasmid M4297 provided by Dr. David Stillman, The University of Utah, U.S.A. The zeocin marker was derived by PCR from the vector pTEF1-Zeo, purchased from Invitrogen, Carlsbad, CA.

### Media and strain cultivation

*Escherichia coli* strain DH5α (Invitrogen) was used for plasmid transformation and propagation. Cells were grown in LB medium (5 g/L yeast extract, 5 g/L NaCl, 10 g/L tryptone) supplemented with ampicillin (100 mg/L), kanamycin (50mg/L) or zeocin (20 mg/L). When zeocin selection was desired LB was adjusted to pH 7.0. Fifteen grams per liter agar was added when solid media was desired.

*Saccharomyces cerevisiae* strains - Y294 (alpha leu2-3,112 ura3-52 his3 trp1-289); BJ5464 (MATalpha ura3-52 trp1 leu2-delta1 his3-delta200 pep4::HIS3 prb1-delta1.6R can1 GAL) and CEN.PK 113-11C (MATa, ura3-52, his3-delta1) - were grown in YPD (10 g/L yeast extract, 20 g/L peptone, 20 g/L glucose) or YPC (10 g/L yeast extract, 20 g/L peptone, 20 g/L cellobiose) media with either G418 (250 mg/L unless specified) or zeocin (20 mg/L unless specified) for selection. Fifteen grams per liter agar was added for solid media.

### EXAMPLE 1

### METHODS FOR ENGINEERING SACCHAROMYCES CEREVISIAE STRAINS WITH TETHERED CELLULASE ENZYMES

### Molecular methods

Standard protocols were followed for DNA manipulations (Sambrook, J.; Fritsch, E.; Maniatis, T. Molecular cloning: A laboratory manual. New York: Cold Spring Harbor Laboratory Press; 1989). PCR was performed using Phusion Polymerase (New England Biolabs, Ipswich, MA) for cloning, and Taq polymerase (New England Biolabs) for screening transformants. Manufacturer's guidelines were followed as supplied. Restriction enzymes were purchased from New England Biolabs and digests were set up according to the supplied guidelines. Ligations were performed using the Quick Ligation Kit (New England Biolabs) as specified by the manufacturer. Gel purification was performed using either Qiagen or Zymo research kits, PCR product and digest purifications were performed using Zymo research kits, and Qiagen midi and miniprep kits were used for purification of plasmid DNA. Sequencing was performed by the Molecular Biology Core Facility at Dartmouth College.

### Synthetic DNA constructs

Sequences for CBHI, CBHII and EGI from *Trichoderma reesei,* linker proteins, secretion signals, and anchoring domains were codon optimized for expression in *Saccharomyces cerevisiae* using either software provided by DNA 2.0, Menlo Park, CA, or using "Synthetic Gene Designer" (Wu, G.; Bashir-Bello, N.; Freeland, S.J. "The Synthetic Gene Designer: A flexible web platform to explore sequence manipulation for heterologous expression" Protein Expr. Purif. 47(2): 441-445, (2006)). The optimized sequences are disclosed as SEQ ID NOS: 27-35.

### Construction of a δ-integrating vector

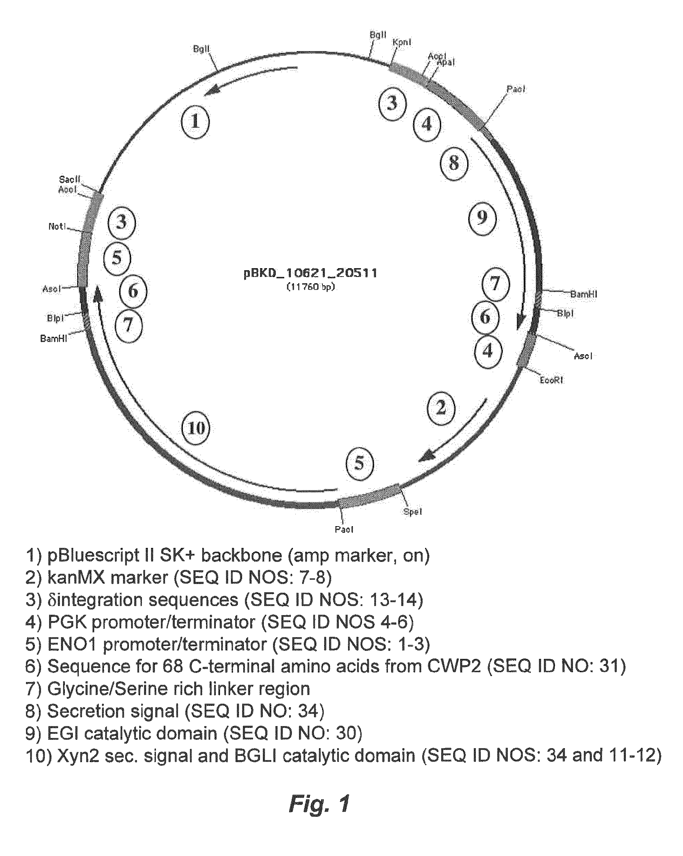

Vectors for integration into the *S*. *cerevisiae* genome in multiple copies were made in a number of steps. FIG. 1 shows an example of the final vector including two operons. Each operon includes a cellulase gene (9 or BGLI of 10) linked to a secretion signal (8 or *xyn2* of 10), that drives constitutive expression, as well as an anchoring domain (6) that facilitates attachment of the cellulase to the cell membrane. The cellulase gene, secretion signal and anchoring domain are flanked by a set of promoter/terminator sequences (4 or 5). The vector was constructed with two different dominant selectable markers, kanMX and TEF1/zeo. These markers were added to pBluescript II SK+ by first generating PCR fragments (primers SEQ ID NOS: 7 and 8 with plasmid 3, Table 2; SEQ ID NOS: 9 and 10 with plasmid 2, Table 2), digesting the fragments with EcoRI and SpeI, and ligating into the doubly digested (EcoRI/SpeI) pBluscript backbone. The constructs were confirmed first by selecting for *E. coli* strains resistant to both ampicillin (pBluescript backbone) and either kanamycin or zeocin, as well as by restriction digest to confirm the size of the insert.

**Table 2. Plasmids**

| # | **Name of Plasmid** | **Used for/Genes carried** | **Reference/ accession #** |
|---|---|---|---|
| 1 | pBluescript II SK+ | Expression vector backbone for assembling expression cassettes | X52328 |
| 2 | pTEF1-zeo | TEF1/Zeo marker | Invitrogen |
| 3 | M4297 | KanMX marker | Prof. David Stillman |
| 4 | ySFI | BGLI | Van Rooyen (2005) |
| 5 | pBK | pBluescript; KanMX marker | This work |
| 6 | pBZ | pBluescript; TEF1/Zeo marker | This work |
| 7 | pBK_1 | pBK + PGK P/T* | This work |
| 8 | pBK_2 | pBK + ENO1 P/T* | This work |
| 9 | pBZ_1 | pBZ + PGK P/T* | This work |
| 10 | pBZ_2 | pBZ + ENO1 P/T* | This work |
| 11 | pBKD1_1 | pBK_1 + 1 δ sequence | This work |
| 12 | pBKD1_2 | pBK_2 + 1 δ sequence | This work |
| 13 | pBZD1_1 | pBZ_1 + 1 δ sequence | This work |
| 14 | pBZD1_2 | pBZ_2 + 1 δ sequence | This work |
| 15 | pBKD_1 | pBK_1 + 2 δ sequences | This work |
| 16 | pBZD_1 | pBK_2 + 2 δ sequences | This work |
| 17 | pBKD_2 | pBZ_1 + 2 δ sequences | This work |
| 18 | pBZD_2 | pBZ_2 + 2 δ sequences | This work |
| 19 | pBKD_10001 | pBKD_1 + L1_A1 (original optimization) | This work |
| 20 | pBKD_20001 | pBKD_2 + L1_A1 (original optimization) | This work |
| 21 | pBZD_20001 a | pBZD_2 + L2 A1a (re-optimized) | This work |
| 22 | pBKD_20511 | pBKD_20001 + BGL1 | This work |
| 23 | pBKD_11621 | pBKD_10001 + S16 + C2 | This work |
| 24 | pBKD_10621 | pBKD_10001 + S06 + C2 | This work |
| 25 | pBKD_10621_20511 | pBKD_10621 + 20511 (i.e., only the cellulase construct) | This work |
| 26 | pBKD_11621_20511 | pBKD_11621 + 20511 (i.e., only the cellulase construct) | This work |
| 27 | pBZD_11631 | pBZD_1 + S16 + C3 L2 A1 | This work |
| 28 | pBZD_20641 | pBZD 20001 a + C4 L3 | This work |
| 29 | pBZD_11631_20641 | pBZD_11631 + 20641 (i.e., only the cellulase construct) | This work |

| | | | |
|---|---|---|---|
| *P/T = Promoter/Terminator | | | |

Promoter/Terminator (P/T) expression regions containing a multiple cloning site were made by overlap PCR using genomic DNA purified from *S*. *cerevisiae* strain Y294 and SEQ ID NOS: 1-3 and SEQ ID NOS: 4-6 for the enolase 1 (ENO1) and phosphoglycerate kinase (PGK), respectively. The first round of PCR utilized the forward and overlap primers (SEQ ID NOS: 1-2 or SEQ ID NOS: 4-5), and the second used the product of the first reaction and the reverse primer (SEQ ID NO: 3 or SEQ ID NO: 6). The products of these reactions were further amplified using only the forward (SEQ ID NO: 1 or SEQ ID NO: 4) and reverse primers (SEQ ID NO: 3 or SEQ ID NO: 6). These regions were restriction cloned into both pBK and pBZ using the ApaI and EcoRI sites encoded in the primers and in pBK and pBZ, creating plasmids 7-10, Table 2. The P/T constructs were sequenced using primers SEQ ID NOS: 15 and 16. The sequences matched the expected sequences exactly, with the exception of a few variations from the published PGK terminator sequences.

The sequences for integration at the δ sites in the *S*. *cerevisiae* genome were cloned into the backbone as follows. One copy was inserted by digesting SEQ ID NO: 27 from the plasmid supplied by DNA 2.0 with ApaI and KpnI, and ligating the resulting piece with ApaI/KpnI doubly digested plasmids 7-10, creating plasmids 11-14 (Table 2). A second copy was generated by performing PCR with SEQ ID NOS: 13 and 14 on the plasmid from DNA 2.0 containing the δ region, digesting the resulting fragment and plasmids 11-14 (Table 2) with NotI and SacII, and performing the ligations. This resulted in plasmids 15-18 (Table 2). The resulting constructs were again sequenced with primers SEQ ID NOS: 15 and 16 to verify the presence of two δ sequences.

An optimized portion of the cell wall gene *cwp2* and a flexible linker region between the cellulase and cell wall anchor (*cwp2*) were then added to the backbone. Plasmids 19 and 20 (Table 2) were constructed by digesting SEQ ID NO: 31 with BamHI and AscI and plasmids 15 and 17 (Table 2) and ligating the resulting fragments. Likewise, plasmid 21 (Table 2) was created by digesting SEQ ID NO: 29 and plasmid 17 with BamHI and AscI and ligating the appropriate fragments.

Cellulase constructs could then be added to the backbone expression vectors in a single, triple ligation step. β-Glucosidase from *Saccharomycopsis fibuligera* (BGLI) did not require the triple ligation as it already had a secretion signal. Therefore, it was prepared by PCR from plasmid 4 (Table 2) using primers comprising SEQ ID NOS: 11 and 12, digested with PacI and BamHI, and ligated with a PacI/BamHI digested plasmid 20, to create plasmid 22 (Table 2). Plasmids 23 and 24 for synthetic EGI expression were created by digesting SEQ ID NOS: 32 and 34 with MlyI and PacI, SEQ ID NO: 28 with MlyI and BamHI, and plasmid 19 (Table 2) with PacI and BamHI, purifying the appropriate fragments, and ligating all together. Plasmid 27 for CBHI expression was created by digesting SEQ ID NO: 34 with MlyI and PacI, SEQ ID NO: 29 with MlyI and AscI, plasmid 16 with PacI and AscI, and ligating these fragments in a triple ligation. Plasmid 28 was created by triple ligation of MlyI and PacI digested SEQ ID NO: 32, MlyI and BlpI digested SEQ ID NO: 30, and PacI and BlpI digested plasmid 21. These new constructs were sequence verified using primers SEQ ID NOS: 6 and 17 for the EGI and CBHI constructs, and primers SEQ ID NOS: 3 and 18 for the BGL and CBHII constructs.

Constructs for expressing two cellulase constructs simultaneously (either EGI and BGLI or CBHI and CBHII) were constructed by ligating the NotI/SpeI fragment of either plasmid 22 with NotI/SpeI digested plasmids 23 and 24, or by ligating the NotI/SpeI fragment of plasmid 28 with NotI/SpeI digested plasmid 27. These reactions resulted in plasmids 25, 26 and 29, which were sequenced to confirm the presence of both cellulase constructs using primers comprising SEQ ID NOS: 1, 3, 4 and 6.

### Yeast transformation

A protocol for electrotransformation of yeast was developed based on Cho, K.M.; Yoo, Y.J.; Kang, H.S. "delta-Integration of endo/exo-glucanase and beta-glucosidase genes into the yeast chromosomes for direct conversion of cellulose to ethanol" Enzyme And microbial Technology, 25: 23-30, (1999) and Ausubel, F.M.; Brent, R.; Kingston, R.; Moore, D.; Seidman, J.; Smith, J.; Struhl, K. Current protocols in molecular biology. USA: John Wiley and Sons, Inc. 1994. Linear fragments of DNA were created by digesting the desired vector with AccI and either BglI (for plasmids 22-26) or FspI (for plasmid 29). AccI has a unique site in the δ sequence and each of the other two enzymes cuts the pBluescript backbone in two places. The fragments were purified by precipitation with 3M sodium acetate and ice cold ethanol, subsequent washing with 70% ethanol, and resuspension in USB dH₂O (DNAse and RNAse free, sterile water) after drying in a 70°C vacuum oven.

Yeast cells for transformation were prepared by growing to saturation in 5mL YPD cultures. 4 mL of the culture was sampled, washed 2X with cold distilled water, and resuspended in 640 µL cold distilled water. 80 µL of 100mM Tris-HCl, 10mM EDTA, pH 7.5 (10X TE buffer - filter sterilized) and 80 µL of 1M lithium acetate, pH 7.5 (10X liAc - filter sterilized) were added and the cell suspension was incubated at 30°C for 45 minutes with gentle shaking. 20 µL of 1M DTT was added and incubation continued for 15 minutes. The cells were then centrifuged, washed once with cold distilled water, and once with electroporation buffer (1M sorbitol, 20mM HEPES), and finally resuspended in 267 µL electroporation buffer.

For electroporation, 10 µg of linearized DNA (measured by estimation on gel) was combined with 50 µL of the cell suspension in a sterile 1.5 mL microcentrifuge tube. The mixture was then transferred to a 0.2 cm electroporation cuvette, and a pulse of 1.4 kV (200Ω, 25 µF) was applied to the sample using the Biorad Gene Pulser device. 1mL of YPD with 1M sorbitol adjusted to pH 7.0 (YPDS) was placed in the cuvette and the cells were allowed to recover for -3 hrs. 100-200 µL cell suspension were spread out on YPDS agar plates with appropriate antibiotic, which were incubated at 30°C for 3-4 days until colonies appeared. Table 3 contains the genotypes of the yeast strains created.

**Table 3. Strains of S. cerevisiae created**

| **Name** | **Starting strain** | **Contains cellulase constructs from this (these) plasmid(s)** |
|---|---|---|
| Y_A1 | Y294 | pBKD_11621_20511 |
| Y_A2 | Y294 | pBKD_10621_20511 |
| Y_A3 | Y294 | pBKD_10421_20511 |
| Y_A4 | Y294 | pBKD_11721_20511 |
| CP1_A1 | CEN.PK 113-11C | pBKD_11621_20511 |
| CP1_A2 | CEN.PK 113-11C | pBKD_10621_20511 |
| CP1_A3 | CEN.PK 113-11C | pBKD_10421_20511 |
| CP1_A4 | CEN.PK 113-11C | pBKD_11721_20511 |
| BJ1_A1 | BJ5464 | pBKD_11621_20511 |
| BJ1_A2 | BJ5464 | pBKD_10621_20511 |
| BJ1_A3 | BJ5464 | pBKD_10421_20511 |
| BJ1_A4 | BJ5464 | pBKD_11721_20511 |
| Y_A1_C #1 | Y_A1 | pBKD_11621_20511; pBZD 11631_20641 |
| Y_A1_C1 #2 | Y_A1 | pBKD_11621_20511; pBZD_11631_20641 |
| Y_A1_C1 #3 | Y_A1 | pBKD_11621_20511; pBZD_11631_20641 |
| Y_A1_C1 #5 | Y_A1 | pBKD_11621_20511; pBZD_11631_20641 |
| Y_A1_C1 #6 | Y_A1 | pBKD_11621_20511; pBZD_11631_20641 |
| CP1_A1_C1 #1 | CP1_A1 | pBKD_11621_20511; pBZD_11631_20641 |
| CP1_A1_C1 #6A | CP1_A1 | pBKD_11621_20511; pBZD_11631_20641 |
| CP1_A1_C1 #11 | CP1_A1 | pBKD_11621_20511; pBZD_11631_20641 |
| CP1_A1_C1 #12 | CP1_A1 | pBKD_11621_20511; pBZD_11631_20641 |
| CP1_A1_C1 #17 | CP1_A1 | pBKD_11621_20511; pBZD_11631_20641 |
| BJ1_A1_C1 #7 | BJ1_A1 | pBKD_11621_20511; pBZD_11631_20641 |
| CP1_ A1_C1 #10 | BJ1_A1 | pBKD_11621_20511; pBZD_11631_20641 |

### Enzyme assays

β-Glucosidase activity was measured in a manner similar to that described by McBride, J.E.; Zietsman, J.J.; Van Zyl, W.H.; and Lynd, L.R. "Utilization of cellobiose by recombinant beta-glucosidase-expressing strains of Saccharomyces cerevisiae: characterization and evaluation of the sufficiency of expression" Enzyme And Microbial Technology, 37: 93-101, (2005), except that the volume of the assay was decreased and the reaction performed in a microtiter plate. Briefly, yeast strains were grown to saturation in YPD or YPC media with or without appropriate antibiotics; the optical density at 600nm (OD(600)) was measured; and a 0.5 mL sample of the culture was centrifuged, the supernatant was separated and saved, and the cell pellet was washed two times with 50mM citrate buffer, pH 5.0. Reactions for supernatants were made up of 50 µL sample, 50 µL citrate buffer, and 50 µL 20 mM p-nitrophenyl-β-D-glucopyranoside (PNPG) substrate. Reactions with washed cells consisted of 25 µL of cells, 75 µL citrate buffer, and 50 µL PNPG substrate. If activity was too high for the range of the standard curve, a lower cell concentration was used and the assay was rerun. The standard curve consisted of a 2-fold dilution series of nitrophenol (PNP) standards, starting at 500 nM, and ending at 7.8 nM, and a buffer blank was included. After appropriate dilutions of supernatant or cells were prepared, the microtiter plate was incubated at 37°C for 10 minutes along with the reaction substrate. The reaction was carried out by adding the substrate, incubating for 30 minutes, and stopping the reaction with 150 µL of 2M Na₂CO₃. The plate was then centrifuged at 2500 rpm for 5 minutes, and 150 µL of supernatant was transferred to another plate. The absorbance at 405 nm was read for each well.

Endoglucanase activity was qualitatively detected by observing clearing zones on synthetic complete media plates (as above, but including 20 g/L glucose) with 0.1 % carboxymethyl cellulose (CMC) stained with Congo red (Beguin, P. "Detection of Cellulase Activity in Polyacrylamide Gels using Congo Red-Stained Agar Replicas" Analytical Biochemistry, 131: 333-336, (1983)). Cells were grown for 2-3 days on the plates and were washed off the plate with 1M Tris-HCl buffer, pH 7.5. The plates were then stained for 10 minutes with a 0.1 % Congo red solution, and extra dye was subsequently washed off with 1M NaCl.

### Verification of transformants

For EGI and BGLI transformants, activities were verified by enzyme assay as specified above. For strains where all four cellulases were transformed, PCR with primers SEQ ID NOS: 19-26 was used to verify the presence in genomic DNA of each of the genes being expressed.

After genetic confirmation of the presence of the genes, strains were grown in rich media (YPD) to saturation, and ∼10^7 cells were washed once with sterile Tris-HCl buffer and inoculated into 10 mL of liquid media in a sealed hungate tube with an air atmosphere. Cell counts were performed on samples taken over time using a haemocytometer. Cell density was measured by spectrophotometry after digestion of the samples with a commercial cellulase preparation (Spezyme CP) added with buffer and sodium azide to inhibit subsequent growth of the cultures. The digestion procedure was verified by plotting the cell number/mL against the OD(600). A value of 3*10^7 cells/mL = 1 OD(600) was obtained.

Growth media with cellulose substrates as the sole carbon source were made using the non-glucose components of synthetic complete medium for yeast including, yeast nitrogen base without amino acids -1.7 g/L, ammonium sulfate - 5 g/L, and supplemented with amino acids. Ten milliliters of PASC media (prepared at 2% dry weight) or BMCC media (prepared at 1% dry weight) were placed in sealed hungate tubes for growth experiments.

### EXAMPLE 2

### SACCHAROMYCES CEREVISIAE STRAINS WITH TETHERED CELLULASE ENZYMES CAPABLE OF GROWING ON PHOSPHORIC ACID SWOLLEN CELLULOSE (PASC)

Endoglucanase I (EGI), cellobiohydrolase I (CBHI) and cellobiohydrolase II (CBHII) from *Trichoderma reesei,* along with β-glucosidase I (BGLI) from *Saccharomycopsis fibuligera,* were expressed as tethered proteins to the *Saccharomyces cerevisiae* cell surface by fusion with the C-terminal portion of *cwp2* from *S*. *cerevisiae,* as described above.

For growth experiments on phosphoric acid swollen cellulose (PASC) media, PASC was added as the sole carbon source to synthetic complete medium for yeast at a concentration of 20 g/L. Phosphoric acid swollen cellulose (PASC) was prepared as in Zhang, Y.H.; Cui, J.; Lynd, L.R.; Kuang, L.S. "A transition from cellulose swelling to cellulose dissolution by o-phosphoric acid: evidence from enzymatic hydrolysis and supramolecular structure" Biomacromolecules, 7, 644-648 (2006), with slight modification. Avicel PH105 (10 g) was wetted with 100 mL of distilled water in a 4L flask. Eight hundred milliliters of 86.2% phosphoric acid was added slowly to the flask with a first addition of 300mL followed by mixing and subsequent additions of 50mL aliquots. The transparent solution was kept at 4°C for 1 hour to allow complete solubilization of the cellulose, at which point no lumps remained in the reaction mixture. Next, 2L of ice-cooled distilled water were added in 500mL aliquots with mixing between additions. Three hundred milliliter aliquots of the mixture were centrifuged at 5,000 rpm for 20 minutes at 2°C and the supernatant removed. Addition of 300 mL cold distilled water and subsequent centrifugation was repeated four times. 4.2 mL of 2M sodium carbonate and 300 mL of water were added to the cellulose, followed by two or three washes with distilled water, until the final pH was ∼6. Samples were dried to constant weight in a 70°C vacuum oven to measure the dry weight.

Growth experiments carried out in sealed hungate tubes as described above, were sampled by syringe, and the cells were counted. Additionally, samples were digested at 37°C with a commercial cellulase preparation and sodium azide until all substrate was digested. The absorbance at 600nm was then taken to measure the cell density. Post digestion OD(600) measurements correlated as expected with cell counts done by haemocytometer.

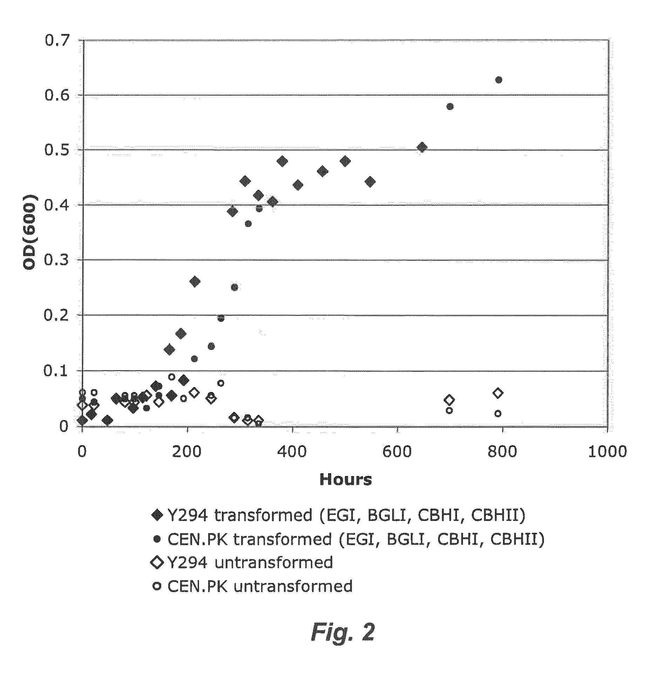

FIG. 2 shows the OD(600) results for growth of native (untransformed) and recombinant strains of *Saccharomyces cerevisiae* on PASC. Strains created in the Y294 and CEN.PK backgrounds expressing all four cellulase enzymes showed slow, but significant increases in OD(600) over the course of the growth experiment. Untransformed controls from both strains showed no increase in OD(600) over the course of the eight hundred hour growth experiment.

### EXAMPLE 3

### SACCHAROMYCES CEREVISIAE STRAINS WITH TETHERED CELLULASE ENZYMES CAPABLE OF GROWING ON BACTERIAL MICROCRYSTALLINE CELLULOSE (BMCC)

Endoglucanase I (EGI), cellobiohydrolase I (CBHI) and cellobiohydrolase II (CBHII) from *Trichoderma reesei,* along with β-glucosidase I (BGLI) from *Saccharomycopsis fibuligera,* were expressed as tethered proteins to the *Saccharomyces cerevisiae* cell surface by fusion with the C-terminal portion of *cwp2* from *S*. *cerevisiae* as described above.

For growth experiments in bacterial microcrystalline cellulose (BMCC) containing media, BMCC was added as the sole carbon source to synthetic complete medium for yeast at a concentration of 10 g/L. Bacterial microcrystalline cellulose (BMCC) was prepared in a similar manner to Jung, H.; Wilson, D.B.; Walker, L.P. "Binding and Reversibility of Thermobifida fusca Cel5A, Cel6B, and Cel48A and their respective catalytic domains to bacterial microcrystalline cellulose" Biotechnology and Bioengineering, 84, 151-159, (2003), except that sodium azide was not added during reconstitution, and washing was carried out by washing and centrifugation five times with distilled water. Quadruplicate 1mL samples were frozen and then freeze dried to determine the dry weight of the final BMCC suspension.

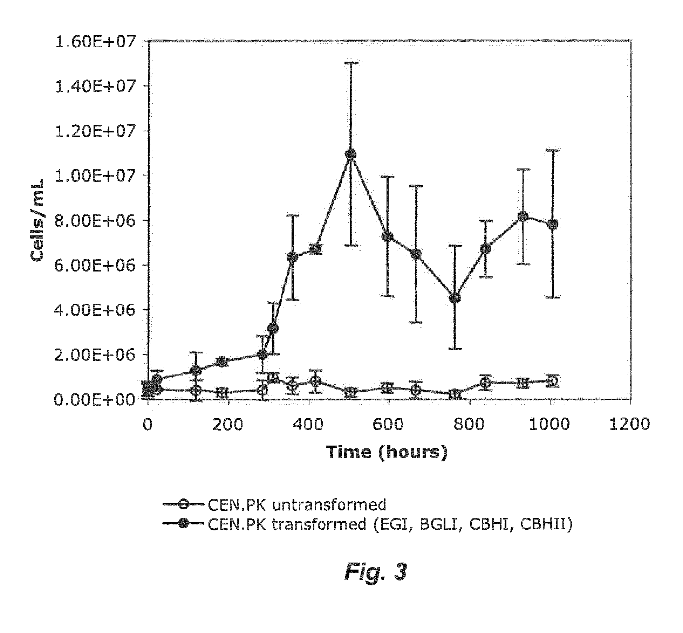

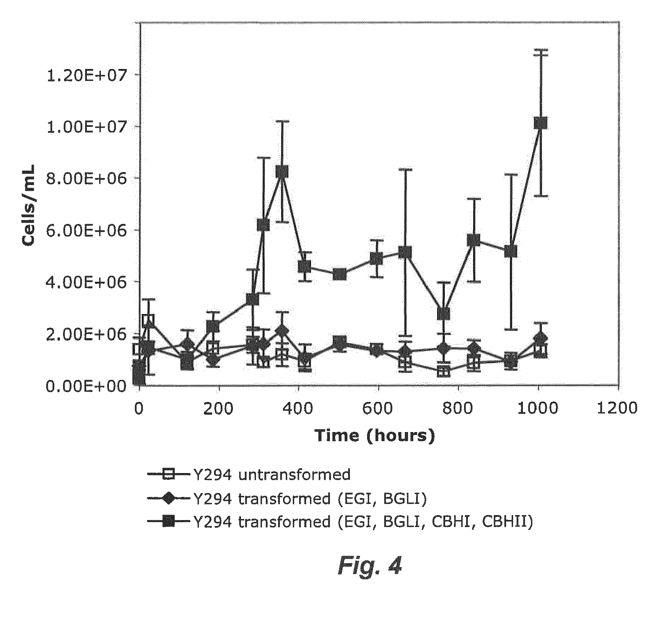

FIGS. 3 and 4 show cell count results for growth of native (untransformed) and recombinant yeast strains of *Saccharomyces cerevisiae* on BMCC. Strains created in the Y294 and CEN.PK backgrounds expressing all four cellulase enzymes showed a slow, but significant increase in cell counts/mL over the course of the growth experiment. Y294 expressing only BGLI and EGI showed no increase in cell counts/mL over the course of the experiment. Untransformed controls from both strains showed no increase in cell counts over the course of the approximately seven hundred hour growth experiment. These results demonstrate the necessity of utilizing all four cellulases to achieve growth on BMCC when the cellulases are tethered.

### EXAMPLE 4

### RECOMBINANT YEAST STRAINS WITH ENHANCED CELLULOSE BINDING PROPERTIES

Endoglucanse I (EGI) from *Trichoderma reesei* and β-glucosidase I (BGLI) from *Saccharomycopsis fibuligera* were expressed as tethered proteins to the *Saccharomyces cerevisiae* cell surface by fusion with the C-terminal portion of *cwp2* from *S*. *cerevisiae,* as described above.

In order to screen the transformed strains for the best cellulose binding individuals, strains expressing tethered enzymes were grown to saturation in 5 mL rich media (∼10^9 total cells). Fifty, ten, or 0.25 mg of ELCHEMA P100 cellulose was washed 5-8 times with distilled water and autoclaved. The cellulose was then added to each enzyme preparation and allowed to settle to the bottom of the tube. The cell containing supernatant was then removed, and the cellulose pellet was resuspended in sterile 50 mM Tris-HCl buffer, pH 7.5. The pellet was allowed to settle again and the buffer was removed. This process was repeated four more times before rich media was added back to the tube containing the cellulose pellet and cells were allowed to grow again to saturation. The selection procedure was performed a number of times for both transformed strains expressing the cellulase enzymes and the untransformed strains.

A cellulose binding assay was used to examine the original and selected strains. The assay was adapted from Ito, J.; Fujita, Y.; Ueda, M.; Fukuda, H.; Kondo, A. "Improvement of cellulose-degrading ability of a yeast strain displaying Trichoderma reesei endoglucanase II by recombination of cellulose-binding domains" Biotechnology Progress, 20: 688-691, (2004) and Nam, J.; Fujita, Y.; Arai, T.; Kondo, A.; Morikawa, Y.; Okada, H.; Ueda, M.; Tanka, A. "Construction of engineered yeast with the ability of binding to cellulose" Journal of Molecular Catalysis B: Enzymatic 17: 197-202, (2002). Cells from a saturated culture grown in rich media were washed twice in citrate buffer, pH 5.0. They were resuspended in citrate buffer at an OD(600) = 2.0, or ~6*10^7 cells/mL, in a volume of 2.75 mL and allowed to sit upright in a test tube for ten minutes. A 0.25 mL sample was taken to measure the initial OD(600) of the suspension. A half milliliter of a 10% solution of cellulose (Avicel PH101) was added to each tube. The tubes were then mixed at room temperature and allowed to stand upright for ten minutes. (Ito and Nam used incubations at 4°C for 24 hours before standing the tubes upright.) A second 0.25 mL sample was obtained and the OD(600) measured.

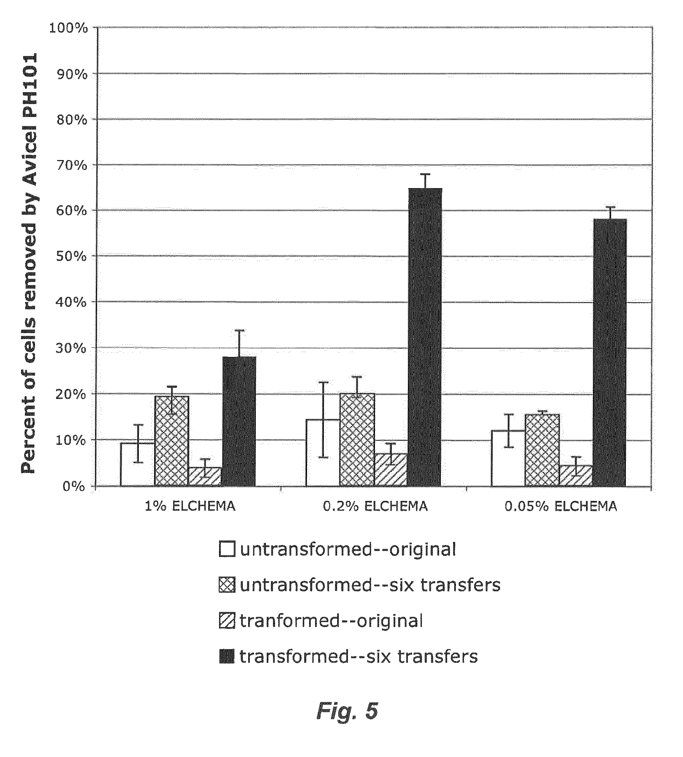

The cellulose binding results for two strains, which were subjected to the washing and re-growth procedure six times with a variety of starting ELCHEMA concentrations are summarized in FIG. 5. Of particular note is that strains with high OD(600) reductions by cellulose were obtained for strains with cellulases expressed when selected with 0.2 or 0.05% ELCHEMA, while untransformed strains increased their binding ability to a lesser degree. For the transformed strains expressing the cellulases, OD(600) reductions were increased by 5.5, 12.7, and 11.3 fold for the 1%, 0.2%, and 0.05% ELCHEMA concentrations used during selection, respectively. By comparison, the untransformed control increased its OD(600) reduction ability by only 1.6, 1.7, and 1.3 fold under the same conditions. These results demonstrate the increased cellulose binding ability of the transformed populations.

For comparison, the highest OD(600) reductions reported for Avicel are: 24.2% in Nam *et al.* and ∼23% in Ito *et al.* (24 hour, 4°C incubation). Fukuda, T.; Ishikawa, T.; Ogawa, M.; Shiraga, S.; Kato, M.; Suye, S.; Ueda, M. "Enhancement of Cellulase Activity by Clones Selected from the Combinatorial Library of the Cellulose-Binding Domain by Cell Surface Engineering", Biotechnology Progress 22: 933-938 (2006) do not report the percent OD(600) reduction for their strains, but indicate that their techniques have increased the strains binding capability by 1.5 fold, as compared to the 12.7 fold improvement observed with the present strains.

### EXAMPLE 5

### SACCHAROMYCES CEREVISIAE STRAINS WITH TETHERED CELLULASE ENZYMES CAPABLE OF GROWING IN SEMI-CONTINUOUS CULTURE WITH AVICEL PH105

Endoglucanase I (EGI), cellobiohydrolase I (CBHI) and cellobiohydrolase II (CBHII) from *Trichoderma reesei,* along with β-glucosidase I (BGLI) from *Saccharomycopsis fibuligera,* were expressed as tethered proteins to the *Saccharomyces cerevisiae* cell surface by fusion with the C-terminal portion of *cwp2* from *S*. *cerevisiae,* as described above.

Semi-continuous cultures of *Saccharomyces cerevisiae* strain CEN.PK 113-11C (both untransformed and transformed with BGLI, EGI, CBHI and CBHII) were carried out in 3L (total volume) Applikon bioreactors. Avicel (-20 g/L; PH105 from FMC Biopolymer, Philadelphia, PA) was added to synthetic complete medium for yeast (yeast nitrogen base without amino acids 1.7 g/L, ammonium sulfate 5 g/L, and supplemented with amino acids) lacking a carbon source. Avicel containing media was stirred in a 5L carboy and intermittently pumped (every 80 minutes) into two side-by-side Applikon reactor systems, with working volumes of 1.8L. The reactors were stirred at 400 rpm, and media was pumped out after a feeding following a 2 minute delay. Pump control, pH control and temperature control were all carried out using a DeltaV control system from Emerson Process Management, St. Louis, Missouri. Conditions in the reactors were maintained at pH 5.0 using 1N HCl and 2N KOH, stirring at 400 rpm, an aeration rate of 1 VVM, and a temperature of 30 °C. The dilution rate was maintained at ∼0.01 hr^-1, which was verified by measuring the volume of the media accumulated in a waste carboy. The total dry weight of a system containing only water and avicel was monitored to verify that avicel was fed evenly over time. Inoculation cultures were pre-grown in YPD (yeast extract 10 g/L, peptone 20 g/L, glucose 20g/L) and washed once with Tris-HCl buffer (pH 7.5) prior to inoculation. Cells were quantified by direct counts and dilution plating on YPD, as described above.

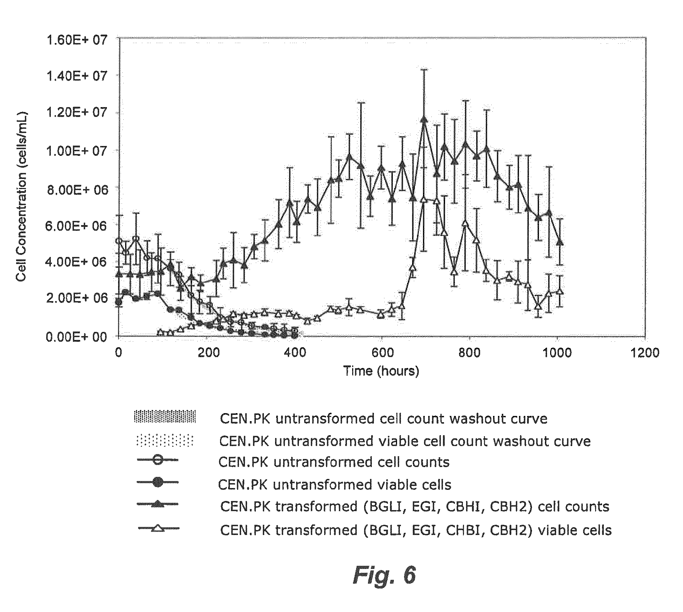

FIG. 6 shows the results from the two side-by-side reactors. The untransformed strain showed decreasing cell counts and viable cell counts over time, as expected in the absence of replication. Dotted lines show calculated wash-out (dilution) curves for non-replicating cells at the dilution rate measured. The observed correlation between the data and calculated wash-out curves confirms that the untransformed CEN.PK strain cannot replicate in the tested media.

On the other hand, the transformed strain of CEN.PK, expressing all four cellulase enzymes, grew and maintained its cell concentration for the duration of the continuous culture experiment (∼1000hrs). In fact, the transformed strain showed a modest increase in cell concentration over the course of the experiment as measured both by cell counts and viable cell counts.

### Deposit of Recombinant Yeast Strains

Y294 and CEN.PK yeast strains containing the cellulase genes BGLI, EGI, CBHI and CBHII have been deposited with the American Type Culture Collection, Manassas, VA 20110-2209. The deposits were made on November 21, 2007 and received Patent Deposit Designation Numbers PTA -XXXX and PTA -XXXX, respectively. These deposits were made in compliance with the Budapest Treaty requirements that the duration of the deposits should be for thirty (30) years from the date of deposit or for five (5) years after the last request for the deposit at the depository or for the enforceable life of a U.S. Patent that matures from this application, whichever is longer. The deposits will be replenished should one or more of them become non-viable at the depository.

The description of the specific embodiments reveals general concepts that others can modify and/or adapt for various applications or uses that do not depart from the general concepts. Therefore, such adaptations and modifications should and are intended to be comprehended within the meaning and range of equivalents of the disclosed embodiments. It is to be understood that the phraseology or terminology employed herein is for the purpose of description and not limitation.

All references mentioned in this application are incorporated by reference to the same extent as though fully replicated herein.

## Claims

1. A transformed yeast cell that expresses one or more genes, wherein said one or more genes encode one or more celllulases expressed in the form of tethered enzymes in said transformed yeast cell, said one or more celllulases being selected from the group consisting of:
(a) a polypeptide having the sequence of SEQ ID No. 36,
(b) a polypeptide having the sequence of SEQ ID No. 37,
(c) a polypeptide having the sequence of SEQ ID No. 38,
(d) a polypeptide having the sequence of SEQ ID No. 39,
(e) a polypeptide having the sequence of SEQ ID No. 40,
(f) a polypeptide having the sequence of SEQ ID No. 41,
(g) a polypeptide having the sequence of SEQ ID No. 42,
(h) a polypeptide having the sequence of SEQ ID No. 43,
(i) a polypeptide having the sequence of SEQ ID No. 44,
(j) a polypeptide having the sequence of SEQ ID No. 45,
(k) a polypeptide having the sequence of SEQ ID No. 46,
(l) a polypeptide having the sequence of SEQ ID No. 47, and
(m) a polypeptide sequence having at least 95% sequence identity with the sequence of any one of the polypeptide sequence of (a)-(1).

2. The yeast cell according to claim 1, wherein the yeast is selected from the group consisting of *Saccharomyces cerevisiae, Schizosaccharomyces pombe, Candida albicans, Kluyveromyces lactis, Pichia pastoris, Pichia stipitis, Yarrowia lipolytica, Hansenula polymorpha, Phaffia rhodozyma, Candida utilis, Arxula adeninivorans, Debaryomyces hansenii, Debaryomyces polymorphus, Kluyveromyces marxianus, Issatchenkia orientalis* and *Schwanniomyces occidentalis.*

3. The yeast cell according to claim 1, wherein the yeast is a member of the *Saccharomyces* genus.

4. A method for selecting a transformed yeast cell with enhanced binding affinity for insoluble cellulose, comprising: transforming a native organism to produce the transformed yeast of claim 1; culturing the transformed yeast under suitable conditions for a period sufficient to allow growth and replication of the transformed yeast; exposing a sample of the transformed yeast from the culture to the insoluble cellulose; and selecting the transformed yeast whose sample provides at least a two fold reduction in supernatant optical density relative to the results from a similarly cultured and exposed sample of the native organism.

5. A method for producing ethanol, said method comprising: transforming a yeast to produce the yeast of claim 1; and culturing the transformed yeast in a medium that contains cellulose under suitable conditions for a period sufficient to allow saccharification and fermentation of the cellulose to ethanol.

6. An isolated polynucleotide encoding a tethered enzyme, wherein said tethered enzyme comprises at least one polypeptide selected from the group consisting of:
(a) a polypeptide having the sequence of SEQ ID No. 36,
(b) a polypeptide having the sequence of SEQ ID No. 37,
(c) a polypeptide having the sequence of SEQ ID No. 38,
(d) a polypeptide having the sequence of SEQ ID No. 39,
(e) a polypeptide having the sequence of SEQ ID No. 40,
(f) a polypeptide having the sequence of SEQ ID No. 41,
(g) a polypeptide having the sequence of SEQ ID No. 42,
(h) a polypeptide having the sequence of SEQ ID No. 43,
(i) a polypeptide having the sequence of SEQ ID No. 44,
(j) a polypeptide having the sequence of SEQ ID No. 45,
(k) a polypeptide having the sequence of SEQ ID No. 46,
(l) a polypeptide having the sequence of SEQ ID No. 47, and
(m) a polypeptide sequence having at least 95% sequence identity with the sequence of any one of the polypeptide sequence of (a)-(1).

7. A vector comprising the isolated polynucleotide of claim 6.

8. A host cell comprising the polynucleotide of claim 6, wherein said polynucleotide is introduced into said host cell through transformation.

9. A method of producing ethanol, comprising: culturing a yeast host cell according to claim 8 in a medium containing cellulose under suitable conditions for a period of time sufficient to allow saccharification and fermentation of the cellulose to ethanol, wherein the yeast host cell is selected from the group consisting of *Saccharomyces cerevisiae, Schizosaccharomyces pombe, Candida albicans, Kluyveromyces lactis, Pichia pastoris, Pichia stipitis, Yarrowia lipolytica, Hansenula polymorpha, Phaffia rhodozyma, Candida utilis, Arxula adeninivorans, Debaryomyces hansenii, Debaryomyces polymorphus, Kluyveromyces marxianus, Issatchenkia orientalis* and *Schwanniomyces occidentalis.*

10. A genetic construct comprising a polynucleotide operably connected to a promoter, said polynucleotide encoding a polypeptide selected from the group consisting of:
(a) a polypeptide having the sequence of SEQ ID No. 36,
(b) a polypeptide having the sequence of SEQ ID No. 37,
(c) a polypeptide having the sequence of SEQ ID No. 38,
(d) a polypeptide having the sequence of SEQ ID No. 39,
(e) a polypeptide having the sequence of SEQ ID No. 40,
(f) a polypeptide having the sequence of SEQ ID No. 41,
(g) a polypeptide having the sequence of SEQ ID No. 42,
(h) a polypeptide having the sequence of SEQ ID No. 43,
(i) a polypeptide having the sequence of SEQ ID No. 44,
(j) a polypeptide having the sequence of SEQ ID No. 45,
(k) a polypeptide having the sequence of SEQ ID No. 46,
(l) a polypeptide having the sequence of SEQ ID No. 47, and
(m) a polypeptide sequence having at least 95% sequence identity with the sequence of any one of the polypeptide sequence of (a)-(1).

11. A recombinant yeast comprising the genetic construct of claim 10.

12. The transformed yeast cell according to claim 1, wherein the encoded enzymes are fused with an anchor protein.

13. The transformed yeast cell according to claim 12, wherein the anchor protein is a polypeptide encoded by the cwp2 gene or a fragment thereof.


SEQUENCE LISTING

<110>  The Trustees of Dartmouth College
       McBride, John E.E.
       Deleault, Kirsten M.
       Lynd, Lee R.
       Pronk, Jack T.

<120>  Recombinant Yeast Strains Expressing Tethered Cellulase Enzymes

<130>  474292

<150>  60/867,018
<151>  2006-11-22

<160>  47

<170>  PatentIn version 3.2

<210>  1
<211>  43
<212>  DNA
<213>  Artificial Sequence

<220>
<223>  Enolase 1 (ENO1) forward primer


<220>
<221>  exon
<222>  (1)..(43)

<400>  1
tat atg ggc cca cta gtc ttc tag gcg ggt tat cta ctg atc c          43
Tyr Met Gly Pro Leu Val Phe     Ala Gly Tyr Leu Leu Ile
1               5                       10


<210>  2
<211>  72
<212>  DNA
<213>  Artificial Sequence

<220>
<223>  Enolase 1 (ENO1) overlap primer


<220>
<221>  exon
<222>  (1)..(72)

<400>  2
gga cta gaa ggc tta atc aaa agc ggc gcg ccg gat cct taa tta agt      48
Gly Leu Glu Gly Leu Ile Lys Ser Gly Ala Pro Asp Pro     Leu Ser
1               5                   10                      15

gtg ttg ata agc agt tgc ttg gtt                                      72
Val Leu Ile Ser Ser Cys Leu Val
                20


<210>  3
<211>  42
<212>  DNA
<213>  Artificial Sequence

<220>
<223>  Enolase 1 (ENO1) reverse primer


<220>
<221>  exon
<222>  (1)..(42)

<400>  3
gct acg aat tcg cgg ccg ccg tcg aac aac gtt cta tta gga                42
Ala Thr Asn Ser Arg Pro Pro Ser Asn Asn Val Leu Leu Gly
1               5                   10


<210>  4
<211>  33
<212>  DNA
<213>  Artificial Sequence

<220>
<223>  Phosphoglycerate kinase (PGK) forward primer


<220>
<221>  exon
<222>  (1)..(33)

<400>  4
tat atg ggc cct ccc tcc ttc ttg aat tga tgt                            33
Tyr Met Gly Pro Pro Ser Phe Leu Asn     Cys
1               5                       10


<210>  5
<211>  79
<212>  DNA
<213>  Artificial Sequence

<220>
<223>  Phosphoglycerate kinase (PGK) overlap primer


<220>
<221>  exon
<222>  (1)..(79)

<400>  5
gat cta tcg att tca att caa ttc aat ggc gcg ccg gat cct taa tta        48
Asp Leu Ser Ile Ser Ile Gln Phe Asn Gly Ala Pro Asp Pro     Leu
1               5                   10                      15

atg taa aaa gta gat aat tac ttc ctt gat g                              79
Met     Lys Val Asp Asn Tyr Phe Leu Asp
                    20


<210>  6
<211>  31
<212>  DNA
<213>  Artificial Sequence

<220>
<223>  Phosphoglycerate kinase (PGK) reverse primer

<220>
<221>  exon
<222>  (1)..(31)

<400>  6
ctt agg aat tct ttc gaa acg cag aat ttt c                                31
Leu Arg Asn Ser Phe Glu Thr Gln Asn Phe
1               5                   10


<210>  7
<211>  30
<212>  DNA
<213>  Artificial Sequence

<220>
<223>  Kanamycin (kanMX) forward primer


<220>
<221>  exon
<222>  (1)..(30)

<400>  7
gat ccg aat tcg ttt agc ttg cct cgt ccc                                  30
Asp Pro Asn Ser Phe Ser Leu Pro Arg Pro
1               5                   10


<210>  8
<211>  30
<212>  DNA
<213>  Artificial Sequence

<220>
<223>  Kanamycin (kanMX) reverse primer


<220>
<221>  exon
<222>  (1)..(30)

<400>  8
cag tcg act agt ttt cga cac tgg atg gcg                                  30
Gln Ser Thr Ser Phe Arg His Trp Met Ala
1               5                   10


<210>  9
<211>  33
<212>  DNA
<213>  Artificial Sequence

<220>
<223>  Zeocin (Zeo) forward primer


<220>
<221>  exon
<222>  (1)..(33)

<400>  9
gcg cta gaa ttc ccc aca cac cat agc ttc aaa                              33
Ala Leu Glu Phe Pro Thr His His Ser Phe Lys

1                 5                   10


<210>  10
<211>  41
<212>  DNA
<213>  Artificial Sequence

<220>
<223>  Zeocin (Zeo) reverse primer



<220>
<221>  exon
<222>  (1)..(41)

<400>  10
ccg cat act agt aat tca gct tgc aaa tta aag cct tcg ag                41
Pro His Thr Ser Asn Ser Ala Cys Lys Leu Lys Pro Ser
1                 5                   10


<210>  11
<211>  41
<212>  DNA
<213>  Artificial Sequence

<220>
<223>  Beta-glucosidase I (BGLI) forward primer



<220>
<221>  exon
<222>  (1)..(41)

<400>  11
gcc gcc tta att aaa aac aaa atg gtc tcc ttc acc tcc ct                41
Ala Ala Leu Ile Lys Asn Lys Met Val Ser Phe Thr Ser
1                 5                   10


<210>  12
<211>  38
<212>  DNA
<213>  Artificial Sequence

<220>
<223>  Beta-glucosidase I (BGLI) reverse primer



<220>
<221>  exon
<222>  (1)..(38)

<400>  12
cgg ttg gat cca ata gta aac agg aca gat gtc ttg at                    38
Arg Leu Asp Pro Ile Val Asn Arg Thr Asp Val Leu
1                 5                   10


<210>  13
<211>  37
<212>  DNA
<213>  Artificial Sequence

<220>
<223>  Delta2 forward primer


<220>
<221>  exon
<222>  (1)..(37)

<400>  13
agt cgc ggc cgc tgt tgg aat aaa aat cca cta tcg t                    37
Ser Arg Gly Arg Cys Trp Asn Lys Asn Pro Leu Ser
1               5                   10


<210>  14
<211>  40
<212>  DNA
<213>  Artificial Sequence

<220>
<223>  Delta2 reverse primer


<220>
<221>  exon
<222>  (1)..(40)

<400>  14
gcg ccc gcg gtg aga tat atg tgg gta att aga taa ttg t                40
Ala Pro Ala Val Arg Tyr Met Trp Val Ile Arg     Leu
1               5                   10


<210>  15
<211>  18
<212>  DNA
<213>  Artificial Sequence

<220>
<223>  M13 forward primer


<220>
<221>  exon
<222>  (1)..(18)

<400>  15
tcc cag tca cga cgt cgt                                              18
Ser Gln Ser Arg Arg Arg
1               5


<210>  16
<211>  19
<212>  DNA
<213>  Artificial Sequence

<220>
<223>  M13 reverse primer


<220>
<221>  exon
<222>  (1)..(19)

<400>  16
gga aac agc tat gac cat g                                              19
Gly Asn Ser Tyr Asp His
1               5


<210>  17
<211>  27
<212>  DNA
<213>  Artificial Sequence

<220>
<223>  Phosphoglycerate kinase (PGK) seq forward primer


<220>
<221>  exon
<222>  (1)..(27)

<400>  17
tct ttt tct ctt ttt tac aga tca tca                                    27
Ser Phe Ser Leu Phe Tyr Arg Ser Ser
1               5


<210>  18
<211>  27
<212>  DNA
<213>  Artificial Sequence

<220>
<223>  Enolase 1 (ENO1) seq forward primer


<220>
<221>  exon
<222>  (1)..(27)

<400>  18
tcc ttc tag cta ttt ttc ata aaa aac                                    27
Ser Phe     Leu Phe Phe Ile Lys Asn
1                   5


<210>  19
<211>  20
<212>  DNA
<213>  Artificial Sequence

<220>
<223>  Endoglucase I (EGI) detection forward primer


<220>
<221>  exon
<222>  (1)..(20)

<400>  19
cgc tag tgg tgt tac gac ga                                             20
Arg     Trp Cys Tyr Asp
1                   5


<210>  20
<211>  20

<212>  DNA
<213>  Artificial Sequence

<220>
<223>  Endoglucanase I (EGI) detection reverse primer


<220>
<221>  exon
<222>  (1)..(20)

<400>  20
ctc caa gtc tgc act gga ca                                          20
Leu Gln Val Cys Thr Gly
1               5


<210>  21
<211>  20
<212>  DNA
<213>  Artificial Sequence

<220>
<223>  Beta-glucosidase I (BGLI) detection forward primer


<220>
<221>  exon
<222>  (1)..(20)

<400>  21
gag ccc gca tta tta tcc aa                                          20
Glu Pro Ala Leu Leu Ser
1               5


<210>  22
<211>  20
<212>  DNA
<213>  Artificial Sequence

<220>
<223>  Beta-glucosidase I (BGLI) detection reverse primer


<220>
<221>  exon
<222>  (1)..(20)

<400>  22
caa agt cag cga atc gaa ca                                          20
Gln Ser Gln Arg Ile Glu
1               5


<210>  23
<211>  20
<212>  DNA
<213>  Artificial Sequence

<220>
<223>  Cellobiohydrolase I (CBHI) detection forward primer


<220>

<221>  exon
<222>  (1)..(20)

<400>  23
aga cgg ttg tga ctg gaa cc                                          20
Arg Arg Leu     Leu Glu
1               5


<210>  24
<211>  20
<212>  DNA
<213>  Artificial Sequence

<220>
<223>  Cellobiohydrolase I (CBHI) detection reverse primer


<220>
<221>  exon
<222>  (1)..(20)

<400>  24
caa ctt gag ctg gaa cac ca                                          20
Gln Leu Glu Leu Glu His
1               5


<210>  25
<211>  20
<212>  DNA
<213>  Artificial Sequence

<220>
<223>  Cellobiohydrolase II (CBHII) detection forward primer


<220>
<221>  exon
<222>  (1)..(20)

<400>  25
cag aga ctg tgc tgc ttt gg                                          20
Gln Arg Leu Cys Cys Phe
1               5


<210>  26
<211>  20
<212>  DNA
<213>  Artificial Sequence

<220>
<223>  Cellobiohydrolase II (CBHII) detection reverse primer


<220>
<221>  exon
<222>  (1)..(20)

<400>  26
gga tct acc ttg gtc ggt ga                                          20
Gly Ser Thr Leu Val Gly
1               5

<210> 27
<211> 358
<212> DNA
<213> Saccharomyces cerevisiae

<220>
<221> exon
<222> (1)..(358)

<400> 27
agt cgg tac ctg ttg gaa taa aaa tcc act atc gtc tat caa cta ata 48
Ser Arg Tyr Leu Leu Glu Lys Ser Thr Ile Val Tyr Gln Leu Ile
1               5                   10                  15

gtt ata tta tca ata tat tat cat ata cgg tgt taa gat gat gac ata 96
Val Ile Leu Ser Ile Tyr Tyr His Ile Arg Cys Asp Asp Asp Ile
                20                  25                  30

agt tat gag aag ctg tca tcg atg tta gag gaa gct gaa acg caa gga 144
Ser Tyr Glu Lys Leu Ser Ser Met Leu Glu Glu Ala Glu Thr Gln Gly
                35                  40                  45

ttg ata atg taa tag gat caa tga ata taa aca tat aaa acg gaa tga 192
Leu Ile Met Asp Gln Ile Thr Tyr Lys Thr Glu
            50                  55

gga ata atc gta ata tta gta tgt aga aat ata gat tcc att ttg agg 240
Gly Ile Ile Val Ile Leu Val Cys Arg Asn Ile Asp Ser Ile Leu Arg
        60                  65                  70

att cct ata tcc tcg agg aga act tct agt ata ttc tgt ata cct aat 288
Ile Pro Ile Ser Ser Arg Arg Thr Ser Ser Ile Phe Cys Ile Pro Asn
    75                  80                  85

att ata gcc ttt atc aac aat gga atc cca aca att atc taa tta ccc 336
Ile Ile Ala Phe Ile Asn Asn Gly Ile Pro Thr Ile Ile Leu Pro
90                  95                  100

aca tat atc tca ggg ccc gcg c 358
Thr Tyr Ile Ser Gly Pro Ala
105                 110

<210> 28
<211> 1354
<212> DNA
<213> Trichoderma reesei

<220>
<221> exon
<222> (1)..(1354)

<400> 28
gag tcc cgg gca aca acc agg aac atc aac acc aga agt cca tcc aaa 48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga 96
Val Asn Asn Leu Met Tyr Glu Trp Arg Val Cys Ser Ala Gly
            20                  25                  30

cac aag tgt ggt ctt aga ctg gaa tta tcg ttg gat gca tga tgc caa 144

His Lys Cys Gly Leu Arg Leu Glu Leu Ser Leu Asp Ala     Cys Gln
            35                  40                      45

tta taa ttc ctg tac tgt taa cgg cgg tgt taa cac tac gtt atg ccc      192
Leu     Phe Leu Tyr Cys     Arg Arg Cys     His Tyr Val Met Pro
                    50                      55

cga tga agc gac ttg tgg taa gaa ttg ttt tat tga agg ggt tga cta      240
Arg     Ser Asp Leu Trp     Glu Leu Phe Tyr     Arg Gly     Leu
        60                      65                          70

cgc cgc tag tgg tgt tac gac gag tgg gtc atc ctt gac gat gaa tca      288
Arg Arg     Trp Cys Tyr Asp Glu Trp Val Ile Leu Asp Asp Glu Ser
                75                  80                      85

ata cat gcc ttc ttc tag tgg tgg gta ttc ctc tgt gtc tcc aag gct      336
Ile His Ala Phe Phe     Trp Trp Val Phe Leu Cys Val Ser Lys Ala
                90                      95                  100

gta ttt att gga ttc cga tgg gga ata tgt tat gtt aaa att aaa tgg      384
Val Phe Ile Gly Phe Arg Trp Gly Ile Cys Tyr Val Lys Ile Lys Trp
            105                 110                     115

gca aga act gag ttt tga tgt gga tct atc tgc att acc ttg tgg aga      432
Ala Arg Thr Glu Phe     Cys Gly Ser Ile Cys Ile Thr Leu Trp Arg
        120                     125                     130

aaa tgg tag tct tta ttt atc aca aat gga cga aaa cgg cgg agc caa      480
Lys Trp     Ser Leu Phe Ile Thr Asn Gly Arg Lys Arg Arg Ser Gln
            135                     140                     145

tca gta caa tac agc tgg tgc taa tta tgg ttc agg cta ttg tga tgc      528
Ser Val Gln Tyr Ser Trp Cys     Leu Trp Phe Arg Leu Leu     Cys
        150                     155                         160

tca atg tcc agt gca gac ttg gag gaa tgg cac ctt aaa cac atc aca      576
Ser Met Ser Ser Ala Asp Leu Glu Glu Trp His Leu Lys His Ile Thr
            165                 170                     175

tca agg att ttg ctg taa cga aat gga cat att aga agg taa ttc aag      624
Ser Arg Ile Leu Leu     Arg Asn Gly His Ile Arg Arg     Phe Lys
        180                     185                         190

agc taa tgc act aac tcc gca ctc ttg tac tgc gac cgc atg tga ttc      672
Ser     Cys Thr Asn Ser Ala Leu Leu Tyr Cys Asp Arg Met     Phe
            195                     200

tgc cgg ttg tgg ttt caa ccc tta tgg ttc tgg tta taa gag tta cta      720
Cys Arg Leu Trp Phe Gln Pro Leu Trp Phe Trp Leu     Glu Leu Leu
205                 210                     215

cgg tcc ggg aga cac cgt gga tac gtc aaa gac ctt cac tat aat cac      768
Arg Ser Gly Arg His Arg Gly Tyr Val Lys Asp Leu His Tyr Asn His
220                 225                     230                 235

tca gtt taa cac aga taa cgg atc tcc gag tgg taa ttt ggt gag tat      816
Ser Val     His Arg     Arg Ile Ser Glu Trp     Phe Gly Glu Tyr
                        240                         245

tac tag gaa ata tca gca gaa cgg tgt tga tat tcc gtc cgc gca gcc      864
Tyr     Glu Ile Ser Ala Glu Arg Cys     Tyr Ser Val Arg Ala Ala
        250                     255                     260

agg cgg tga cac tat atc tag ctg tcc ttc cgc cag tgc cta tgg cgg      912
Arg Arg     His Tyr Ile     Leu Ser Phe Arg Gln Cys Leu Trp Arg

265                             270                             275

act tgc tac aat ggg taa ggc att gtc ctc agg tat ggt cct agt att        960
Thr Cys Tyr Asn Gly     Gly Ile Val Leu Arg Tyr Gly Pro Ser Ile
                280                     285                     290

ttc tat ttg gaa tga taa ttc aca ata cat gaa ttg gct gga ttc tgg       1008
Phe Tyr Leu Glu         Phe Thr Ile His Glu Leu Ala Gly Phe Trp
                295                         300                     305

taa tgc agg ccc ttg ctc ctc tac aga agg taa ccc aag caa tat act       1056
    Cys Arg Pro Leu Leu Leu Tyr Arg Arg     Pro Lys Gln Tyr Thr
                    310                         315

agc taa taa ccc aaa tac tca tgt tgt ctt tag taa tat tag atg ggg       1104
Ser         Pro Lys Tyr Ser Cys Cys Leu         Tyr     Met Gly
320                         325                                 330

cga tat agg tag cac tac gaa cag tac cgc acc tcc tcc tcc acc tgc       1152
Arg Tyr Arg     His Tyr Glu Gln Tyr Arg Thr Ser Ser Ser Thr Cys
                    335                     340                     345

tag ctc cac gac att ttc cac tac tag aag gtc cag cac tac cag ctc       1200
    Leu His Asp Ile Phe His Tyr     Lys Val Gln His Tyr Gln Leu
                    350                         355

atc acc atc ttg tac tca aac cca ttg ggg aca gtg tgg tgg tat agg       1248
Ile Thr Ile Leu Tyr Ser Asn Pro Leu Gly Thr Val Trp Trp Tyr Arg
360                 365                     370                     375

tta cag cgg ttg caa aac ttg cac atc tgg tac tac atg cca ata cag       1296
Leu Gln Arg Leu Gln Asn Leu His Ile Trp Tyr Tyr Met Pro Ile Gln
                380                     385                     390

taa tga cta tta ctc aca atg ttt acc agg tgc tgc gtc aag ttc aag       1344
        Leu Leu Leu Thr Met Phe Thr Arg Cys Cys Val Lys Phe Lys
                    395                     400                     405

tag tgg atc c                                                         1354
    Trp Ile

<210>  29
<211>  2520
<212>  DNA
<213>  Trichoderma reesei

<220>
<221>  exon
<222>  (1)..(2520)

<400>  29
gag tcc cgg gca aca acc agg aac atc aac acc aga agt cca tcc aaa         48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga         96
Val Asn Asn Leu     Met Tyr     Glu Trp Arg Val Cys Ser Ala Gly
                20                          25                  30

cac aag tgt ggt ctt aga ctg gaa tta tcg ttg gat gca tga tgc caa        144
His Lys Cys Gly Leu Arg Leu Glu Leu Ser Leu Asp Ala     Cys Gln
                    35                  40                          45

tta taa ttc ctg tac tgt taa cgg cgg tgt taa cac tac gtt atg ccc 192
Leu Phe Leu Tyr Cys Arg Arg Cys His Tyr Val Met Pro
                50                  55

cga tga agc gac ttg tgg taa gaa ttg ttt tat tga agg ggt tga cta 240
Arg Ser Asp Leu Trp Glu Leu Phe Tyr Arg Gly Leu
    60                  65                  70

cgc cgc tag tgg tgt tac gac gag tgg gtc atc ctt gac gat gaa tca 288
Arg Arg Trp Cys Tyr Asp Glu Trp Val Ile Leu Asp Asp Glu Ser
                75                  80                  85

ata cat gcc ttc ttc tag tgg tgg gta ttc ctc tgt gtc tcc aag gct 336
Ile His Ala Phe Phe Trp Trp Val Phe Leu Cys Val Ser Lys Ala
                90                  95                  100

gta ttt att gga ttc cga tgg gga ata tgt tat gtt aaa att aaa tgg 384
Val Phe Ile Gly Phe Arg Trp Gly Ile Cys Tyr Val Lys Ile Lys Trp
                105                 110                 115

gca aga act gag ttt tga tgt gga tct atc tgc att acc ttg tgg aga 432
Ala Arg Thr Glu Phe Cys Gly Ser Ile Cys Ile Thr Leu Trp Arg
            120                 125                 130

aaa tgg tag tct tta ttt atc aca aat gga cga aaa cgg cgg agc caa 480
Lys Trp Ser Leu Phe Ile Thr Asn Gly Arg Lys Arg Arg Ser Gln
            135                 140                 145

tca gta caa tac agc tgg tgc taa tta tgg ttc agg cta ttg tga tgc 528
Ser Val Gln Tyr Ser Trp Cys Leu Trp Phe Arg Leu Leu Cys
            150                 155                 160

tca atg tcc agt gca gac ttg gag gaa tgg cac ctt aaa cac atc aca 576
Ser Met Ser Ser Ala Asp Leu Glu Glu Trp His Leu Lys His Ile Thr
                165                 170                 175

tca agg att ttg ctg taa cga aat gga cat att aga agg taa ttc aag 624
Ser Arg Ile Leu Leu Arg Asn Gly His Ile Arg Arg Phe Lys
            180                 185                 190

agc taa tgc act aac tcc gca ctc ttg tac tgc gag tcc cgg gca atc 672
Ser Cys Thr Asn Ser Ala Leu Leu Tyr Cys Glu Ser Arg Ala Ile
                195                 200                 205

cgc ttg tac cct aca atc cga aac tca ccc acc att gac ctg gca aaa 720
Arg Leu Tyr Pro Thr Ile Arg Asn Ser Pro Thr Ile Asp Leu Ala Lys
                210                 215                 220

gtg ttc tag cgg tgg aac ttg tac tca aca aac tgg ttc tgt tgt tat 768
Val Phe Arg Trp Asn Leu Tyr Ser Thr Asn Trp Phe Cys Cys Tyr
            225                 230                 235

cga cgc taa ctg gag atg gac aca cgc cac taa ctc ttc tac caa ctg 816
Arg Arg Leu Glu Met Asp Thr Arg His Leu Phe Tyr Gln Leu
            240                 245                 250

tta cga cgg taa cac ttg gtc ttc cac ttt atg tcc aga taa cga aac 864
Leu Arg Arg His Leu Val Phe His Phe Met Ser Arg Arg Asn
                255                 260

ttg tgc taa gaa ttg ctg ttt gga cgg tgc cgc cta cgc ttc tac cta 912
Leu Cys Glu Leu Leu Phe Gly Arg Cys Arg Leu Arg Phe Tyr Leu
265                 270                 275

cgg tgt tac cac ctc cgg taa ctc ctt gtc tat tgg ttt cgt cac tca     960
Arg Cys Tyr His Leu Arg     Leu Leu Val Tyr Trp Phe Arg His Ser
280                 285                 290

atc cgc tca aaa gaa cgt tgg tgc tag att gta ctt gat ggc ttc tga     1008
Ile Arg Ser Lys Glu Arg Trp Cys     Ile Val Leu Asp Gly Phe
295                 300                 305

cac tac tta tca aga att tac ttt gtt ggg taa cga att ttc ttt cga     1056
His Tyr Leu Ser Arg Ile Tyr Phe Val Gly     Arg Ile Phe Phe Arg
    310                 315                     320

tgt tga cgt ttc cca att gcc atg tgg ctt gaa cgg tgc ttt gta ctt     1104
Cys     Arg Phe Pro Ile Ala Met Trp Leu Glu Arg Cys Phe Val Leu
        325                 330                 335

tgt ctc tat gga tgc tga cgg tgg tgt ttc taa gta ccc aac taa cac     1152
Cys Leu Tyr Gly Cys     Arg Trp Cys Phe     Val Pro Asn     His
    340                     345                     350

tgc cgg tgc taa gta cgg tac tgg tta ctg tga ttc tca atg tcc acg     1200
Cys Arg Cys     Val Arg Tyr Trp Leu Leu     Phe Ser Met Ser Thr
                355                 360                     365

tga ctt gaa gtt cat taa cgg tca agc caa cgt cga agg ttg gga acc     1248
    Leu Glu Val His     Arg Ser Ser Gln Arg Arg Arg Leu Gly Thr
                        370                 375

atc ctc caa caa cgc taa cac cgg tat cgg tgg tca cgg ttc ctg ttg     1296
Ile Leu Gln Gln Arg     His Arg Tyr Arg Trp Ser Arg Phe Leu Leu
380                     385                 390

ttc cga aat gga cat ctg gga agc taa cag tat ttc tga agc ttt gac     1344
Phe Arg Asn Gly His Leu Gly Ser     Gln Tyr Phe     Ser Phe Asp
395                 400                 405

acc aca ccc atg cac cac tgt cgg tca aga aat ttg tga agg tga tgg     1392
Thr Thr Pro Met His His Cys Arg Ser Arg Asn Leu     Arg     Trp
    410                 415                 420

atg tgg tgg aac cta ctc tga taa cag ata cgg tgg tac ttg tga ccc     1440
Met Trp Trp Asn Leu Leu         Gln Ile Arg Trp Tyr Leu     Pro
        425                         430                     435

aga cgg ttg tga ctg gaa ccc ata cag att ggg taa cac ttc ttt cta     1488
Arg Arg Leu     Leu Glu Pro Ile Gln Ile Gly     His Phe Phe Leu
                    440                 445

tgg tcc agg ttc ttc ttt cac ctt gga tac cac caa gaa gtt gac tgt     1536
Trp Ser Arg Phe Phe Phe His Leu Gly Tyr His Gln Glu Val Asp Cys
450                 455                 460                 465

tgt tac cca att cga aac ttc tgg tgc tat caa cag ata cta cgt tca     1584
Cys Tyr Pro Ile Arg Asn Phe Trp Cys Tyr Gln Gln Ile Leu Arg Ser
                470                 475                 480

aaa cgg tgt cac ctt cca aca acc aaa cgc tga att ggg ttc tta ctc     1632
Lys Arg Cys His Leu Pro Thr Thr Lys Arg     Ile Gly Phe Leu Leu
        485                 490                     495

tgg taa tga att gaa cga cga cta ctg tac cgc tga aga agc tga att     1680
Trp         Ile Glu Arg Arg Leu Leu Tyr Arg     Arg Ser     Ile
                500                 505

tgg tgg ttc ctc ttt ctc cga caa ggg tgg ttt gac cca att caa gaa     1728

Trp Trp Phe Leu Phe Leu Arg Gln Gly Trp Phe Asp Pro Ile Gln Glu
    510                 515                 520

ggc tac ctc cgg tgg tat ggt ttt ggt tat gtc ctt gtg gga tga tta      1776
Gly Tyr Leu Arg Trp Tyr Gly Phe Gly Tyr Val Leu Val Gly     Leu
525                 530                 535

cta cgc aaa cat gtt atg gtt aga cag tac tta ccc aac taa cga aac      1824
Leu Arg Lys His Val Met Val Arg Gln Tyr Leu Pro Asn     Arg Asn
540                 545                 550

ctc ctc tac tcc agg tgc tgt cag agg ttc ctg ttc tac ctc ttc tgg      1872
Leu Leu Tyr Ser Arg Cys Cys Gln Arg Phe Leu Phe Tyr Leu Phe Trp
555                 560                 565                 570

tgt tcc agc tca agt tga atc tca atc tcc aaa cgc taa ggt cac ttt      1920
Cys Ser Ser Ser Ser     Ile Ser Ile Ser Lys Arg     Gly His Phe
                575                 580

ctc caa cat caa gtt cgg tcc aat cgg ttc cac tgg taa tcc atc tgg      1968
Leu Gln His Gln Val Arg Ser Asn Arg Phe His Trp     Ser Ile Trp
585                 590                 595

tgg aaa ccc tcc agg tgg taa cag agg tac tac cac tac tcg tag gcc      2016
Trp Lys Pro Ser Arg Trp     Gln Arg Tyr Tyr His Tyr Ser     Ala
600                 605                 610

agc tac tac aac tgg ttc ttc ccc agg ccc aac cca atc cca cta cgg      2064
Ser Tyr Tyr Asn Trp Phe Phe Pro Arg Pro Asn Pro Ile Pro Leu Arg
    615                 620                 625

tca atg tgg tgg tat cgg tta ctc tgg tcc aac cgt ctg tgc ttc tgg      2112
Ser Met Trp Trp Tyr Arg Leu Leu Trp Ser Asn Arg Leu Cys Phe Trp
630                 635                 640                 645

tac tac ctg tca agt ttt aaa ccc ata cta ctc tca atg ttt gcc tgg      2160
Tyr Tyr Leu Ser Ser Phe Lys Pro Ile Leu Leu Ser Met Phe Ala Trp
                650                 655                 660

tgc tgc ttc cag ttc atc tag tgg atc cgg tgg cgg tgg atc tgg agg      2208
Cys Cys Phe Gln Phe Ile     Trp Ile Arg Trp Arg Trp Ile Trp Arg
                665                 670                 675

agg cgg ttc ttg gtc tca ccc aca att tga aaa ggg tgg aga aaa ctt      2256
Arg Arg Phe Leu Val Ser Pro Thr Ile     Lys Gly Trp Arg Lys Leu
                680                 685                 690

gta ctt tca agg cgg tgg tgg agg ttc tgg cgg agg tgg ctc cgg ctc      2304
Val Leu Ser Arg Arg Trp Trp Arg Phe Trp Arg Arg Trp Leu Arg Leu
                695                 700                 705

agc tat ctc tca aat cac cga cgg tca aat cca agc cac tac cac agc      2352
Ser Tyr Leu Ser Asn His Arg Arg Ser Asn Pro Ser His Tyr His Ser
        710                 715                 720

tac cac tga agc tac aac tac cgc tgc tcc ttc atc tac tgt tga aac      2400
Tyr His     Ser Tyr Asn Tyr Arg Cys Ser Phe Ile Tyr Cys     Asn
    725                 730                 735

tgt ttc tcc atc ttc cac cga aac cat ctc tca aca aac cga aaa cgg      2448
Cys Phe Ser Ile Phe His Arg Asn His Leu Ser Thr Asn Arg Lys Arg
        740                 745                 750

tgc tgc taa ggc tgc tgt tgg tat ggg tgc tgg tgc ttt ggc tgc tgc      2496
Cys Cys     Gly Cys Cys Trp Tyr Gly Cys Trp Cys Phe Gly Cys Cys

755                 760                 765

tgc tat gtt gtt gta ggg cgc gcc                                 2520
Cys Tyr Val Val Val Gly Arg Ala
    770                 775

<210>  30
<211>  3314
<212>  DNA
<213>  Trichoderma reesei

<220>
<221>  exon
<222>  (1)..(3314)

<400>  30
gag tcc cgg gca aca acc agg aac atc aac acc aga agt cca tcc aaa      48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga      96
Val Asn Asn Leu     Met Tyr     Glu Trp Arg Val Cys Ser Ala Gly
            20                          25                  30

cac aag tgt ggt ctt aga ctg gaa tta tcg ttg gat gca tga tgc caa     144
His Lys Cys Gly Leu Arg Leu Glu Leu Ser Leu Asp Ala     Cys Gln
                35                  40                      45

tta taa ttc ctg tac tgt taa cgg cgg tgt taa cac tac gtt atg ccc     192
Leu     Phe Leu Tyr Cys     Arg Arg Cys     His Tyr Val Met Pro
                    50                          55

cga tga agc gac ttg tgg taa gaa ttg ttt tat tga agg ggt tga cta     240
Arg     Ser Asp Leu Trp     Glu Leu Phe Tyr     Arg Gly     Leu
        60                      65                              70

cgc cgc tag tgg tgt tac gac gag tgg gtc atc ctt gac gat gaa tca     288
Arg Arg     Trp Cys Tyr Asp Glu Trp Val Ile Leu Asp Asp Glu Ser
                    75                  80                  85

ata cat gcc ttc ttc tag tgg tgg gta ttc ctc tgt gtc tcc aag gct     336
Ile His Ala Phe Phe     Trp Trp Val Phe Leu Cys Val Ser Lys Ala
                90                      95                  100

gta ttt att gga ttc cga tgg gga ata tgt tat gtt aaa att aaa tgg     384
Val Phe Ile Gly Phe Arg Trp Gly Ile Cys Tyr Val Lys Ile Lys Trp
                105                 110                 115

gca aga act gag ttt tga tgt gga tct atc tgc att acc ttg tgg aga     432
Ala Arg Thr Glu Phe     Cys Gly Ser Ile Cys Ile Thr Leu Trp Arg
            120                     125                 130

aaa tgg tag tct tta ttt atc aca aat gga cga aaa cgg cgg agc caa     480
Lys Trp     Ser Leu Phe Ile Thr Asn Gly Arg Lys Arg Arg Ser Gln
                135                 140                 145

tca gta caa tac agc tgg tgc taa tta tgg ttc agg cta ttg tga tgc     528
Ser Val Gln Tyr Ser Trp Cys     Leu Trp Phe Arg Leu Leu     Cys
        150                         155                     160

tca atg tcc agt gca gac ttg gag gaa tgg cac ctt aaa cac atc aca     576
Ser Met Ser Ser Ala Asp Leu Glu Glu Trp His Leu Lys His Ile Thr
            165                 170                 175

tca agg att ttg ctg taa cga aat gga cat att aga agg taa ttc aag 624
Ser Arg Ile Leu Leu Arg Asn Gly His Ile Arg Arg Phe Lys
            180                 185                 190

agc taa tgc act aac tcc gca ctc ttg tac tgc gag tcc cgg gca atc 672
Ser Cys Thr Asn Ser Ala Leu Leu Tyr Cys Glu Ser Arg Ala Ile
                195                 200                 205

cgc ttg tac cct aca atc cga aac tca ccc acc att gac ctg gca aaa 720
Arg Leu Tyr Pro Thr Ile Arg Asn Ser Pro Thr Ile Asp Leu Ala Lys
                210                 215                 220

gtg ttc tag cgg tgg aac ttg tac tca aca aac tgg ttc tgt tgt tat 768
Val Phe Arg Trp Asn Leu Tyr Ser Thr Asn Trp Phe Cys Cys Tyr
            225                 230                 235

cga cgc taa ctg gag atg gac aca cgc cac taa ctc ttc tac caa ctg 816
Arg Arg Leu Glu Met Asp Thr Arg His Leu Phe Tyr Gln Leu
            240                 245                 250

tta cga cgg taa cac ttg gtc ttc cac ttt atg tcc aga taa cga aac 864
Leu Arg Arg His Leu Val Phe His Phe Met Ser Arg Arg Asn
                255                 260

ttg tgc taa gaa ttg ctg ttt gga cgg tgc cgc cta cgc ttc tac cta 912
Leu Cys Glu Leu Leu Phe Gly Arg Cys Arg Leu Arg Phe Tyr Leu
265                 270                 275

cgg tgt tac cac ctc cgg taa ctc ctt gtc tat tgg ttt cgt cac tca 960
Arg Cys Tyr His Leu Arg Leu Leu Val Tyr Trp Phe Arg His Ser
280                 285                 290

atc cgc tca aaa gaa cgt tgg tgc tag att gta ctt gat ggc ttc tga 1008
Ile Arg Ser Lys Glu Arg Trp Cys Ile Val Leu Asp Gly Phe
295                 300                 305

cac tac tta tca aga att tac ttt gtt ggg taa cga att ttc ttt cga 1056
His Tyr Leu Ser Arg Ile Tyr Phe Val Gly Arg Ile Phe Phe Arg
    310                 315                 320

tgt tga cgt ttc cca att gcc atg tgg ctt gaa cgg tgc ttt gta ctt 1104
Cys Arg Phe Pro Ile Ala Met Trp Leu Glu Arg Cys Phe Val Leu
    325                 330                 335

tgt ctc tat gga tgc tga cgg tgg tgt ttc taa gta ccc aac taa cac 1152
Cys Leu Tyr Gly Cys Arg Trp Cys Phe Val Pro Asn His
    340                 345                 350

tgc cgg tgc taa gta cgg tac tgg tta ctg tga ttc tca atg tcc acg 1200
Cys Arg Cys Val Arg Tyr Trp Leu Leu Phe Ser Met Ser Thr
            355                 360                 365

tga ctt gaa gtt cat taa cgg tca agc caa cgt cga agg ttg gga acc 1248
Leu Glu Val His Arg Ser Ser Gln Arg Arg Arg Leu Gly Thr
                370                 375

atc ctc caa caa cgc taa cac cgg tat cgg tgg tca cgg ttc ctg ttg 1296
Ile Leu Gln Gln Arg His Arg Tyr Arg Trp Ser Arg Phe Leu Leu
380                 385                 390

ttc cga aat gga cat ctg gga agc taa cag tat ttc tga agc ttt gac 1344
Phe Arg Asn Gly His Leu Gly Ser Gln Tyr Phe Ser Phe Asp
395                 400                 405

acc aca ccc atg cac cac tgt cgg tca aga aat ttg tga agg tga tgg     1392
Thr Thr Pro Met His His Cys Arg Ser Arg Asn Leu     Arg     Trp
    410                 415                 420

atg tgg tgg aac cta ctc tga taa cag ata cgg tgg tac ttg tga ccc     1440
Met Trp Trp Asn Leu Leu         Gln Ile Arg Trp Tyr Leu     Pro
        425                         430                     435

aga cgg ttg tga ctg gaa ccc ata cag att ggg taa cac ttc ttt cta     1488
Arg Arg Leu     Leu Glu Pro Ile Gln Ile Gly     His Phe Phe Leu
                    440                 445

tgg tcc agg ttc ttc ttt cac ctt gga tac cac caa gaa gtt gac tgt     1536
Trp Ser Arg Phe Phe Phe His Leu Gly Tyr His Gln Glu Val Asp Cys
450                 455                 460                 465

tgt tac cca att cga aac ttc tgg tgc tat caa cag ata cta cgt tca     1584
Cys Tyr Pro Ile Arg Asn Phe Trp Cys Tyr Gln Gln Ile Leu Arg Ser
                470                 475                 480

aaa cgg tgt cac ctt cca aca acc aaa cgc tga att ggg ttc tta ctc     1632
Lys Arg Cys His Leu Pro Thr Thr Lys Arg     Ile Gly Phe Leu Leu
            485                 490                     495

tgg taa tga att gaa cga cga cta ctg tac cgc tga aga agc tga att     1680
Trp         Ile Glu Arg Arg Leu Leu Tyr Arg     Arg Ser     Ile
                500                 505

tgg tgg ttc ctc ttt ctc cga caa ggg tgg ttt gac cca att caa gaa     1728
Trp Trp Phe Leu Phe Leu Arg Gln Gly Trp Phe Asp Pro Ile Gln Glu
    510                 515                 520

ggc tac ctc cgg tgg tat ggt ttt ggt tat gtc ctt gtg gga tga tta     1776
Gly Tyr Leu Arg Trp Tyr Gly Phe Gly Tyr Val Leu Val Gly     Leu
525                 530                 535

cta cgc aaa cat gtt atg gtt aga cag tac tta ccc aac taa cga aac     1824
Leu Arg Lys His Val Met Val Arg Gln Tyr Leu Pro Asn     Arg Asn
540                 545                 550

cga gtc ccg ggg tcc cat tag aag aaa gac aag cct gct cct ctg ttt     1872
Arg Val Pro Gly Ser His     Lys Lys Asp Lys Pro Ala Pro Leu Phe
555                 560                 565

ggg gtc aat gtg gtg gtc aaa act ggt ctg gtc caa ctt gtt gtg ctt     1920
Gly Val Asn Val Val Val Lys Thr Gly Leu Val Gln Leu Val Val Leu
570                 575                 580                 585

ccg gtt cta cct gtg ttt act cca acg act act att ccc aat gtt tgc     1968
Pro Val Leu Pro Val Phe Thr Pro Thr Thr Thr Ile Pro Asn Val Cys
                590                 595                 600

cag gtg ctg ctt cct ctt cct ctt caa cta gag ctg ctt cta caa ctt     2016
Gln Val Leu Leu Pro Leu Pro Leu Gln Leu Glu Leu Leu Leu Gln Leu
            605                 610                 615

cta ggg tct ccc caa cca ctt cca gat cct ctt ctg cta ctc cac cac     2064
Leu Gly Ser Pro Gln Pro Leu Pro Asp Pro Leu Leu Leu Leu His His
        620                 625                 630

cag gtt cta cta cca cta gag ttc cac cag tcg gtt ccg gta ctg cta     2112
Gln Val Leu Leu Pro Leu Glu Phe His Gln Ser Val Pro Val Leu Leu
    635                 640                 645

ctt act ctg gta acc ctt tcg tcg gtg tta ctc cat ggg cta acg ctt     2160

Leu Thr Leu Val Thr Leu Ser Ser Val Leu Leu His Gly Leu Thr Leu
650                 655                 660                 665

act acg ctt ctg aag ttt ctt ctt tgg cta tcc cat ctt tga ctg gtg     2208
Thr Thr Leu Leu Lys Phe Leu Leu Trp Leu Ser His Leu     Leu Val
            670                 675                         680

cta tgg cta ccg ctg ctg ctg ctg tcg cca aag ttc cat cct tca tgt     2256
Leu Trp Leu Pro Leu Leu Leu Leu Ser Pro Lys Phe His Pro Ser Cys
            685                 690                 695

ggt tgg aca cct tgg aca aaa ctc cat taa tgg aac aaa cct tgg cag     2304
Gly Trp Thr Pro Trp Thr Lys Leu His     Trp Asn Lys Pro Trp Gln
        700                 705                     710

aca taa gga ctg cta aca aga acg gcg gta act acg ctg gtc aat ttg     2352
Thr     Gly Leu Leu Thr Arg Thr Ala Val Thr Thr Leu Val Asn Leu
            715                 720                 725

ttg tgt acg act tgc cag aca gag act gtg ctg ctt tgg ctt cca acg     2400
Leu Cys Thr Thr Cys Gln Thr Glu Thr Val Leu Leu Trp Leu Pro Thr
        730                 735                 740

gtg aat act cca tcg ctg acg gtg gtg tcg cca agt aca aga act aca     2448
Val Asn Thr Pro Ser Leu Thr Val Val Ser Pro Ser Thr Arg Thr Thr
    745                 750                 755

ttg ata cca tta gac aaa tcg ttg tcg aat act ctg aca tca gaa cct     2496
Leu Ile Pro Leu Asp Lys Ser Leu Ser Asn Thr Leu Thr Ser Glu Pro
    760                 765                 770

tgt tag tca tcg aac cag att ctt tag cca att tag tca cca act tgg     2544
Cys     Ser Ser Asn Gln Ile Leu     Pro Ile     Ser Pro Thr Trp
775                 780                         785

gta ctc caa agt gtg cta acg ctc aat ctg cct act tag aat gta tca     2592
Val Leu Gln Ser Val Leu Thr Leu Asn Leu Pro Thr     Asn Val Ser
        790                 795                     800

att atg cag tta ccc aat tga act tgc caa acg ttg cta tgt act tgg     2640
Ile Met Gln Leu Pro Asn     Thr Cys Gln Thr Leu Leu Cys Thr Trp
        805                     810                 815

acg ctg gtc acg ccg gtt ggt tgg gtt ggc cag cta acc aag acc cag     2688
Thr Leu Val Thr Pro Val Gly Trp Val Gly Gln Leu Thr Lys Thr Gln
        820                 825                 830

ccg ctc aat tat tcg cca acg ttt aca aga atg cct ctt ctc cta gag     2736
Pro Leu Asn Tyr Ser Pro Thr Phe Thr Arg Met Pro Leu Leu Leu Glu
    835                 840                 845

cct tgc gtg gtt tgg cta cta acg tcg cta act aca acg gtt gga aca     2784
Pro Cys Val Val Trp Leu Leu Thr Ser Leu Thr Thr Thr Val Gly Thr
850                 855                 860                 865

tca ctt ctc cac cat ctt aca ccc aag gta acg ctg ttt aca acg aaa     2832
Ser Leu Leu His His Leu Thr Pro Lys Val Thr Leu Phe Thr Thr Lys
            870                 875                 880

agt tgt aca ttc acg cta tcg gtc cat tat tgg cta acc atg gtt ggt     2880
Ser Cys Thr Phe Thr Leu Ser Val His Tyr Trp Leu Thr Met Val Gly
        885                 890                 895

cta acg cct tct tca tca ccg acc aag gta gat ccg gta aac aac caa     2928
Leu Thr Pro Ser Ser Ser Pro Thr Lys Val Asp Pro Val Asn Asn Gln

900                 905                 910

ctg gtc aac aac aat ggg gtg att ggt gta acg tca tcg gta ctg gtt      2976
Leu Val Asn Asn Asn Gly Val Ile Gly Val Thr Ser Ser Val Leu Val
    915                 920                 925

tcg gta tca gac cat ccg cta aca ctg gtg att cct tgt tgg att cct      3024
Ser Val Ser Asp His Pro Leu Thr Leu Val Ile Pro Cys Trp Ile Pro
930                 935                 940                 945

tcg tct ggg tta agc cag gtg gtg aat gtg atg gca cct ctg att cct      3072
Ser Ser Gly Leu Ser Gln Val Val Asn Val Met Ala Pro Leu Ile Pro
                950                 955                 960

ctg ctc caa gat tcg att ccc act gcg cct tgc cag acg ctt tgc aac      3120
Leu Leu Gln Asp Ser Ile Pro Thr Ala Pro Cys Gln Thr Leu Cys Asn
            965                 970                 975

cag ccc cac aag ctg gtg cat ggt tcc aag ctt act ttg tcc aat tgt      3168
Gln Pro His Lys Leu Val His Gly Ser Lys Leu Thr Leu Ser Asn Cys
        980                 985                 990

tga cca acg cta acc cat ctt tct  tgg gat ccg gtg gcg  gtg gat ctg      3216
    Pro Thr Leu Thr His Leu Ser  Trp Asp Pro Val Ala  Val Asp Leu
        995                 1000                1005

gtg gag  gcg gtt ctc atc acc  acc atc atc acg gtg  gcg aaa act      3261
Val Glu  Ala Val Leu Ile Thr  Thr Ile Ile Thr Val  Ala Lys Thr
    1010                1015                1020

tgt act  ttc aag gcg gcg gtg  gag gta gtg gag gag  gtg gct ccg      3306
Cys Thr  Phe Lys Ala Ala Val  Glu Val Val Glu Glu  Val Ala Pro
    1025                1030                1035

gct cag  ct                                                          3314
Ala Gln
    1040


<210>  31
<211>  2996
<212>  DNA
<213>  Artificial Sequence

<220>
<223>  68 C-terminal AA from CWP2p


<220>
<221>  exon
<222>  (1)..(2996)

<400>  31
gag tcc cgg gca aca acc agg aac atc aac acc aga agt cca tcc aaa      48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga      96
Val Asn Asn Leu     Met Tyr     Glu Trp Arg Val Cys Ser Ala Gly
            20                      25                      30

cac aag tgt ggt ctt aga ctg gaa tta tcg ttg gat gca tga tgc caa      144
His Lys Cys Gly Leu Arg Leu Glu Leu Ser Leu Asp Ala     Cys Gln
                35                  40                      45

tta taa ttc ctg tac tgt taa cgg cgg tgt taa cac tac gtt atg ccc     192
Leu     Phe Leu Tyr Cys     Arg Arg Cys     His Tyr Val Met Pro
                50                          55

cga tga agc gac ttg tgg taa gaa ttg ttt tat tga agg ggt tga cta     240
Arg     Ser Asp Leu Trp     Glu Leu Phe Tyr     Arg Gly     Leu
        60                  65                              70

cgc cgc tag tgg tgt tac gac gag tgg gtc atc ctt gac gat gaa tca     288
Arg Arg     Trp Cys Tyr Asp Glu Trp Val Ile Leu Asp Asp Glu Ser
                75                  80                      85

ata cat gcc ttc ttc tag tgg tgg gta ttc ctc tgt gtc tcc aag gct     336
Ile His Ala Phe Phe     Trp Trp Val Phe Leu Cys Val Ser Lys Ala
                90                  95                      100

gta ttt att gga ttc cga tgg gga ata tgt tat gtt aaa att aaa tgg     384
Val Phe Ile Gly Phe Arg Trp Gly Ile Cys Tyr Val Lys Ile Lys Trp
            105                     110                 115

gca aga act gag ttt tga tgt gga tct atc tgc att acc ttg tgg aga     432
Ala Arg Thr Glu Phe     Cys Gly Ser Ile Cys Ile Thr Leu Trp Arg
        120                     125                     130

aaa tgg tag tct tta ttt atc aca aat gga cga aaa cgg cgg agc caa     480
Lys Trp     Ser Leu Phe Ile Thr Asn Gly Arg Lys Arg Arg Ser Gln
            135                     140                 145

tca gta caa tac agc tgg tgc taa tta tgg ttc agg cta ttg tga tgc     528
Ser Val Gln Tyr Ser Trp Cys     Leu Trp Phe Arg Leu Leu     Cys
        150                     155                         160

tca atg tcc agt gca gac ttg gag gaa tgg cac ctt aaa cac atc aca     576
Ser Met Ser Ser Ala Asp Leu Glu Glu Trp His Leu Lys His Ile Thr
            165                     170                 175

tca agg att ttg ctg taa cga aat gga cat att aga agg taa ttc aag     624
Ser Arg Ile Leu Leu     Arg Asn Gly His Ile Arg Arg     Phe Lys
        180                     185                         190

agc taa tgc act aac tcc gca ctc ttg tac tgc gag tcc cgg gca atc     672
Ser     Cys Thr Asn Ser Ala Leu Leu Tyr Cys Glu Ser Arg Ala Ile
                195                 200                     205

cgc ttg tac cct aca atc cga aac tca ccc acc att gac ctg gca aaa     720
Arg Leu Tyr Pro Thr Ile Arg Asn Ser Pro Thr Ile Asp Leu Ala Lys
            210                     215                 220

gtg ttc tag cgg tgg aac ttg tac tca aca aac tgg ttc tgt tgt tat     768
Val Phe     Arg Trp Asn Leu Tyr Ser Thr Asn Trp Phe Cys Cys Tyr
            225                     230                 235

cga cgc taa ctg gag atg gac aca cgc cac taa ctc ttc tac caa ctg     816
Arg Arg     Leu Glu Met Asp Thr Arg His     Leu Phe Tyr Gln Leu
            240                     245                     250

tta cga cgg taa cac ttg gtc ttc cac ttt atg tcc aga taa cga aac     864
Leu Arg Arg     His Leu Val Phe His Phe Met Ser Arg     Arg Asn
                255                 260

ttg tgc taa gaa ttg ctg ttt gga cgg tgc cgc cta cgc ttc tac cta     912
Leu Cys     Glu Leu Leu Phe Gly Arg Cys Arg Leu Arg Phe Tyr Leu
265                     270                 275

cgg tgt tac cac ctc cgg taa ctc ctt gtc tat tgg ttt cgt cac tca     960

Arg Cys Tyr His Leu Arg     Leu Leu Val Tyr Trp Phe Arg His Ser
280                 285                     290

atc cgc tca aaa gaa cgt tgg tgc tag att gta ctt gat ggc ttc tga     1008
Ile Arg Ser Lys Glu Arg Trp Cys     Ile Val Leu Asp Gly Phe
295                 300                     305

cac tac tta tca aga att tac ttt gtt ggg taa cga att ttc ttt cga     1056
His Tyr Leu Ser Arg Ile Tyr Phe Val Gly     Arg Ile Phe Phe Arg
    310                 315                     320

tgt tga cgt ttc cca att gcc atg tgg ctt gaa cgg tgc ttt gta ctt     1104
Cys     Arg Phe Pro Ile Ala Met Trp Leu Glu Arg Cys Phe Val Leu
        325                 330                     335

tgt ctc tat gga tgc tga cgg tgg tgt ttc taa gta ccc aac taa cac     1152
Cys Leu Tyr Gly Cys     Arg Trp Cys Phe     Val Pro Asn     His
    340                     345                     350

tgc cgg tgc taa gta cgg tac tgg tta ctg tga ttc tca atg tcc acg     1200
Cys Arg Cys     Val Arg Tyr Trp Leu Leu     Phe Ser Met Ser Thr
                355                 360                     365

tga ctt gaa gtt cat taa cgg tca agc caa cgt cga agg ttg gga acc     1248
    Leu Glu Val His     Arg Ser Ser Gln Arg Arg Arg Leu Gly Thr
                        370                 375

atc ctc caa caa cgc taa cac cgg tat cgg tgg tca cgg ttc ctg ttg     1296
Ile Leu Gln Gln Arg     His Arg Tyr Arg Trp Ser Arg Phe Leu Leu
380                     385                 390

ttc cga aat gga cat ctg gga agc taa cag tat ttc tga agc ttt gac     1344
Phe Arg Asn Gly His Leu Gly Ser     Gln Tyr Phe     Ser Phe Asp
395                 400                     405

acc aca ccc atg cac cac tgt cgg tca aga aat ttg tga agg tga tgg     1392
Thr Thr Pro Met His His Cys Arg Ser Arg Asn Leu     Arg     Trp
    410                 415                 420

atg tgg tgg aac cta ctc tga taa cag ata cgg tgg tac ttg tga ccc     1440
Met Trp Trp Asn Leu Leu         Gln Ile Arg Trp Tyr Leu     Pro
        425                         430                     435

aga cgg ttg tga ctg gaa ccc ata cag att ggg taa cac ttc ttt cta     1488
Arg Arg Leu     Leu Glu Pro Ile Gln Ile Gly     His Phe Phe Leu
                    440                 445

tgg tcc agg ttc ttc ttt cac ctt gga tac cac caa gaa gtt gac tgt     1536
Trp Ser Arg Phe Phe Phe His Leu Gly Tyr His Gln Glu Val Asp Cys
450                 455                 460                     465

tgt tac cca att cga aac ttc tgg tgc tat caa cag ata cta cgt tca     1584
Cys Tyr Pro Ile Arg Asn Phe Trp Cys Tyr Gln Gln Ile Leu Arg Ser
                470                 475                     480

aaa cgg tgt cac ctt cca aca acc aaa cgc tga att ggg ttc tta ctc     1632
Lys Arg Cys His Leu Pro Thr Thr Lys Arg     Ile Gly Phe Leu Leu
            485                 490                     495

tgg taa tga att gaa cga cga cta ctg tac cgc tga aga agc tga att     1680
Trp         Ile Glu Arg Arg Leu Leu Tyr Arg     Arg Ser     Ile
                    500                 505

tgg tgg ttc ctc ttt ctc cga caa ggg tgg ttt gac cca att caa gaa     1728
Trp Trp Phe Leu Phe Leu Arg Gln Gly Trp Phe Asp Pro Ile Gln Glu

510                 515                 520

ggc tac ctc cgg tgg tat ggt ttt ggt tat gtc ctt gtg gga tga tta      1776
Gly Tyr Leu Arg Trp Tyr Gly Phe Gly Tyr Val Leu Val Gly     Leu
525                 530                 535

cta cgc aaa cat gtt atg gtt aga cag tac tta ccc aac taa cga aac      1824
Leu Arg Lys His Val Met Val Arg Gln Tyr Leu Pro Asn     Arg Asn
540                 545                 550

cga gtc ccg ggg tcc cat tag aag aaa gac aag cct gct cct ctg ttt      1872
Arg Val Pro Gly Ser His     Lys Lys Asp Lys Pro Ala Pro Leu Phe
555                 560                     565

ggg gtc aat gtg gtg gtc aaa act ggt ctg gtc caa ctt gtt gtg ctt      1920
Gly Val Asn Val Val Val Lys Thr Gly Leu Val Gln Leu Val Val Leu
570                 575                 580                 585

ccg gtt cta cct gtg ttt act cca acg act act att ccc aat gtt tgc      1968
Pro Val Leu Pro Val Phe Thr Pro Thr Thr Thr Ile Pro Asn Val Cys
                590                 595                 600

cag gtg ctg ctt cct ctt cct ctt caa cta gag ctg ctt cta caa ctt      2016
Gln Val Leu Leu Pro Leu Pro Leu Gln Leu Glu Leu Leu Leu Gln Leu
            605                 610                 615

cta ggg tct ccc caa cca ctt cca gat cct ctt ctg cta ctc cac cac      2064
Leu Gly Ser Pro Gln Pro Leu Pro Asp Pro Leu Leu Leu Leu His His
        620                 625                 630

cag gtt cta cta cca cta gag ttc cac cag tcg gtt ccg gta ctg cta      2112
Gln Val Leu Leu Pro Leu Glu Phe His Gln Ser Val Pro Val Leu Leu
    635                 640                 645

ctt act ctg gta acc ctt tcg tcg gtg tta ctc cat ggg cta acg ctt      2160
Leu Thr Leu Val Thr Leu Ser Ser Val Leu Leu His Gly Leu Thr Leu
650                 655                 660                 665

act acg ctt ctg aag ttt ctt ctt tgg cta tcc cat ctt tga ctg gtg      2208
Thr Thr Leu Leu Lys Phe Leu Leu Trp Leu Ser His Leu     Leu Val
                670                 675                     680

cta tgg cta ccg ctg ctg ctg ctg tcg cca aag ttc cat cct tca tgt      2256
Leu Trp Leu Pro Leu Leu Leu Leu Ser Pro Lys Phe His Pro Ser Cys
                685                 690                 695

ggt tgg aca cct tgg aca aaa ctc cat taa tgg aac aaa cct tgg cag      2304
Gly Trp Thr Pro Trp Thr Lys Leu His     Trp Asn Lys Pro Trp Gln
            700                 705                     710

aca taa gga ctg cta aca aga acg gcg gta act acg ctg gtc aat ttg      2352
Thr     Gly Leu Leu Thr Arg Thr Ala Val Thr Thr Leu Val Asn Leu
                715                 720                 725

ttg tgt acg act tgc cag aca gag act gtg ctg ctt tgg ctt cca acg      2400
Leu Cys Thr Thr Cys Gln Thr Glu Thr Val Leu Leu Trp Leu Pro Thr
            730                 735                 740

gtg aat act cca tcg ctg acg gtg gtg tcg cca agt aca aga act aca      2448
Val Asn Thr Pro Ser Leu Thr Val Val Ser Pro Ser Thr Arg Thr Thr
        745                 750                 755

ttg ata cca tta gac aaa tcg ttg tcg aat act ctg aca tca gaa cct      2496
Leu Ile Pro Leu Asp Lys Ser Leu Ser Asn Thr Leu Thr Ser Glu Pro
    760                 765                 770

tgt tag tca tcg aac cag att ctt tag cca att tag tca cca act tgg     2544
Cys     Ser Ser Asn Gln Ile Leu     Pro Ile     Ser Pro Thr Trp
775                     780                     785

gta ctc caa agt gtg cta acg ctc aat ctg cct act tag aat gta tca     2592
Val Leu Gln Ser Val Leu Thr Leu Asn Leu Pro Thr     Asn Val Ser
        790                 795                     800

att atg cag tta ccc aat tga act tgc caa acg ttg gga tcc gga ggt     2640
Ile Met Gln Leu Pro Asn     Thr Cys Gln Thr Leu Gly Ser Gly Gly
        805                     810                 815

ggt tca gga ggt ggt ggg tct gct tgg cat cca caa ttt gga gga ggc     2688
Gly Ser Gly Gly Gly Gly Ser Ala Trp His Pro Gln Phe Gly Gly Gly
        820                 825                 830

ggt ggt gaa aat ctg tat ttc cag gga ggc gga ggt gat tac aag gat     2736
Gly Gly Glu Asn Leu Tyr Phe Gln Gly Gly Gly Gly Asp Tyr Lys Asp
    835                 840                 845

gac gac aaa gga ggt ggt gga tca gga ggt ggt ggc tcc ggc tca gct     2784
Asp Asp Lys Gly Gly Gly Gly Ser Gly Gly Gly Gly Ser Gly Ser Ala
850                 855                 860                 865

att agc caa ata act gat ggt caa ata caa gca act aca aca gca aca     2832
Ile Ser Gln Ile Thr Asp Gly Gln Ile Gln Ala Thr Thr Thr Ala Thr
                870                 875                 880

acc gaa gct act acc aca gcc gcg cct tct tca act gtt gag act gtt     2880
Thr Glu Ala Thr Thr Thr Ala Ala Pro Ser Ser Thr Val Glu Thr Val
            885                 890                 895

agt cct tcc tcc acg gaa acg att tct caa cag act gaa aac ggt gca     2928
Ser Pro Ser Ser Thr Glu Thr Ile Ser Gln Gln Thr Glu Asn Gly Ala
        900                 905                 910

gcc aaa gca gca gtc ggc atg ggt gcc gga gcc cta gca gct gca gca     2976
Ala Lys Ala Ala Val Gly Met Gly Ala Gly Ala Leu Ala Ala Ala Ala
    915                 920                 925

atg ctt ttg taa ggc gcg cc                                          2996
Met Leu Leu     Gly Ala
930

<210>  32
<211>  2776
<212>  DNA
<213>  Trichoderma reesei

<220>
<221>  exon
<222>  (1)..(2776)

<400>  32
gag tcc cgg gca aca acc agg aac atc aac acc aga agt cca tcc aaa       48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga       96
Val Asn Asn Leu     Met Tyr     Glu Trp Arg Val Cys Ser Ala Gly
            20                      25                  30

cac aag tgt ggt ctt aga ctg gaa tta tcg ttg gat gca tga tgc caa 144
His Lys Cys Gly Leu Arg Leu Glu Leu Ser Leu Asp Ala Cys Gln
                35                  40                  45

tta taa ttc ctg tac tgt taa cgg cgg tgt taa cac tac gtt atg ccc 192
Leu Phe Leu Tyr Cys Arg Arg Cys His Tyr Val Met Pro
                50                  55

cga tga agc gac ttg tgg taa gaa ttg ttt tat tga agg ggt tga cta 240
Arg Ser Asp Leu Trp Glu Leu Phe Tyr Arg Gly Leu
    60                  65                  70

cgc cgc tag tgg tgt tac gac gag tgg gtc atc ctt gac gat gaa tca 288
Arg Arg Trp Cys Tyr Asp Glu Trp Val Ile Leu Asp Asp Glu Ser
                75                  80                  85

ata cat gcc ttc ttc tag tgg tgg gta ttc ctc tgt gtc tcc aag gct 336
Ile His Ala Phe Phe Trp Trp Val Phe Leu Cys Val Ser Lys Ala
                90                  95                  100

gta ttt att gga ttc cga tgg gga ata tgt tat gtt aaa att aaa tgg 384
Val Phe Ile Gly Phe Arg Trp Gly Ile Cys Tyr Val Lys Ile Lys Trp
                105                 110                 115

gca aga act gag ttt tga tgt gga tct atc tgc att acc ttg tgg aga 432
Ala Arg Thr Glu Phe Cys Gly Ser Ile Cys Ile Thr Leu Trp Arg
            120                 125                 130

aaa tgg tag tct tta ttt atc aca aat gga cga aaa cgg cgg agc caa 480
Lys Trp Ser Leu Phe Ile Thr Asn Gly Arg Lys Arg Arg Ser Gln
            135                 140                 145

tca gta caa tac agc tgg tgc taa tta tgg ttc agg cta ttg tga tgc 528
Ser Val Gln Tyr Ser Trp Cys Leu Trp Phe Arg Leu Leu Cys
            150                 155                 160

tca atg tcc agt gca gac ttg gag gaa tgg cac ctt aaa cac atc aca 576
Ser Met Ser Ser Ala Asp Leu Glu Glu Trp His Leu Lys His Ile Thr
                165                 170                 175

tca agg att ttg ctg taa cga aat gga cat att aga agg taa ttc aag 624
Ser Arg Ile Leu Leu Arg Asn Gly His Ile Arg Arg Phe Lys
            180                 185                 190

agc taa tgc act aac tcc gca ctc ttg tac tgc gag tcc cgg gca atc 672
Ser Cys Thr Asn Ser Ala Leu Leu Tyr Cys Glu Ser Arg Ala Ile
                195                 200                 205

cgc ttg tac cct aca atc cga aac tca ccc acc att gac ctg gca aaa 720
Arg Leu Tyr Pro Thr Ile Arg Asn Ser Pro Thr Ile Asp Leu Ala Lys
                210                 215                 220

gtg ttc tag cgg tgg aac ttg tac tca aca aac tgg ttc tgt tgt tat 768
Val Phe Arg Trp Asn Leu Tyr Ser Thr Asn Trp Phe Cys Cys Tyr
            225                 230                 235

cga cgc taa ctg gag atg gac aca cgc cac taa ctc ttc tac caa ctg 816
Arg Arg Leu Glu Met Asp Thr Arg His Leu Phe Tyr Gln Leu
            240                 245                 250

tta cga cgg taa cac ttg gtc ttc cac ttt atg tcc aga taa cga aac 864
Leu Arg Arg His Leu Val Phe His Phe Met Ser Arg Arg Asn
                255                 260

ttg tgc taa gaa ttg ctg ttt gga cgg tgc cgc cta cgc ttc tac cta 912

Leu Cys     Glu Leu Leu Phe Gly Arg Cys Arg Leu Arg Phe Tyr Leu
265                 270                 275

cgg tgt tac cac ctc cgg taa ctc ctt gtc tat tgg ttt cgt cac tca     960
Arg Cys Tyr His Leu Arg     Leu Leu Val Tyr Trp Phe Arg His Ser
280                 285                 290

atc cgc tca aaa gaa cgt tgg tgc tag att gta ctt gat ggc ttc tga     1008
Ile Arg Ser Lys Glu Arg Trp Cys     Ile Val Leu Asp Gly Phe
295                 300                 305

cac tac tta tca aga att tac ttt gtt ggg taa cga att ttc ttt cga     1056
His Tyr Leu Ser Arg Ile Tyr Phe Val Gly     Arg Ile Phe Phe Arg
    310                 315                 320

tgt tga cgt ttc cca att gcc atg tgg ctt gaa cgg tgc ttt gta ctt     1104
Cys     Arg Phe Pro Ile Ala Met Trp Leu Glu Arg Cys Phe Val Leu
        325                 330                 335

tgt ctc tat gga tgc tga cgg tgg tgt ttc taa gta ccc aac taa cac     1152
Cys Leu Tyr Gly Cys     Arg Trp Cys Phe     Val Pro Asn     His
    340                 345                 350

tgc cgg tgc taa gta cgg tac tgg tta ctg tga ttc tca atg tcc acg     1200
Cys Arg Cys     Val Arg Tyr Trp Leu Leu     Phe Ser Met Ser Thr
                355                 360                 365

tga ctt gaa gtt cat taa cgg tca agc caa cgt cga agg ttg gga acc     1248
    Leu Glu Val His     Arg Ser Ser Gln Arg Arg Arg Leu Gly Thr
                        370                 375

atc ctc caa caa cgc taa cac cgg tat cgg tgg tca cgg ttc ctg ttg     1296
Ile Leu Gln Gln Arg     His Arg Tyr Arg Trp Ser Arg Phe Leu Leu
380                     385                 390

ttc cga aat gga cat ctg gga agc taa cag tat ttc tga agc ttt gac     1344
Phe Arg Asn Gly His Leu Gly Ser     Gln Tyr Phe     Ser Phe Asp
395                 400                 405

acc aca ccc atg cac cac tgt cgg tca aga aat ttg tga agg tga tgg     1392
Thr Thr Pro Met His His Cys Arg Ser Arg Asn Leu     Arg     Trp
    410                 415                 420

atg tgg tgg aac cta ctc tga taa cag ata cgg tgg tac ttg tga ccc     1440
Met Trp Trp Asn Leu Leu         Gln Ile Arg Trp Tyr Leu     Pro
        425                     430                 435

aga cgg ttg tga ctg gaa ccc ata cag att ggg taa cac ttc ttt cta     1488
Arg Arg Leu     Leu Glu Pro Ile Gln Ile Gly     His Phe Phe Leu
                    440                 445

tgg tcc agg ttc ttc ttt cac ctt gga tac cac caa gaa gtt gac tgt     1536
Trp Ser Arg Phe Phe Phe His Leu Gly Tyr His Gln Glu Val Asp Cys
450                 455                 460                 465

tgt tac cca att cga aac ttc tgg tgc tat caa cag ata cta cgt tca     1584
Cys Tyr Pro Ile Arg Asn Phe Trp Cys Tyr Gln Gln Ile Leu Arg Ser
                470                 475                 480

aaa cgg tgt cac ctt cca aca acc aaa cgc tga att ggg ttc tta ctc     1632
Lys Arg Cys His Leu Pro Thr Thr Lys Arg     Ile Gly Phe Leu Leu
            485                 490                 495

tgg taa tga att gaa cga cga cta ctg tac cgc tga aga agc tga att     1680
Trp         Ile Glu Arg Arg Leu Leu Tyr Arg     Arg Ser     Ile

500                 505

tgg tgg ttc ctc ttt ctc cga caa ggg tgg ttt gac cca att caa gaa     1728
Trp Trp Phe Leu Phe Leu Arg Gln Gly Trp Phe Asp Pro Ile Gln Glu
    510                 515                 520

ggc tac ctc cgg tgg tat ggt ttt ggt tat gtc ctt gtg gga tga tta     1776
Gly Tyr Leu Arg Trp Tyr Gly Phe Gly Tyr Val Leu Val Gly     Leu
525                 530                 535

cta cgc aaa cat gtt atg gtt aga cag tac tta ccc aac taa cga aac     1824
Leu Arg Lys His Val Met Val Arg Gln Tyr Leu Pro Asn     Arg Asn
540                 545                 550

cga gtc ccg ggg tcc cat tag aag aaa gac aag cct gct cct ctg ttt     1872
Arg Val Pro Gly Ser His     Lys Lys Asp Lys Pro Ala Pro Leu Phe
555                 560                     565

ggg gtc aat gtg gtg gtc aaa act ggt ctg gtc caa ctt gtt gtg ctt     1920
Gly Val Asn Val Val Val Lys Thr Gly Leu Val Gln Leu Val Val Leu
570                 575                 580                 585

ccg gtt cta cct gtg ttt act cca acg act act att ccc aat gtt tgc     1968
Pro Val Leu Pro Val Phe Thr Pro Thr Thr Thr Ile Pro Asn Val Cys
                590                 595                 600

cag gtg ctg ctt cct ctt cct ctt caa cta gag ctg ctt cta caa ctt     2016
Gln Val Leu Leu Pro Leu Pro Leu Gln Leu Glu Leu Leu Leu Gln Leu
            605                 610                 615

cta ggg tct ccc caa cca ctt cca gat cct ctt ctg cta ctc cac cac     2064
Leu Gly Ser Pro Gln Pro Leu Pro Asp Pro Leu Leu Leu Leu His His
        620                 625                 630

cag gtt cta cta cca cta gag ttc cac cag tcg gtt ccg gta ctg cta     2112
Gln Val Leu Leu Pro Leu Glu Phe His Gln Ser Val Pro Val Leu Leu
    635                 640                 645

ctt act ctg gta acc ctt tcg tcg gtg tta ctc cat ggg cta acg ctt     2160
Leu Thr Leu Val Thr Leu Ser Ser Val Leu Leu His Gly Leu Thr Leu
650                 655                 660                 665

act acg ctt ctg aag ttt ctt ctt tgg cta tcc cat ctt tga ctg gtg     2208
Thr Thr Leu Leu Lys Phe Leu Leu Trp Leu Ser His Leu     Leu Val
                670                 675                     680

cta tgg cta ccg ctg ctg ctg ctg tcg cca aag ttc cat cct tca tgt     2256
Leu Trp Leu Pro Leu Leu Leu Leu Ser Pro Lys Phe His Pro Ser Cys
            685                 690                 695

ggt tgg aca cct tgg aca aaa ctc cat taa tgg aac aaa cct tgg cag     2304
Gly Trp Thr Pro Trp Thr Lys Leu His     Trp Asn Lys Pro Trp Gln
        700                 705                     710

aca taa gga ctg cta aca aga acg gcg gta act acg ctg gtc aat ttg     2352
Thr     Gly Leu Leu Thr Arg Thr Ala Val Thr Thr Leu Val Asn Leu
            715                 720                 725

ttg tgt acg act tgc cag aca gag act gtg ctg ctt tgg ctt cca acg     2400
Leu Cys Thr Thr Cys Gln Thr Glu Thr Val Leu Leu Trp Leu Pro Thr
        730                 735                 740

gtg aat act cca tcg ctg acg gtg gtg tcg cca agt aca aga act aca     2448
Val Asn Thr Pro Ser Leu Thr Val Val Ser Pro Ser Thr Arg Thr Thr
    745                 750                 755

ttg ata cca tta gac aaa tcg ttg tcg aat act ctg aca tca gaa cct     2496
Leu Ile Pro Leu Asp Lys Ser Leu Ser Asn Thr Leu Thr Ser Glu Pro
    760                 765                 770

tgt tag tca tcg aac cag att ctt tag cca att tag tca cca act tgg     2544
Cys     Ser Ser Asn Gln Ile Leu     Pro Ile     Ser Pro Thr Trp
775                     780                     785

gta ctc caa agt gtg cta acg ctc aat ctg cct act tag aat gta tca     2592
Val Leu Gln Ser Val Leu Thr Leu Asn Leu Pro Thr     Asn Val Ser
        790                 795                     800

att atg cag tta ccc aat tga act tgc caa acg ttg gaa ttc tta att     2640
Ile Met Gln Leu Pro Asn     Thr Cys Gln Thr Leu Glu Phe Leu Ile
        805                     810                 815

aaa aac aaa atg gtc tcc ttc acc tcc ctg ctg gcc ggc gtt gcc gct     2688
Lys Asn Lys Met Val Ser Phe Thr Ser Leu Leu Ala Gly Val Ala Ala
        820                 825                 830

atc tct ggt gtc cta gca gcc cct gcc gca gaa gtt gaa cct gtc gca     2736
Ile Ser Gly Val Leu Ala Ala Pro Ala Ala Glu Val Glu Pro Val Ala
    835                 840                 845

gtt gag aaa cgt gag gcc gaa gca gaa gct ccc ggg act c               2776
Val Glu Lys Arg Glu Ala Glu Ala Glu Ala Pro Gly Thr
850                 855                 860


<210>  33
<211>  2932
<212>  DNA
<213>  Artificial Sequence

<220>
<223>  Mfalpha pre-pro secretion signal and spacer


<220>
<221>  exon
<222>  (1)..(2932)

<400>  33
gag tcc cgg gca aca acc agg aac atc aac acc aga agt cca tcc aaa       48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga       96
Val Asn Asn Leu     Met Tyr     Glu Trp Arg Val Cys Ser Ala Gly
            20                      25                      30

cac aag tgt ggt ctt aga ctg gaa tta tcg ttg gat gca tga tgc caa      144
His Lys Cys Gly Leu Arg Leu Glu Leu Ser Leu Asp Ala     Cys Gln
                35                  40                          45

tta taa ttc ctg tac tgt taa cgg cgg tgt taa cac tac gtt atg ccc      192
Leu     Phe Leu Tyr Cys     Arg Arg Cys     His Tyr Val Met Pro
                50                              55

cga tga agc gac ttg tgg taa gaa ttg ttt tat tga agg ggt tga cta      240
Arg     Ser Asp Leu Trp     Glu Leu Phe Tyr     Arg Gly     Leu
        60                      65                          70

cgc cgc tag tgg tgt tac gac gag tgg gtc atc ctt gac gat gaa tca      288

Arg Arg     Trp Cys Tyr Asp Glu Trp Val Ile Leu Asp Asp Glu Ser
                    75                  80                  85

ata cat gcc ttc ttc tag tgg tgg gta ttc ctc tgt gtc tcc aag gct      336
Ile His Ala Phe Phe     Trp Trp Val Phe Leu Cys Val Ser Lys Ala
                90                  95                  100

gta ttt att gga ttc cga tgg gga ata tgt tat gtt aaa att aaa tgg      384
Val Phe Ile Gly Phe Arg Trp Gly Ile Cys Tyr Val Lys Ile Lys Trp
            105                 110                 115

gca aga act gag ttt tga tgt gga tct atc tgc att acc ttg tgg aga      432
Ala Arg Thr Glu Phe     Cys Gly Ser Ile Cys Ile Thr Leu Trp Arg
        120                     125                 130

aaa tgg tag tct tta ttt atc aca aat gga cga aaa cgg cgg agc caa      480
Lys Trp     Ser Leu Phe Ile Thr Asn Gly Arg Lys Arg Arg Ser Gln
            135                 140                 145

tca gta caa tac agc tgg tgc taa tta tgg ttc agg cta ttg tga tgc      528
Ser Val Gln Tyr Ser Trp Cys     Leu Trp Phe Arg Leu Leu     Cys
        150                     155                     160

tca atg tcc agt gca gac ttg gag gaa tgg cac ctt aaa cac atc aca      576
Ser Met Ser Ser Ala Asp Leu Glu Glu Trp His Leu Lys His Ile Thr
            165                 170                 175

tca agg att ttg ctg taa cga aat gga cat att aga agg taa ttc aag      624
Ser Arg Ile Leu Leu     Arg Asn Gly His Ile Arg Arg     Phe Lys
        180                     185                     190

agc taa tgc act aac tcc gca ctc ttg tac tgc gag tcc cgg gca atc      672
Ser     Cys Thr Asn Ser Ala Leu Leu Tyr Cys Glu Ser Arg Ala Ile
                    195                 200                 205

cgc ttg tac cct aca atc cga aac tca ccc acc att gac ctg gca aaa      720
Arg Leu Tyr Pro Thr Ile Arg Asn Ser Pro Thr Ile Asp Leu Ala Lys
            210                 215                 220

gtg ttc tag cgg tgg aac ttg tac tca aca aac tgg ttc tgt tgt tat      768
Val Phe     Arg Trp Asn Leu Tyr Ser Thr Asn Trp Phe Cys Cys Tyr
                225                 230                 235

cga cgc taa ctg gag atg gac aca cgc cac taa ctc ttc tac caa ctg      816
Arg Arg     Leu Glu Met Asp Thr Arg His     Leu Phe Tyr Gln Leu
                240                 245                     250

tta cga cgg taa cac ttg gtc ttc cac ttt atg tcc aga taa cga aac      864
Leu Arg Arg     His Leu Val Phe His Phe Met Ser Arg     Arg Asn
                    255                 260

ttg tgc taa gaa ttg ctg ttt gga cgg tgc cgc cta cgc ttc tac cta      912
Leu Cys     Glu Leu Leu Phe Gly Arg Cys Arg Leu Arg Phe Tyr Leu
265                     270                 275

cgg tgt tac cac ctc cgg taa ctc ctt gtc tat tgg ttt cgt cac tca      960
Arg Cys Tyr His Leu Arg     Leu Leu Val Tyr Trp Phe Arg His Ser
280                 285                     290

atc cgc tca aaa gaa cgt tgg tgc tag att gta ctt gat ggc ttc tga     1008
Ile Arg Ser Lys Glu Arg Trp Cys     Ile Val Leu Asp Gly Phe
295                 300                     305

cac tac tta tca aga att tac ttt gtt ggg taa cga att ttc ttt cga     1056
His Tyr Leu Ser Arg Ile Tyr Phe Val Gly     Arg Ile Phe Phe Arg

310                 315                 320

tgt tga cgt ttc cca att gcc atg tgg ctt gaa cgg tgc ttt gta ctt      1104
Cys     Arg Phe Pro Ile Ala Met Trp Leu Glu Arg Cys Phe Val Leu
        325                 330                 335

tgt ctc tat gga tgc tga cgg tgg tgt ttc taa gta ccc aac taa cac      1152
Cys Leu Tyr Gly Cys     Arg Trp Cys Phe     Val Pro Asn     His
    340                 345                 350

tgc cgg tgc taa gta cgg tac tgg tta ctg tga ttc tca atg tcc acg      1200
Cys Arg Cys     Val Arg Tyr Trp Leu Leu     Phe Ser Met Ser Thr
            355                 360                 365

tga ctt gaa gtt cat taa cgg tca agc caa cgt cga agg ttg gga acc      1248
    Leu Glu Val His     Arg Ser Ser Gln Arg Arg Arg Leu Gly Thr
                        370                 375

atc ctc caa caa cgc taa cac cgg tat cgg tgg tca cgg ttc ctg ttg      1296
Ile Leu Gln Gln Arg     His Arg Tyr Arg Trp Ser Arg Phe Leu Leu
380                     385                 390

ttc cga aat gga cat ctg gga agc taa cag tat ttc tga agc ttt gac      1344
Phe Arg Asn Gly His Leu Gly Ser     Gln Tyr Phe     Ser Phe Asp
395                 400                 405

acc aca ccc atg cac cac tgt cgg tca aga aat ttg tga agg tga tgg      1392
Thr Thr Pro Met His His Cys Arg Ser Arg Asn Leu     Arg     Trp
    410                 415                 420

atg tgg tgg aac cta ctc tga taa cag ata cgg tgg tac ttg tga ccc      1440
Met Trp Trp Asn Leu Leu         Gln Ile Arg Trp Tyr Leu     Pro
    425                             430                     435

aga cgg ttg tga ctg gaa ccc ata cag att ggg taa cac ttc ttt cta      1488
Arg Arg Leu     Leu Glu Pro Ile Gln Ile Gly     His Phe Phe Leu
                440                 445

tgg tcc agg ttc ttc ttt cac ctt gga tac cac caa gaa gtt gac tgt      1536
Trp Ser Arg Phe Phe Phe His Leu Gly Tyr His Gln Glu Val Asp Cys
450                 455                 460                 465

tgt tac cca att cga aac ttc tgg tgc tat caa cag ata cta cgt tca      1584
Cys Tyr Pro Ile Arg Asn Phe Trp Cys Tyr Gln Gln Ile Leu Arg Ser
            470                 475                 480

aaa cgg tgt cac ctt cca aca acc aaa cgc tga att ggg ttc tta ctc      1632
Lys Arg Cys His Leu Pro Thr Thr Lys Arg     Ile Gly Phe Leu Leu
        485                 490                 495

tgg taa tga att gaa cga cga cta ctg tac cgc tga aga agc tga att      1680
Trp         Ile Glu Arg Arg Leu Leu Tyr Arg     Arg Ser     Ile
                500                 505

tgg tgg ttc ctc ttt ctc cga caa ggg tgg ttt gac cca att caa gaa      1728
Trp Trp Phe Leu Phe Leu Arg Gln Gly Trp Phe Asp Pro Ile Gln Glu
    510                 515                 520

ggc tac ctc cgg tgg tat ggt ttt ggt tat gtc ctt gtg gga tga tta      1776
Gly Tyr Leu Arg Trp Tyr Gly Phe Gly Tyr Val Leu Val Gly     Leu
525                 530                 535

cta cgc aaa cat gtt atg gtt aga cag tac tta ccc aac taa cga aac      1824
Leu Arg Lys His Val Met Val Arg Gln Tyr Leu Pro Asn     Arg Asn
540                 545                 550

cga gtc ccg ggg tcc cat tag aag aaa gac aag cct gct cct ctg ttt 1872
Arg Val Pro Gly Ser His Lys Lys Asp Lys Pro Ala Pro Leu Phe
555                 560                 565

ggg gtc aat gtg gtg gtc aaa act ggt ctg gtc caa ctt gtt gtg ctt 1920
Gly Val Asn Val Val Val Lys Thr Gly Leu Val Gln Leu Val Val Leu
570                 575                 580                 585

ccg gtt cta cct gtg ttt act cca acg act act att ccc aat gtt tgc 1968
Pro Val Leu Pro Val Phe Thr Pro Thr Thr Thr Ile Pro Asn Val Cys
                590                 595                 600

cag gtg ctg ctt cct ctt cct ctt caa cta gag ctg ctt cta caa ctt 2016
Gln Val Leu Leu Pro Leu Pro Leu Gln Leu Glu Leu Leu Leu Gln Leu
            605                 610                 615

cta ggg tct ccc caa cca ctt cca gat cct ctt ctg cta ctc cac cac 2064
Leu Gly Ser Pro Gln Pro Leu Pro Asp Pro Leu Leu Leu Leu His His
        620                 625                 630

cag gtt cta cta cca cta gag ttc cac cag tcg gtt ccg gta ctg cta 2112
Gln Val Leu Leu Pro Leu Glu Phe His Gln Ser Val Pro Val Leu Leu
    635                 640                 645

ctt act ctg gta acc ctt tcg tcg gtg tta ctc cat ggg cta acg ctt 2160
Leu Thr Leu Val Thr Leu Ser Ser Val Leu Leu His Gly Leu Thr Leu
650                 655                 660                 665

act acg ctt ctg aag ttt ctt ctt tgg cta tcc cat ctt tga ctg gtg 2208
Thr Thr Leu Leu Lys Phe Leu Leu Trp Leu Ser His Leu Leu Val
                670                 675                 680

cta tgg cta ccg ctg ctg ctg ctg tcg cca aag ttc cat cct tca tgt 2256
Leu Trp Leu Pro Leu Leu Leu Leu Ser Pro Lys Phe His Pro Ser Cys
                685                 690                 695

ggt tgg aca cct tgg aca aaa ctc cat taa tgg aac aaa cct tgg cag 2304
Gly Trp Thr Pro Trp Thr Lys Leu His Trp Asn Lys Pro Trp Gln
            700                 705                 710

aca taa gga ctg cta aca aga acg gcg gta act acg ctg gtc aat ttg 2352
Thr Gly Leu Leu Thr Arg Thr Ala Val Thr Thr Leu Val Asn Leu
            715                 720                 725

ttg tgt acg act tgc cag aca gag act gtg ctg ctt tgg ctt cca acg 2400
Leu Cys Thr Thr Cys Gln Thr Glu Thr Val Leu Leu Trp Leu Pro Thr
            730                 735                 740

gtg aat act cca tcg ctg acg gtg gtg tcg cca agt aca aga act aca 2448
Val Asn Thr Pro Ser Leu Thr Val Val Ser Pro Ser Thr Arg Thr Thr
        745                 750                 755

ttg ata cca tta gac aaa tcg ttg tcg aat act ctg aca tca gaa cct 2496
Leu Ile Pro Leu Asp Lys Ser Leu Ser Asn Thr Leu Thr Ser Glu Pro
    760                 765                 770

tgt tag tca tcg aac cag att ctt tag cca att tag tca cca act tgg 2544
Cys Ser Ser Asn Gln Ile Leu Pro Ile Ser Pro Thr Trp
775                 780                 785

gta ctc caa agt gtg cta acg ctc aat ctg cct act tag aat gta tca 2592
Val Leu Gln Ser Val Leu Thr Leu Asn Leu Pro Thr Asn Val Ser
        790                 795                 800

att atg cag tta ccc aat tga act tgc caa acg ttg gaa ttc tta att     2640
Ile Met Gln Leu Pro Asn     Thr Cys Gln Thr Leu Glu Phe Leu Ile
        805                 810                 815

aaa aac aaa atg aga ttt cca tca ata ttt aca gca gtt ttg ttt gcg     2688
Lys Asn Lys Met Arg Phe Pro Ser Ile Phe Thr Ala Val Leu Phe Ala
        820                 825                 830

gcg agt tca gcc ctt gca gca ccc gtc aat acc acg acg gag gat gag     2736
Ala Ser Ser Ala Leu Ala Ala Pro Val Asn Thr Thr Thr Glu Asp Glu
    835                 840                 845

aca gcc cag atc cca gca gag gct gtg ata gga tat tta gac ctg gaa     2784
Thr Ala Gln Ile Pro Ala Glu Ala Val Ile Gly Tyr Leu Asp Leu Glu
850                 855                 860                 865

ggc gat ttt gat gtg gcc gta tta ccg ttt tct aac tct acg aat aat     2832
Gly Asp Phe Asp Val Ala Val Leu Pro Phe Ser Asn Ser Thr Asn Asn
                870                 875                 880

gga ttg tta ttt att aat act aca att gcc tct ata gcc gca aag gaa     2880
Gly Leu Leu Phe Ile Asn Thr Thr Ile Ala Ser Ile Ala Ala Lys Glu
            885                 890                 895

gaa ggg gtg tct tta gat aag aga gaa gct gag gct gaa gcc ccc ggg     2928
Glu Gly Val Ser Leu Asp Lys Arg Glu Ala Glu Ala Glu Ala Pro Gly
        900                 905                 910

act c                                                               2932
Thr

<210>  34
<211>  2812
<212>  DNA
<213>  Artificial Sequence

<220>
<223>  Hybrid killer toxin

<220>
<221>  exon
<222>  (1)..(2812)

<400>  34
gag tcc cgg gca aca acc agg aac atc aac acc aga agt cca tcc aaa      48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga      96
Val Asn Asn Leu     Met Tyr     Glu Trp Arg Val Cys Ser Ala Gly
            20                      25                  30

cac aag tgt ggt ctt aga ctg gaa tta tcg ttg gat gca tga tgc caa     144
His Lys Cys Gly Leu Arg Leu Glu Leu Ser Leu Asp Ala     Cys Gln
                35                  40                      45

tta taa ttc ctg tac tgt taa cgg cgg tgt taa cac tac gtt atg ccc     192
Leu     Phe Leu Tyr Cys     Arg Arg Cys     His Tyr Val Met Pro
                    50                          55

cga tga agc gac ttg tgg taa gaa ttg ttt tat tga agg ggt tga cta     240
Arg     Ser Asp Leu Trp     Glu Leu Phe Tyr     Arg Gly     Leu

60                  65                  70

cgc cgc tag tgg tgt tac gac gag tgg gtc atc ctt gac gat gaa tca      288
Arg Arg     Trp Cys Tyr Asp Glu Trp Val Ile Leu Asp Asp Glu Ser
                75                  80                  85

ata cat gcc ttc ttc tag tgg tgg gta ttc ctc tgt gtc tcc aag gct      336
Ile His Ala Phe Phe     Trp Trp Val Phe Leu Cys Val Ser Lys Ala
                90                  95                  100

gta ttt att gga ttc cga tgg gga ata tgt tat gtt aaa att aaa tgg      384
Val Phe Ile Gly Phe Arg Trp Gly Ile Cys Tyr Val Lys Ile Lys Trp
            105                 110                 115

gca aga act gag ttt tga tgt gga tct atc tgc att acc ttg tgg aga      432
Ala Arg Thr Glu Phe     Cys Gly Ser Ile Cys Ile Thr Leu Trp Arg
        120                 125                 130

aaa tgg tag tct tta ttt atc aca aat gga cga aaa cgg cgg agc caa      480
Lys Trp     Ser Leu Phe Ile Thr Asn Gly Arg Lys Arg Arg Ser Gln
            135                 140                 145

tca gta caa tac agc tgg tgc taa tta tgg ttc agg cta ttg tga tgc      528
Ser Val Gln Tyr Ser Trp Cys     Leu Trp Phe Arg Leu Leu     Cys
        150                 155                 160

tca atg tcc agt gca gac ttg gag gaa tgg cac ctt aaa cac atc aca      576
Ser Met Ser Ser Ala Asp Leu Glu Glu Trp His Leu Lys His Ile Thr
            165                 170                 175

tca agg att ttg ctg taa cga aat gga cat att aga agg taa ttc aag      624
Ser Arg Ile Leu Leu     Arg Asn Gly His Ile Arg Arg     Phe Lys
        180                 185                 190

agc taa tgc act aac tcc gca ctc ttg tac tgc gag tcc cgg gca atc      672
Ser     Cys Thr Asn Ser Ala Leu Leu Tyr Cys Glu Ser Arg Ala Ile
            195                 200                 205

cgc ttg tac cct aca atc cga aac tca ccc acc att gac ctg gca aaa      720
Arg Leu Tyr Pro Thr Ile Arg Asn Ser Pro Thr Ile Asp Leu Ala Lys
            210                 215                 220

gtg ttc tag cgg tgg aac ttg tac tca aca aac tgg ttc tgt tgt tat      768
Val Phe     Arg Trp Asn Leu Tyr Ser Thr Asn Trp Phe Cys Cys Tyr
            225                 230                 235

cga cgc taa ctg gag atg gac aca cgc cac taa ctc ttc tac caa ctg      816
Arg Arg     Leu Glu Met Asp Thr Arg His     Leu Phe Tyr Gln Leu
            240                 245                 250

tta cga cgg taa cac ttg gtc ttc cac ttt atg tcc aga taa cga aac      864
Leu Arg Arg     His Leu Val Phe His Phe Met Ser Arg     Arg Asn
                255                 260

ttg tgc taa gaa ttg ctg ttt gga cgg tgc cgc cta cgc ttc tac cta      912
Leu Cys     Glu Leu Leu Phe Gly Arg Cys Arg Leu Arg Phe Tyr Leu
265                 270                 275

cgg tgt tac cac ctc cgg taa ctc ctt gtc tat tgg ttt cgt cac tca      960
Arg Cys Tyr His Leu Arg     Leu Leu Val Tyr Trp Phe Arg His Ser
280                 285                 290

atc cgc tca aaa gaa cgt tgg tgc tag att gta ctt gat ggc ttc tga      1008
Ile Arg Ser Lys Glu Arg Trp Cys     Ile Val Leu Asp Gly Phe
295                 300                 305

cac tac tta tca aga att tac ttt gtt ggg taa cga att ttc ttt cga 1056
His Tyr Leu Ser Arg Ile Tyr Phe Val Gly Arg Ile Phe Phe Arg
    310                 315                 320

tgt tga cgt ttc cca att gcc atg tgg ctt gaa cgg tgc ttt gta ctt 1104
Cys Arg Phe Pro Ile Ala Met Trp Leu Glu Arg Cys Phe Val Leu
    325                 330                 335

tgt ctc tat gga tgc tga cgg tgg tgt ttc taa gta ccc aac taa cac 1152
Cys Leu Tyr Gly Cys Arg Trp Cys Phe Val Pro Asn His
    340                 345                 350

tgc cgg tgc taa gta cgg tac tgg tta ctg tga ttc tca atg tcc acg 1200
Cys Arg Cys Val Arg Tyr Trp Leu Leu Phe Ser Met Ser Thr
            355                 360                 365

tga ctt gaa gtt cat taa cgg tca agc caa cgt cga agg ttg gga acc 1248
Leu Glu Val His Arg Ser Ser Gln Arg Arg Arg Leu Gly Thr
                370                 375

atc ctc caa caa cgc taa cac cgg tat cgg tgg tca cgg ttc ctg ttg 1296
Ile Leu Gln Gln Arg His Arg Tyr Arg Trp Ser Arg Phe Leu Leu
380                 385                 390

ttc cga aat gga cat ctg gga agc taa cag tat ttc tga agc ttt gac 1344
Phe Arg Asn Gly His Leu Gly Ser Gln Tyr Phe Ser Phe Asp
395                 400                 405

acc aca ccc atg cac cac tgt cgg tca aga aat ttg tga agg tga tgg 1392
Thr Thr Pro Met His His Cys Arg Ser Arg Asn Leu Arg Trp
    410                 415                 420

atg tgg tgg aac cta ctc tga taa cag ata cgg tgg tac ttg tga ccc 1440
Met Trp Trp Asn Leu Leu Gln Ile Arg Trp Tyr Leu Pro
        425                 430                 435

aga cgg ttg tga ctg gaa ccc ata cag att ggg taa cac ttc ttt cta 1488
Arg Arg Leu Leu Glu Pro Ile Gln Ile Gly His Phe Phe Leu
                440                 445

tgg tcc agg ttc ttc ttt cac ctt gga tac cac caa gaa gtt gac tgt 1536
Trp Ser Arg Phe Phe Phe His Leu Gly Tyr His Gln Glu Val Asp Cys
450                 455                 460                 465

tgt tac cca att cga aac ttc tgg tgc tat caa cag ata cta cgt tca 1584
Cys Tyr Pro Ile Arg Asn Phe Trp Cys Tyr Gln Gln Ile Leu Arg Ser
                470                 475                 480

aaa cgg tgt cac ctt cca aca acc aaa cgc tga att ggg ttc tta ctc 1632
Lys Arg Cys His Leu Pro Thr Thr Lys Arg Ile Gly Phe Leu Leu
            485                 490                 495

tgg taa tga att gaa cga cga cta ctg tac cgc tga aga agc tga att 1680
Trp Ile Glu Arg Arg Leu Leu Tyr Arg Arg Ser Ile
            500                 505

tgg tgg ttc ctc ttt ctc cga caa ggg tgg ttt gac cca att caa gaa 1728
Trp Trp Phe Leu Phe Leu Arg Gln Gly Trp Phe Asp Pro Ile Gln Glu
    510                 515                 520

ggc tac ctc cgg tgg tat ggt ttt ggt tat gtc ctt gtg gga tga tta 1776
Gly Tyr Leu Arg Trp Tyr Gly Phe Gly Tyr Val Leu Val Gly Leu
525                 530                 535

cta cgc aaa cat gtt atg gtt aga cag tac tta ccc aac taa cga aac 1824
Leu Arg Lys His Val Met Val Arg Gln Tyr Leu Pro Asn Arg Asn
540                 545                 550

cga gtc ccg ggg tcc cat tag aag aaa gac aag cct gct cct ctg ttt 1872
Arg Val Pro Gly Ser His Lys Lys Asp Lys Pro Ala Pro Leu Phe
555                 560                 565

ggg gtc aat gtg gtg gtc aaa act ggt ctg gtc caa ctt gtt gtg ctt 1920
Gly Val Asn Val Val Val Lys Thr Gly Leu Val Gln Leu Val Val Leu
570                 575                 580                 585

ccg gtt cta cct gtg ttt act cca acg act act att ccc aat gtt tgc 1968
Pro Val Leu Pro Val Phe Thr Pro Thr Thr Thr Ile Pro Asn Val Cys
                590                 595                 600

cag gtg ctg ctt cct ctt cct ctt caa cta gag ctg ctt cta caa ctt 2016
Gln Val Leu Leu Pro Leu Pro Leu Gln Leu Glu Leu Leu Leu Gln Leu
            605                 610                 615

cta ggg tct ccc caa cca ctt cca gat cct ctt ctg cta ctc cac cac 2064
Leu Gly Ser Pro Gln Pro Leu Pro Asp Pro Leu Leu Leu Leu His His
        620                 625                 630

cag gtt cta cta cca cta gag ttc cac cag tcg gtt ccg gta ctg cta 2112
Gln Val Leu Leu Pro Leu Glu Phe His Gln Ser Val Pro Val Leu Leu
    635                 640                 645

ctt act ctg gta acc ctt tcg tcg gtg tta ctc cat ggg cta acg ctt 2160
Leu Thr Leu Val Thr Leu Ser Ser Val Leu Leu His Gly Leu Thr Leu
650                 655                 660                 665

act acg ctt ctg aag ttt ctt ctt tgg cta tcc cat ctt tga ctg gtg 2208
Thr Thr Leu Leu Lys Phe Leu Leu Trp Leu Ser His Leu Leu Val
                670                 675                 680

cta tgg cta ccg ctg ctg ctg ctg tcg cca aag ttc cat cct tca tgt 2256
Leu Trp Leu Pro Leu Leu Leu Leu Ser Pro Lys Phe His Pro Ser Cys
                685                 690                 695

ggt tgg aca cct tgg aca aaa ctc cat taa tgg aac aaa cct tgg cag 2304
Gly Trp Thr Pro Trp Thr Lys Leu His Trp Asn Lys Pro Trp Gln
            700                 705                 710

aca taa gga ctg cta aca aga acg gcg gta act acg ctg gtc aat ttg 2352
Thr Gly Leu Leu Thr Arg Thr Ala Val Thr Thr Leu Val Asn Leu
            715                 720                 725

ttg tgt acg act tgc cag aca gag act gtg ctg ctt tgg ctt cca acg 2400
Leu Cys Thr Thr Cys Gln Thr Glu Thr Val Leu Leu Trp Leu Pro Thr
            730                 735                 740

gtg aat act cca tcg ctg acg gtg gtg tcg cca agt aca aga act aca 2448
Val Asn Thr Pro Ser Leu Thr Val Val Ser Pro Ser Thr Arg Thr Thr
        745                 750                 755

ttg ata cca tta gac aaa tcg ttg tcg aat act ctg aca tca gaa cct 2496
Leu Ile Pro Leu Asp Lys Ser Leu Ser Asn Thr Leu Thr Ser Glu Pro
    760                 765                 770

tgt tag tca tcg aac cag att ctt tag cca att tag tca cca act tgg 2544
Cys Ser Ser Asn Gln Ile Leu Pro Ile Ser Pro Thr Trp
775                 780                 785

gta ctc caa agt gtg cta acg ctc aat ctg cct act tag aat gta tca 2592

Val Leu Gln Ser Val Leu Thr Leu Asn Leu Pro Thr     Asn Val Ser
        790                 795                     800

att atg cag tta ccc aat tga act tgc caa acg ttg gaa ttc tta att      2640
Ile Met Gln Leu Pro Asn     Thr Cys Gln Thr Leu Glu Phe Leu Ile
        805                     810                 815

aaa aac aaa atg aat ata ttt tat att ttc cta ttt ctt tta tca ttt      2688
Lys Asn Lys Met Asn Ile Phe Tyr Ile Phe Leu Phe Leu Leu Ser Phe
        820                 825                 830

gtg cag gga tca tta aat tgt aca tta aga gat tca caa caa aag tct      2736
Val Gln Gly Ser Leu Asn Cys Thr Leu Arg Asp Ser Gln Gln Lys Ser
    835                 840                 845

tta gta atg tca ggt cca tat gaa tta aaa gca tcc ctt gat aaa agg      2784
Leu Val Met Ser Gly Pro Tyr Glu Leu Lys Ala Ser Leu Asp Lys Arg
850                 855                 860                 865

gaa gcc gaa gcc gaa gct ccc ggg act c                                2812
Glu Ala Glu Ala Glu Ala Pro Gly Thr
                870

<210>  35
<211>  2881
<212>  DNA
<213>  Artificial Sequence

<220>
<223>  Kjeldsen synthetic and spacer

<220>
<221>  exon
<222>  (1)..(2881)

<400>  35
gag tcc cgg gca aca acc agg aac atc aac acc aga agt cca tcc aaa        48
Glu Ser Arg Ala Thr Thr Arg Asn Ile Asn Thr Arg Ser Pro Ser Lys
1               5                   10                  15

gtt aac aac cta taa atg tac taa gag tgg agg gtg tgt agc gca gga        96
Val Asn Asn Leu     Met Tyr     Glu Trp Arg Val Cys Ser Ala Gly
            20                      25                      30

cac aag tgt ggt ctt aga ctg gaa tta tcg ttg gat gca tga tgc caa       144
His Lys Cys Gly Leu Arg Leu Glu Leu Ser Leu Asp Ala     Cys Gln
                35                  40                      45

tta taa ttc ctg tac tgt taa cgg cgg tgt taa cac tac gtt atg ccc       192
Leu     Phe Leu Tyr Cys     Arg Arg Cys     His Tyr Val Met Pro
                50                          55

cga tga agc gac ttg tgg taa gaa ttg ttt tat tga agg ggt tga cta       240
Arg     Ser Asp Leu Trp     Glu Leu Phe Tyr     Arg Gly     Leu
        60                      65                          70

cgc cgc tag tgg tgt tac gac gag tgg gtc atc ctt gac gat gaa tca       288
Arg Arg     Trp Cys Tyr Asp Glu Trp Val Ile Leu Asp Asp Glu Ser
                75                  80                  85

ata cat gcc ttc ttc tag tgg tgg gta ttc ctc tgt gtc tcc aag gct       336
Ile His Ala Phe Phe     Trp Trp Val Phe Leu Cys Val Ser Lys Ala
                90                  95                  100

gta ttt att gga ttc cga tgg gga ata tgt tat gtt aaa att aaa tgg 384
Val Phe Ile Gly Phe Arg Trp Gly Ile Cys Tyr Val Lys Ile Lys Trp
                105                 110                 115

gca aga act gag ttt tga tgt gga tct atc tgc att acc ttg tgg aga 432
Ala Arg Thr Glu Phe Cys Gly Ser Ile Cys Ile Thr Leu Trp Arg
            120                 125                 130

aaa tgg tag tct tta ttt atc aca aat gga cga aaa cgg cgg agc caa 480
Lys Trp Ser Leu Phe Ile Thr Asn Gly Arg Lys Arg Arg Ser Gln
            135                 140                 145

tca gta caa tac agc tgg tgc taa tta tgg ttc agg cta ttg tga tgc 528
Ser Val Gln Tyr Ser Trp Cys Leu Trp Phe Arg Leu Leu Cys
            150                 155                 160

tca atg tcc agt gca gac ttg gag gaa tgg cac ctt aaa cac atc aca 576
Ser Met Ser Ser Ala Asp Leu Glu Glu Trp His Leu Lys His Ile Thr
                165                 170                 175

tca agg att ttg ctg taa cga aat gga cat att aga agg taa ttc aag 624
Ser Arg Ile Leu Leu Arg Asn Gly His Ile Arg Arg Phe Lys
            180                 185                 190

agc taa tgc act aac tcc gca ctc ttg tac tgc gag tcc cgg gca atc 672
Ser Cys Thr Asn Ser Ala Leu Leu Tyr Cys Glu Ser Arg Ala Ile
                195                 200                 205

cgc ttg tac cct aca atc cga aac tca ccc acc att gac ctg gca aaa 720
Arg Leu Tyr Pro Thr Ile Arg Asn Ser Pro Thr Ile Asp Leu Ala Lys
                210                 215                 220

gtg ttc tag cgg tgg aac ttg tac tca aca aac tgg ttc tgt tgt tat 768
Val Phe Arg Trp Asn Leu Tyr Ser Thr Asn Trp Phe Cys Cys Tyr
            225                 230                 235

cga cgc taa ctg gag atg gac aca cgc cac taa ctc ttc tac caa ctg 816
Arg Arg Leu Glu Met Asp Thr Arg His Leu Phe Tyr Gln Leu
            240                 245                 250

tta cga cgg taa cac ttg gtc ttc cac ttt atg tcc aga taa cga aac 864
Leu Arg Arg His Leu Val Phe His Phe Met Ser Arg Arg Asn
                255                 260

ttg tgc taa gaa ttg ctg ttt gga cgg tgc cgc cta cgc ttc tac cta 912
Leu Cys Glu Leu Leu Phe Gly Arg Cys Arg Leu Arg Phe Tyr Leu
265                 270                 275

cgg tgt tac cac ctc cgg taa ctc ctt gtc tat tgg ttt cgt cac tca 960
Arg Cys Tyr His Leu Arg Leu Leu Val Tyr Trp Phe Arg His Ser
280                 285                 290

atc cgc tca aaa gaa cgt tgg tgc tag att gta ctt gat ggc ttc tga 1008
Ile Arg Ser Lys Glu Arg Trp Cys Ile Val Leu Asp Gly Phe
295                 300                 305

cac tac tta tca aga att tac ttt gtt ggg taa cga att ttc ttt cga 1056
His Tyr Leu Ser Arg Ile Tyr Phe Val Gly Arg Ile Phe Phe Arg
    310                 315                 320

tgt tga cgt ttc cca att gcc atg tgg ctt gaa cgg tgc ttt gta ctt 1104
Cys Arg Phe Pro Ile Ala Met Trp Leu Glu Arg Cys Phe Val Leu
    325                 330                 335

tgt ctc tat gga tgc tga cgg tgg tgt ttc taa gta ccc aac taa cac     1152
Cys Leu Tyr Gly Cys     Arg Trp Cys Phe     Val Pro Asn     His
    340                 345                 350

tgc cgg tgc taa gta cgg tac tgg tta ctg tga ttc tca atg tcc acg     1200
Cys Arg Cys     Val Arg Tyr Trp Leu Leu     Phe Ser Met Ser Thr
                355                 360                     365

tga ctt gaa gtt cat taa cgg tca agc caa cgt cga agg ttg gga acc     1248
    Leu Glu Val His     Arg Ser Ser Gln Arg Arg Arg Leu Gly Thr
                        370                 375

atc ctc caa caa cgc taa cac cgg tat cgg tgg tca cgg ttc ctg ttg     1296
Ile Leu Gln Gln Arg     His Arg Tyr Arg Trp Ser Arg Phe Leu Leu
380                     385                 390

ttc cga aat gga cat ctg gga agc taa cag tat ttc tga agc ttt gac     1344
Phe Arg Asn Gly His Leu Gly Ser     Gln Tyr Phe     Ser Phe Asp
395                 400                 405

acc aca ccc atg cac cac tgt cgg tca aga aat ttg tga agg tga tgg     1392
Thr Thr Pro Met His His Cys Arg Ser Arg Asn Leu     Arg     Trp
    410                 415                 420

atg tgg tgg aac cta ctc tga taa cag ata cgg tgg tac ttg tga ccc     1440
Met Trp Trp Asn Leu Leu         Gln Ile Arg Trp Tyr Leu     Pro
        425                         430                     435

aga cgg ttg tga ctg gaa ccc ata cag att ggg taa cac ttc ttt cta     1488
Arg Arg Leu     Leu Glu Pro Ile Gln Ile Gly     His Phe Phe Leu
                440                 445

tgg tcc agg ttc ttc ttt cac ctt gga tac cac caa gaa gtt gac tgt     1536
Trp Ser Arg Phe Phe Phe His Leu Gly Tyr His Gln Glu Val Asp Cys
450                 455                 460                 465

tgt tac cca att cga aac ttc tgg tgc tat caa cag ata cta cgt tca     1584
Cys Tyr Pro Ile Arg Asn Phe Trp Cys Tyr Gln Gln Ile Leu Arg Ser
                470                 475                 480

aaa cgg tgt cac ctt cca aca acc aaa cgc tga att ggg ttc tta ctc     1632
Lys Arg Cys His Leu Pro Thr Thr Lys Arg     Ile Gly Phe Leu Leu
            485                 490                 495

tgg taa tga att gaa cga cga cta ctg tac cgc tga aga agc tga att     1680
Trp         Ile Glu Arg Arg Leu Leu Tyr Arg     Arg Ser     Ile
                500                 505

tgg tgg ttc ctc ttt ctc cga caa ggg tgg ttt gac cca att caa gaa     1728
Trp Trp Phe Leu Phe Leu Arg Gln Gly Trp Phe Asp Pro Ile Gln Glu
    510                 515                 520

ggc tac ctc cgg tgg tat ggt ttt ggt tat gtc ctt gtg gga tga tta     1776
Gly Tyr Leu Arg Trp Tyr Gly Phe Gly Tyr Val Leu Val Gly     Leu
525                 530                 535

cta cgc aaa cat gtt atg gtt aga cag tac tta ccc aac taa cga aac     1824
Leu Arg Lys His Val Met Val Arg Gln Tyr Leu Pro Asn     Arg Asn
540                 545                 550

cga gtc ccg ggg tcc cat tag aag aaa gac aag cct gct cct ctg ttt     1872
Arg Val Pro Gly Ser His     Lys Lys Asp Lys Pro Ala Pro Leu Phe
555                 560                 565

ggg gtc aat gtg gtg gtc aaa act ggt ctg gtc caa ctt gtt gtg ctt     1920

Gly Val Asn Val Val Val Lys Thr Gly Leu Val Gln Leu Val Val Leu
570                 575                 580                 585

ccg gtt cta cct gtg ttt act cca acg act act att ccc aat gtt tgc      1968
Pro Val Leu Pro Val Phe Thr Pro Thr Thr Thr Ile Pro Asn Val Cys
                590                 595                 600

cag gtg ctg ctt cct ctt cct ctt caa cta gag ctg ctt cta caa ctt      2016
Gln Val Leu Leu Pro Leu Pro Leu Gln Leu Glu Leu Leu Leu Gln Leu
            605                 610                 615

cta ggg tct ccc caa cca ctt cca gat cct ctt ctg cta ctc cac cac      2064
Leu Gly Ser Pro Gln Pro Leu Pro Asp Pro Leu Leu Leu Leu His His
        620                 625                 630

cag gtt cta cta cca cta gag ttc cac cag tcg gtt ccg gta ctg cta      2112
Gln Val Leu Leu Pro Leu Glu Phe His Gln Ser Val Pro Val Leu Leu
    635                 640                 645

ctt act ctg gta acc ctt tcg tcg gtg tta ctc cat ggg cta acg ctt      2160
Leu Thr Leu Val Thr Leu Ser Ser Val Leu Leu His Gly Leu Thr Leu
650                 655                 660                 665

act acg ctt ctg aag ttt ctt ctt tgg cta tcc cat ctt tga ctg gtg      2208
Thr Thr Leu Leu Lys Phe Leu Leu Trp Leu Ser His Leu     Leu Val
                670                 675                 680

cta tgg cta ccg ctg ctg ctg ctg tcg cca aag ttc cat cct tca tgt      2256
Leu Trp Leu Pro Leu Leu Leu Leu Ser Pro Lys Phe His Pro Ser Cys
                685                 690                 695

ggt tgg aca cct tgg aca aaa ctc cat taa tgg aac aaa cct tgg cag      2304
Gly Trp Thr Pro Trp Thr Lys Leu His     Trp Asn Lys Pro Trp Gln
            700                 705                 710

aca taa gga ctg cta aca aga acg gcg gta act acg ctg gtc aat ttg      2352
Thr     Gly Leu Leu Thr Arg Thr Ala Val Thr Thr Leu Val Asn Leu
                715                 720                 725

ttg tgt acg act tgc cag aca gag act gtg ctg ctt tgg ctt cca acg      2400
Leu Cys Thr Thr Cys Gln Thr Glu Thr Val Leu Leu Trp Leu Pro Thr
            730                 735                 740

gtg aat act cca tcg ctg acg gtg gtg tcg cca agt aca aga act aca      2448
Val Asn Thr Pro Ser Leu Thr Val Val Ser Pro Ser Thr Arg Thr Thr
        745                 750                 755

ttg ata cca tta gac aaa tcg ttg tcg aat act ctg aca tca gaa cct      2496
Leu Ile Pro Leu Asp Lys Ser Leu Ser Asn Thr Leu Thr Ser Glu Pro
    760                 765                 770

tgt tag tca tcg aac cag att ctt tag cca att tag tca cca act tgg      2544
Cys     Ser Ser Asn Gln Ile Leu     Pro Ile     Ser Pro Thr Trp
775                 780                 785

gta ctc caa agt gtg cta acg ctc aat ctg cct act tag aat gta tca      2592
Val Leu Gln Ser Val Leu Thr Leu Asn Leu Pro Thr     Asn Val Ser
        790                 795                 800

att atg cag tta ccc aat tga act tgc caa acg ttg gaa ttc tta att      2640
Ile Met Gln Leu Pro Asn     Thr Cys Gln Thr Leu Glu Phe Leu Ile
        805                 810                 815

aaa aac aaa atg aag ttg aag act gtt agg tca gcc gtt ttg agt agt      2688
Lys Asn Lys Met Lys Leu Lys Thr Val Arg Ser Ala Val Leu Ser Ser

820                 825                 830

tta ttt gcc tct caa gtc ttg ggt caa cca att gat gat acg gaa agt     2736
Leu Phe Ala Ser Gln Val Leu Gly Gln Pro Ile Asp Asp Thr Glu Ser
    835                 840                 845

aat acc act tca gtt aat ttg atg gct gac gat acg gaa tct agg ttt     2784
Asn Thr Thr Ser Val Asn Leu Met Ala Asp Asp Thr Glu Ser Arg Phe
850                 855                 860                 865

gca acg aac acg acc tta gct cta gat gtt gtg aat tta att tca atg     2832
Ala Thr Asn Thr Thr Leu Ala Leu Asp Val Val Asn Leu Ile Ser Met
                870                 875                 880

gct aaa aga gaa gag gct gaa gct gag gcg gag ccc aag ccc ggg act c   2881
Ala Lys Arg Glu Glu Ala Glu Ala Glu Ala Glu Pro Lys Pro Gly Thr
            885                 890                 895

<210>  36
<211>  915
<212>  PRT
<213>  Saccharomycopsis fibuliga

<400>  36

Met Val Ser Phe Thr Ser Leu Leu Ala Gly Val Ala Ala Ile Ser Gly
1               5                   10                  15

Val Leu Ala Ala Pro Ala Ala Glu Val Glu Pro Val Ala Val Glu Lys
            20                  25                  30

Arg Glu Ala Glu Ala Glu Ala Met Leu Met Ile Val Gln Leu Leu Val
        35                  40                  45

Phe Ala Leu Gly Leu Ala Val Ala Val Pro Ile Gln Asn Tyr Thr Gln
    50                  55                  60

Ser Pro Ser Gln Arg Asp Glu Ser Ser Gln Trp Val Ser Pro His Tyr
65                  70                  75                  80

Tyr Pro Thr Pro Gln Gly Gly Arg Leu Gln Asp Val Trp Gln Glu Ala
                85                  90                  95

Tyr Ala Arg Ala Lys Ala Ile Val Gly Gln Met Thr Ile Val Glu Lys
            100                 105                 110

Val Asn Leu Thr Thr Gly Thr Gly Trp Gln Leu Asp Pro Cys Val Gly
        115                 120                 125

Asn Thr Gly Ser Val Pro Arg Phe Gly Ile Pro Asn Leu Cys Leu Gln
    130                 135                 140

Asp Gly Pro Leu Gly Val Arg Phe Ala Asp Phe Val Thr Gly Tyr Pro
145                 150                 155                 160

Ser Gly Leu Ala Thr Gly Ala Thr Phe Asn Lys Asp Leu Phe Leu Gln
                165                 170                 175

Arg Gly Gln Ala Leu Gly His Glu Phe Asn Ser Lys Gly Val His Ile
            180                 185                 190

Ala Leu Gly Pro Ala Val Gly Pro Leu Gly Val Lys Ala Arg Gly Gly
        195                 200                 205

Arg Asn Phe Glu Ala Phe Gly Ser Asp Pro Tyr Leu Gln Gly Thr Ala
    210                 215                 220

Ala Ala Ala Thr Ile Lys Gly Leu Gln Glu Asn Asn Val Met Ala Cys
225                 230                 235                 240

Val Lys His Phe Ile Gly Asn Glu Gln Glu Lys Tyr Arg Gln Pro Asp
                245                 250                 255

Asp Ile Asn Pro Ala Thr Asn Gln Thr Thr Lys Glu Ala Ile Ser Ala
            260                 265                 270

Asn Ile Pro Asp Arg Ala Met His Ala Leu Tyr Leu Trp Pro Phe Ala
        275                 280                 285

Asp Ser Val Arg Ala Gly Val Gly Ser Val Met Cys Ser Tyr Asn Arg
    290                 295                 300

Val Asn Asn Thr Tyr Ala Cys Glu Asn Ser Tyr Met Met Asn His Leu
305                 310                 315                 320

Leu Lys Glu Glu Leu Gly Phe Gln Gly Phe Val Val Ser Asp Trp Gly
                325                 330                 335

Ala Gln Leu Ser Gly Val Tyr Ser Ala Ile Ser Gly Leu Asp Met Ser
            340                 345                 350

Met Pro Gly Glu Val Tyr Gly Gly Trp Asn Thr Gly Thr Ser Phe Trp
        355                 360                 365

Gly Gln Asn Leu Thr Lys Ala Ile Tyr Asn Glu Thr Val Pro Ile Glu
    370                 375                 380

Arg Leu Asp Asp Met Ala Thr Arg Ile Leu Ala Ala Leu Tyr Ala Thr
385                 390                 395                 400

Asn Ser Phe Pro Thr Glu Asp His Leu Pro Asn Phe Ser Ser Trp Thr
                405                 410                 415

Thr Lys Glu Tyr Gly Asn Lys Tyr Tyr Ala Asp Asn Thr Thr Glu Ile
            420                 425                 430

Val Lys Val Asn Tyr Asn Val Asp Pro Ser Asn Asp Phe Thr Glu Asp
        435                 440                 445

Thr Ala Leu Lys Val Ala Glu Glu Ser Ile Val Leu Leu Lys Asn Glu
    450                 455                 460

Asn Asn Thr Leu Pro Ile Ser Pro Glu Lys Ala Lys Arg Leu Leu Leu
465                 470                 475                 480

Ser Gly Ile Ala Ala Gly Pro Asp Pro Ile Gly Tyr Gln Cys Glu Asp
                485                 490                 495

Gln Ser Cys Thr Asn Gly Ala Leu Phe Gln Gly Trp Gly Ser Gly Ser
            500                 505                 510

Val Gly Ser Pro Lys Tyr Gln Val Thr Pro Phe Glu Glu Ile Ser Tyr
        515                 520                 525

Leu Ala Arg Lys Asn Lys Met Gln Phe Asp Tyr Ile Arg Glu Ser Tyr
    530                 535                 540

Asp Leu Ala Gln Val Thr Lys Val Ala Ser Asp Ala His Leu Ser Ile
545                 550                 555                 560

Val Val Val Ser Ala Ala Ser Gly Glu Gly Tyr Ile Thr Val Asp Gly
                565                 570                 575

Asn Gln Gly Asp Arg Lys Asn Leu Thr Leu Trp Asn Asn Gly Asp Lys
            580                 585                 590

Leu Ile Glu Thr Val Ala Glu Asn Cys Ala Asn Thr Val Val Val Val
        595                 600                 605

Thr Ser Thr Gly Gln Ile Asn Phe Glu Gly Phe Ala Asp His Pro Asn
    610                 615                 620

Val Thr Ala Ile Val Trp Ala Gly Pro Leu Gly Asp Arg Ser Gly Thr
625                 630                 635                 640

Ala Ile Ala Asn Ile Leu Phe Gly Lys Ala Asn Pro Ser Gly His Leu
                645                 650                 655

Pro Phe Thr Ile Ala Lys Thr Asp Asp Asp Tyr Ile Pro Ile Glu Thr
            660                 665                 670

Tyr Ser Pro Ser Ser Gly Glu Pro Glu Asp Asn His Leu Val Glu Asn

675                 680                 685

Asp Leu Leu Val Asp Tyr Arg Tyr Phe Glu Glu Lys Asn Ile Glu Pro
    690                 695                 700

Arg Tyr Ala Phe Gly Tyr Gly Leu Ser Tyr Asn Glu Tyr Glu Val Ser
705                 710                 715                 720

Asn Ala Lys Val Ser Ala Ala Lys Lys Val Asp Glu Glu Leu Pro Glu
                725                 730                 735

Pro Ala Thr Tyr Leu Ser Glu Phe Ser Tyr Gln Asn Ala Lys Asp Ser
            740                 745                 750

Lys Asn Pro Ser Asp Ala Phe Ala Pro Ala Asp Leu Asn Arg Val Asn
        755                 760                 765

Glu Tyr Leu Tyr Pro Tyr Leu Asp Ser Asn Val Thr Leu Lys Asp Gly
    770                 775                 780

Asn Tyr Glu Tyr Pro Asp Gly Tyr Ser Thr Glu Gln Arg Thr Thr Pro
785                 790                 795                 800

Asn Gln Pro Gly Gly Gly Leu Gly Gly Asn Asp Ala Leu Trp Glu Val
                805                 810                 815

Ala Tyr Asn Ser Thr Asp Lys Phe Val Pro Gln Gly Asn Ser Thr Asp
            820                 825                 830

Lys Phe Val Pro Gln Leu Tyr Leu Lys His Pro Glu Asp Gly Lys Phe
        835                 840                 845

Glu Thr Pro Ile Gln Leu Arg Gly Phe Glu Lys Val Glu Leu Ser Pro
    850                 855                 860

Gly Glu Lys Lys Thr Val Asp Leu Arg Leu Leu Arg Arg Asp Leu Ser
865                 870                 875                 880

Val Trp Asp Thr Thr Arg Gln Ser Trp Ile Val Glu Ser Gly Thr Tyr
                885                 890                 895

Glu Ala Leu Ile Gly Val Ala Val Asn Asp Ile Lys Thr Ser Val Leu
            900                 905                 910

Phe Thr Ile
        915

<210>  37
<211>  488

<212>  PRT
<213>  Trichoderma reesei

<400>  37

Met Asn Ile Phe Tyr Ile Phe Leu Phe Leu Leu Ser Phe Val Gln Gly
1               5                   10                  15

Ser Leu Asn Cys Thr Leu Arg Asp Ser Gln Gln Lys Ser Leu Val Met
            20                  25                  30

Ser Gly Pro Tyr Glu Leu Lys Ala Ser Leu Asp Lys Arg Glu Ala Glu
        35                  40                  45

Ala Glu Ala Gln Gln Pro Gly Thr Ser Thr Pro Glu Val His Pro Lys
    50                  55                  60

Leu Thr Thr Tyr Lys Cys Thr Lys Ser Gly Gly Cys Val Ala Gln Asp
65                  70                  75                  80

Thr Ser Val Val Leu Asp Trp Asn Tyr Arg Trp Met His Asp Ala Asn
                85                  90                  95

Tyr Asn Ser Cys Thr Val Asn Gly Gly Val Asn Thr Thr Leu Cys Pro
            100                 105                 110

Asp Glu Ala Thr Cys Gly Lys Asn Cys Phe Ile Glu Gly Val Asp Tyr
        115                 120                 125

Ala Ala Ser Gly Val Thr Thr Ser Gly Ser Ser Leu Thr Met Asn Gln
    130                 135                 140

Tyr Met Pro Ser Ser Ser Gly Gly Tyr Ser Ser Val Ser Pro Arg Leu
145                 150                 155                 160

Tyr Leu Leu Asp Ser Asp Gly Glu Tyr Val Met Leu Lys Leu Asn Gly
                165                 170                 175

Gln Glu Leu Ser Phe Asp Val Asp Leu Ser Ala Leu Pro Cys Gly Glu
            180                 185                 190

Asn Gly Ser Leu Tyr Leu Ser Gln Met Asp Glu Asn Gly Gly Ala Asn
        195                 200                 205

Gln Tyr Asn Thr Ala Gly Ala Asn Tyr Gly Ser Gly Tyr Cys Asp Ala
    210                 215                 220

Gln Cys Pro Val Gln Thr Trp Arg Asn Gly Thr Leu Asn Thr Ser His
225                 230                 235                 240

Gln Gly Phe Cys Cys Asn Glu Met Asp Ile Leu Glu Gly Asn Ser Arg
                245                 250                 255

Ala Asn Ala Leu Thr Pro His Ser Cys Thr Ala Thr Ala Cys Asp Ser
            260                 265                 270

Ala Gly Cys Gly Phe Asn Pro Tyr Gly Ser Gly Tyr Lys Ser Tyr Tyr
        275                 280                 285

Gly Pro Gly Asp Thr Val Asp Thr Ser Lys Thr Phe Thr Ile Ile Thr
    290                 295                 300

Gln Phe Asn Thr Asp Asn Gly Ser Pro Ser Gly Asn Leu Val Ser Ile
305                 310                 315                 320

Thr Arg Lys Tyr Gln Gln Asn Gly Val Asp Ile Pro Ser Ala Gln Pro
                325                 330                 335

Gly Gly Asp Thr Ile Ser Ser Cys Pro Ser Ala Ser Ala Tyr Gly Gly
            340                 345                 350

Leu Ala Thr Met Gly Lys Ala Leu Ser Ser Gly Met Val Leu Val Phe
        355                 360                 365

Ser Ile Trp Asn Asp Asn Ser Gln Tyr Met Asn Trp Leu Asp Ser Gly
    370                 375                 380

Asn Ala Gly Pro Cys Ser Ser Thr Glu Gly Asn Pro Ser Asn Ile Leu
385                 390                 395                 400

Ala Asn Asn Pro Asn Thr His Val Val Phe Ser Asn Ile Arg Trp Gly
                405                 410                 415

Asp Ile Gly Ser Thr Thr Asn Ser Thr Ala Pro Pro Pro Pro Pro Ala
            420                 425                 430

Ser Ser Thr Thr Phe Ser Thr Thr Arg Arg Ser Ser Thr Thr Ser Ser
        435                 440                 445

Ser Pro Ser Cys Thr Gln Thr His Trp Gly Gln Cys Gly Gly Ile Gly
    450                 455                 460

Tyr Ser Gly Cys Lys Thr Cys Thr Ser Gly Thr Thr Cys Gln Tyr Ser
465                 470                 475                 480

Asn Asp Tyr Tyr Ser Gln Cys Leu
                485

<210>  38

<211>  547
<212>  PRT
<213>  Trichoderma reesei

<400>  38

Met Asn Ile Phe Tyr Ile Phe Leu Phe Leu Leu Ser Phe Val Gln Gly
1               5                   10                  15

Ser Leu Asn Cys Thr Leu Arg Asp Ser Gln Gln Lys Ser Leu Val Met
            20                  25                  30

Ser Gly Pro Tyr Glu Leu Lys Ala Ser Leu Asp Lys Arg Glu Ala Glu
        35                  40                  45

Ala Glu Ala Gln Ser Ala Cys Thr Leu Gln Ser Glu Thr His Pro Pro
    50                  55                  60

Leu Thr Trp Gln Lys Cys Ser Ser Gly Gly Thr Cys Thr Gln Gln Thr
65                  70                  75                  80

Gly Ser Val Val Ile Asp Ala Asn Trp Arg Trp Thr His Ala Thr Asn
                85                  90                  95

Ser Ser Thr Asn Cys Tyr Asp Gly Asn Thr Trp Ser Ser Thr Leu Cys
            100                 105                 110

Pro Asp Asn Glu Thr Cys Ala Lys Asn Cys Cys Leu Asp Gly Ala Ala
        115                 120                 125

Tyr Ala Ser Thr Tyr Gly Val Thr Thr Ser Gly Asn Ser Leu Ser Ile
    130                 135                 140

Gly Phe Val Thr Gln Ser Ala Gln Lys Asn Val Gly Ala Arg Leu Tyr
145                 150                 155                 160

Leu Met Ala Ser Asp Thr Thr Tyr Gln Glu Phe Thr Leu Leu Gly Asn
                165                 170                 175

Glu Phe Ser Phe Asp Val Asp Val Ser Gln Leu Pro Cys Gly Leu Asn
            180                 185                 190

Gly Ala Leu Tyr Phe Val Ser Met Asp Ala Asp Gly Gly Val Ser Lys
        195                 200                 205

Tyr Pro Thr Asn Thr Ala Gly Ala Lys Tyr Gly Thr Gly Tyr Cys Asp
    210                 215                 220

Ser Gln Cys Pro Arg Asp Leu Lys Phe Ile Asn Gly Gln Ala Asn Val
225                 230                 235                 240

Glu Gly Trp Glu Pro Ser Ser Asn Asn Ala Asn Thr Gly Ile Gly Gly
                245                 250                 255

His Gly Ser Cys Cys Ser Glu Met Asp Ile Trp Glu Ala Asn Ser Ile
            260                 265                 270

Ser Glu Ala Leu Thr Pro His Pro Cys Thr Thr Val Gly Gln Glu Ile
        275                 280                 285

Cys Glu Gly Asp Gly Cys Gly Gly Thr Tyr Ser Asp Asn Arg Tyr Gly
    290                 295                 300

Gly Thr Cys Asp Pro Asp Gly Cys Asp Trp Asn Pro Tyr Arg Leu Gly
305                 310                 315                 320

Asn Thr Ser Phe Tyr Gly Pro Gly Ser Ser Phe Thr Leu Asp Thr Thr
                325                 330                 335

Lys Lys Leu Thr Val Val Thr Gln Phe Glu Thr Ser Gly Ala Ile Asn
            340                 345                 350

Arg Tyr Tyr Val Gln Asn Gly Val Thr Phe Gln Gln Pro Asn Ala Glu
        355                 360                 365

Leu Gly Ser Tyr Ser Gly Asn Glu Leu Asn Asp Asp Tyr Cys Thr Ala
    370                 375                 380

Glu Glu Ala Glu Phe Gly Gly Ser Ser Phe Ser Asp Lys Gly Gly Leu
385                 390                 395                 400

Thr Gln Phe Lys Lys Ala Thr Ser Gly Gly Met Val Leu Val Met Ser
                405                 410                 415

Leu Trp Asp Asp Tyr Tyr Ala Asn Met Leu Trp Leu Asp Ser Thr Tyr
            420                 425                 430

Pro Thr Asn Glu Thr Ser Ser Thr Pro Gly Ala Val Arg Gly Ser Cys
        435                 440                 445

Ser Thr Ser Ser Gly Val Pro Ala Gln Val Glu Ser Gln Ser Pro Asn
    450                 455                 460

Ala Lys Val Thr Phe Ser Asn Ile Lys Phe Gly Pro Ile Gly Ser Thr
465                 470                 475                 480

Gly Asn Pro Ser Gly Gly Asn Pro Pro Gly Gly Asn Arg Gly Thr Thr
                485                 490                 495

Thr Thr Arg Arg Pro Ala Thr Thr Thr Gly Ser Ser Pro Gly Pro Thr
            500                 505                 510

Gln Ser His Tyr Gly Gln Cys Gly Gly Ile Gly Tyr Ser Gly Pro Thr
        515                 520                 525

Val Cys Ala Ser Gly Thr Thr Cys Gln Val Leu Asn Pro Tyr Tyr Ser
    530                 535                 540

Gln Cys Leu
545

<210>  39
<211>  492
<212>  PRT
<213>  Trichoderma reesei

<400>  39

Met Val Ser Phe Thr Ser Leu Leu Ala Gly Val Ala Ala Ile Ser Gly
1               5                   10                  15

Val Leu Ala Ala Pro Ala Ala Glu Val Glu Pro Val Ala Val Glu Lys
            20                  25                  30

Arg Glu Ala Glu Ala Glu Ala Val Pro Leu Glu Glu Arg Gln Ala Cys
        35                  40                  45

Ser Ser Val Trp Gly Gln Cys Gly Gly Gln Asn Trp Ser Gly Pro Thr
    50                  55                  60

Cys Cys Ala Ser Gly Ser Thr Cys Val Tyr Ser Asn Asp Tyr Tyr Ser
65                  70                  75                  80

Gln Cys Leu Pro Gly Ala Ala Ser Ser Ser Ser Ser Thr Arg Ala Ala
                85                  90                  95

Ser Thr Thr Ser Arg Val Ser Pro Thr Thr Ser Arg Ser Ser Ser Ala
            100                 105                 110

Thr Pro Pro Pro Gly Ser Thr Thr Thr Arg Val Pro Pro Val Gly Ser
        115                 120                 125

Gly Thr Ala Thr Tyr Ser Gly Asn Pro Phe Val Gly Val Thr Pro Trp
    130                 135                 140

Ala Asn Ala Tyr Tyr Ala Ser Glu Val Ser Ser Leu Ala Ile Pro Ser
145                 150                 155                 160

Leu Thr Gly Ala Met Ala Thr Ala Ala Ala Ala Val Ala Lys Val Pro
                165                 170                 175

Ser Phe Met Trp Leu Asp Thr Leu Asp Lys Thr Pro Leu Met Glu Gln
        180                 185                 190

Thr Leu Ala Asp Ile Arg Thr Ala Asn Lys Asn Gly Gly Asn Tyr Ala
    195                 200                 205

Gly Gln Phe Val Val Tyr Asp Leu Pro Asp Arg Asp Cys Ala Ala Leu
210                 215                 220

Ala Ser Asn Gly Glu Tyr Ser Ile Ala Asp Gly Gly Val Ala Lys Tyr
225                 230                 235                 240

Lys Asn Tyr Ile Asp Thr Ile Arg Gln Ile Val Val Glu Tyr Ser Asp
                245                 250                 255

Ile Arg Thr Leu Leu Val Ile Glu Pro Asp Ser Leu Ala Asn Leu Val
            260                 265                 270

Thr Asn Leu Gly Thr Pro Lys Cys Ala Asn Ala Gln Ser Ala Tyr Leu
        275                 280                 285

Glu Cys Ile Asn Tyr Ala Val Thr Gln Leu Asn Leu Pro Asn Val Ala
    290                 295                 300

Met Tyr Leu Asp Ala Gly His Ala Gly Trp Leu Gly Trp Pro Ala Asn
305                 310                 315                 320

Gln Asp Pro Ala Ala Gln Leu Phe Ala Asn Val Tyr Lys Asn Ala Ser
                325                 330                 335

Ser Pro Arg Ala Leu Arg Gly Leu Ala Thr Asn Val Ala Asn Tyr Asn
            340                 345                 350

Gly Trp Asn Ile Thr Ser Pro Pro Ser Tyr Thr Gln Gly Asn Ala Val
        355                 360                 365

Tyr Asn Glu Lys Leu Tyr Ile His Ala Ile Gly Pro Leu Leu Ala Asn
    370                 375                 380

His Gly Trp Ser Asn Ala Phe Phe Ile Thr Asp Gln Gly Arg Ser Gly
385                 390                 395                 400

Lys Gln Pro Thr Gly Gln Gln Gln Trp Gly Asp Trp Cys Asn Val Ile
                405                 410                 415

Gly Thr Gly Phe Gly Ile Arg Pro Ser Ala Asn Thr Gly Asp Ser Leu
            420                 425                 430

Leu Asp Ser Phe Val Trp Val Lys Pro Gly Gly Glu Cys Asp Gly Thr
        435                 440                 445

Ser Asp Ser Ser Ala Pro Arg Phe Asp Ser His Cys Ala Leu Pro Asp
    450                 455                 460

Ala Leu Gln Pro Ala Pro Gln Ala Gly Ala Trp Phe Gln Ala Tyr Phe
465                 470                 475                 480

Val Gln Leu Leu Thr Asn Ala Asn Pro Ser Phe Leu
                485                 490

<210>  40
<211>  879
<212>  PRT
<213>  Clostridium thermocellum

<400>  40

Met Arg Leu Val Asn Ser Leu Gly Arg Arg Lys Ile Leu Leu Ile Leu
1               5                   10                  15

Ala Val Ile Val Ala Phe Ser Thr Val Leu Leu Phe Ala Lys Leu Trp
            20                  25                  30

Gly Arg Lys Thr Ser Ser Thr Leu Asp Glu Val Gly Ser Lys Thr His
        35                  40                  45

Gly Asp Leu Thr Ala Glu Asn Lys Asn Gly Gly Tyr Leu Pro Glu Glu
    50                  55                  60

Glu Ile Pro Asp Gln Pro Pro Ala Thr Gly Ala Phe Asn Tyr Gly Glu
65                  70                  75                  80

Ala Leu Gln Lys Ala Ile Phe Phe Tyr Glu Cys Gln Arg Ser Gly Lys
                85                  90                  95

Leu Asp Pro Ser Thr Leu Arg Leu Asn Trp Arg Gly Asp Ser Gly Leu
            100                 105                 110

Asp Asp Gly Lys Asp Ala Gly Ile Asp Leu Thr Gly Gly Trp Tyr Asp
        115                 120                 125

Ala Gly Asp His Val Lys Phe Asn Leu Pro Met Ser Tyr Ser Ala Ala
    130                 135                 140

Met Leu Gly Trp Ala Val Tyr Glu Tyr Glu Asp Ala Phe Lys Gln Ser
145                 150                 155                 160

Gly Gln Tyr Asn His Ile Leu Asn Asn Ile Lys Trp Ala Cys Asp Tyr

165                 170                 175

Phe Ile Lys Cys His Pro Glu Lys Asp Val Tyr Tyr Tyr Gln Val Gly
            180                 185                 190

Asp Gly His Ala Asp His Ala Trp Trp Gly Pro Ala Glu Val Met Pro
        195                 200                 205

Met Glu Arg Pro Ser Tyr Lys Val Asp Arg Ser Ser Pro Gly Ser Thr
    210                 215                 220

Val Val Ala Glu Thr Ser Ala Ala Leu Ala Ile Ala Ser Ile Ile Phe
225                 230                 235                 240

Lys Lys Val Asp Gly Glu Tyr Ser Lys Glu Cys Leu Lys His Ala Lys
                245                 250                 255

Glu Leu Phe Glu Phe Ala Asp Thr Thr Lys Ser Asp Asp Gly Tyr Thr
            260                 265                 270

Ala Ala Asn Gly Phe Tyr Asn Ser Trp Ser Gly Phe Tyr Asp Glu Leu
        275                 280                 285

Ser Trp Ala Ala Val Trp Leu Tyr Leu Ala Thr Asn Asp Ser Ser Tyr
    290                 295                 300

Leu Asp Lys Ala Glu Ser Tyr Ser Asp Lys Trp Gly Tyr Glu Pro Gln
305                 310                 315                 320

Thr Asn Ile Pro Lys Tyr Lys Trp Ala Gln Cys Trp Asp Asp Val Thr
                325                 330                 335

Tyr Gly Thr Tyr Leu Leu Leu Ala Arg Ile Lys Asn Asp Asn Gly Lys
            340                 345                 350

Tyr Lys Glu Ala Ile Glu Arg His Leu Asp Trp Trp Thr Thr Gly Tyr
        355                 360                 365

Asn Gly Glu Arg Ile Thr Tyr Thr Pro Lys Gly Leu Ala Trp Leu Asp
    370                 375                 380

Gln Trp Gly Ser Leu Arg Tyr Ala Thr Thr Thr Ala Phe Leu Ala Cys
385                 390                 395                 400

Val Tyr Ser Asp Trp Glu Asn Gly Asp Lys Glu Lys Ala Lys Thr Tyr
                405                 410                 415

Leu Glu Phe Ala Arg Ser Gln Ala Asp Tyr Ala Leu Gly Ser Thr Gly
            420                 425                 430

Arg Ser Phe Val Val Gly Phe Gly Glu Asn Pro Pro Lys Arg Pro His
        435                 440                 445

His Arg Thr Ala His Gly Ser Trp Ala Asp Ser Gln Met Glu Pro Pro
    450                 455                 460

Glu His Arg His Val Leu Tyr Gly Ala Leu Val Gly Gly Pro Asp Ser
465                 470                 475                 480

Thr Asp Asn Tyr Thr Asp Asp Ile Ser Asn Tyr Thr Cys Asn Glu Val
                485                 490                 495

Ala Cys Asp Tyr Asn Ala Gly Phe Val Gly Leu Leu Ala Lys Met Tyr
            500                 505                 510

Lys Leu Tyr Gly Gly Ser Pro Asp Pro Lys Phe Asn Gly Ile Glu Glu
        515                 520                 525

Val Pro Glu Asp Glu Ile Phe Val Glu Ala Gly Val Asn Ala Ser Gly
    530                 535                 540

Asn Asn Phe Ile Glu Ile Lys Ala Ile Val Asn Asn Lys Ser Gly Trp
545                 550                 555                 560

Pro Ala Arg Val Cys Glu Asn Leu Ser Phe Arg Tyr Phe Ile Asn Ile
                565                 570                 575

Glu Glu Ile Val Asn Ala Gly Lys Ser Ala Ser Asp Leu Gln Val Ser
            580                 585                 590

Ser Ser Tyr Asn Gln Gly Ala Lys Leu Ser Asp Val Lys His Tyr Lys
        595                 600                 605

Asp Asn Ile Tyr Tyr Val Glu Val Asp Leu Ser Gly Thr Lys Ile Tyr
    610                 615                 620

Pro Gly Gly Gln Ser Ala Tyr Lys Lys Glu Val Gln Phe Arg Ile Ser
625                 630                 635                 640

Ala Pro Glu Gly Thr Val Phe Asn Pro Glu Asn Asp Tyr Ser Tyr Gln
                645                 650                 655

Gly Leu Ser Ala Gly Thr Val Val Lys Ser Glu Tyr Ile Pro Val Tyr
            660                 665                 670

Asp Ala Gly Val Leu Val Phe Gly Arg Glu Pro Gly Ser Ala Ser Lys
        675                 680                 685

Ser Thr Ser Lys Asp Asn Gly Leu Ser Lys Ala Thr Pro Thr Val Lys
    690                 695                 700

Thr Glu Ser Gln Pro Thr Ala Lys His Thr Gln Asn Pro Ala Ser Asp
705                 710                 715                 720

Phe Lys Thr Pro Ala Asn Gln Asn Ser Val Lys Lys Asp Gln Gly Ile
                725                 730                 735

Lys Gly Glu Val Val Leu Gln Tyr Ala Asn Gly Asn Ala Gly Ala Thr
            740                 745                 750

Ser Asn Ser Ile Asn Pro Arg Phe Lys Ile Ile Asn Asn Gly Thr Lys
        755                 760                 765

Ala Ile Asn Leu Ser Asp Val Lys Ile Arg Tyr Tyr Tyr Thr Lys Glu
    770                 775                 780

Gly Gly Ala Ser Gln Asn Phe Trp Cys Asp Trp Ser Ser Ala Gly Asn
785                 790                 795                 800

Ser Asn Val Thr Gly Asn Phe Phe Asn Leu Ser Ser Pro Lys Glu Gly
                805                 810                 815

Ala Asp Thr Cys Leu Glu Val Gly Phe Gly Ser Gly Ala Gly Thr Leu
            820                 825                 830

Asp Pro Gly Gly Ser Val Glu Val Gln Ile Arg Phe Ser Lys Glu Asp
        835                 840                 845

Trp Ser Asn Tyr Asn Gln Ser Asn Asp Tyr Ser Phe Lys Gln Ala Cys
    850                 855                 860

Leu Arg Gln Arg Thr Leu Ile Tyr Leu Tyr Ala Thr Trp Leu Arg
865                 870                 875

<210>  41
<211>  625
<212>  PRT
<213>  Arabidopsis thaliana

<400>  41

Met Gly Ser Arg Thr Thr Ile Ser Ile Leu Val Val Leu Leu Leu Gly
1               5                   10                  15

Leu Val Gln Leu Ala Ile Ser Gly His Asp Tyr Lys Gln Ala Leu Ser
            20                  25                  30

Lys Ser Ile Leu Phe Phe Glu Ala Gln Arg Ser Gly His Leu Pro Pro

35                  40                  45

Asn Gln Arg Val Ser Trp Arg Ser His Ser Gly Leu Tyr Asp Gly Lys
    50                  55                  60

Ser Ser Gly Val Asp Leu Val Gly Gly Tyr Tyr Asp Ala Gly Asp Asn
65                  70                  75                  80

Val Lys Phe Gly Leu Pro Met Ala Phe Thr Val Thr Thr Met Cys Trp
                85                  90                  95

Ser Ile Ile Glu Tyr Gly Gly Gln Leu Glu Ser Asn Gly Glu Leu Gly
            100                 105                 110

His Ala Ile Asp Ala Val Lys Trp Gly Thr Asp Tyr Phe Ile Lys Ala
        115                 120                 125

His Pro Glu Pro Asn Val Leu Tyr Gly Glu Val Gly Asp Gly Lys Ser
    130                 135                 140

Asp His Tyr Cys Trp Gln Arg Pro Glu Glu Met Thr Thr Asp Arg Arg
145                 150                 155                 160

Ala Tyr Lys Ile Asp Arg Asn Asn Pro Gly Ser Asp Leu Ala Gly Glu
                165                 170                 175

Thr Ala Ala Ala Met Ala Ala Ala Ser Ile Val Phe Arg Arg Ser Asp
            180                 185                 190

Pro Ser Tyr Ser Ala Glu Leu Leu Arg His Ala His Gln Leu Phe Glu
        195                 200                 205

Phe Ala Asp Lys Tyr Arg Gly Lys Tyr Asp Ser Ser Ile Thr Val Ala
    210                 215                 220

Gln Lys Tyr Tyr Arg Ser Val Ser Gly Tyr Asn Asp Glu Leu Leu Trp
225                 230                 235                 240

Ala Ala Ala Trp Leu Tyr Gln Ala Thr Asn Asp Lys Tyr Tyr Leu Asp
                245                 250                 255

Tyr Leu Gly Lys Asn Gly Asp Ser Met Gly Gly Thr Gly Trp Ser Met
            260                 265                 270

Thr Glu Phe Gly Trp Asp Val Lys Tyr Ala Gly Val Gln Thr Leu Val
        275                 280                 285

Ala Lys Val Leu Met Gln Gly Lys Gly Gly Glu His Thr Ala Val Phe
    290                 295                 300

Glu Arg Tyr Gln Gln Lys Ala Glu Gln Phe Met Cys Ser Leu Leu Gly
305                 310                 315                 320

Lys Ser Thr Lys Asn Ile Lys Lys Thr Pro Gly Gly Leu Ile Phe Arg
                325                 330                 335

Gln Ser Trp Asn Asn Met Gln Phe Val Thr Ser Ala Ser Phe Leu Ala
            340                 345                 350

Thr Val Tyr Ser Asp Tyr Leu Ser Tyr Ser Lys Arg Asp Leu Leu Cys
        355                 360                 365

Ser Gln Gly Asn Ile Ser Pro Ser Gln Leu Leu Glu Phe Ser Lys Ser
    370                 375                 380

Gln Val Asp Tyr Ile Leu Gly Asp Asn Pro Arg Ala Thr Ser Tyr Met
385                 390                 395                 400

Val Gly Tyr Gly Glu Asn Tyr Pro Arg Gln Val His His Arg Gly Ser
                405                 410                 415

Ser Ile Val Ser Phe Asn Val Asp Gln Lys Phe Val Thr Cys Arg Gly
            420                 425                 430

Gly Tyr Ala Thr Trp Phe Ser Arg Lys Gly Ser Asp Pro Asn Val Leu
        435                 440                 445

Thr Gly Ala Leu Val Gly Gly Pro Asp Ala Tyr Asp Asn Phe Ala Asp
    450                 455                 460

Gln Arg Asp Asn Tyr Glu Gln Thr Glu Pro Ala Thr Tyr Asn Asn Ala
465                 470                 475                 480

Pro Leu Leu Gly Val Leu Ala Arg Leu Ile Ser Gly Ser Thr Gly Phe
                485                 490                 495

Asp Gln Leu Leu Pro Gly Val Ser Pro Thr Pro Ser Pro Val Ile Ile
            500                 505                 510

Lys Pro Ala Pro Val Pro Gln Arg Lys Pro Thr Lys Pro Pro Ala Ser
        515                 520                 525

Ser Pro Ser Pro Ile Thr Ile Ser Gln Lys Met Thr Asn Ser Trp Lys
    530                 535                 540

Asn Glu Gly Lys Val Tyr Tyr Arg Tyr Ser Thr Ile Leu Thr Asn Arg
545                 550                 555                 560

Ser Thr Lys Thr Leu Lys Ile Leu Lys Ile Ser Ile Thr Lys Leu Tyr
                565                 570                 575

Gly Pro Ile Trp Gly Val Thr Lys Thr Gly Asn Ser Phe Ser Phe Pro
            580                 585                 590

Ser Trp Met Gln Ser Leu Pro Ser Gly Lys Ser Met Glu Phe Val Tyr
        595                 600                 605

Ile His Ser Ala Ser Pro Ala Asp Val Leu Val Ser Asn Tyr Ser Leu
    610                 615                 620

Glu
625

<210>  42
<211>  237
<212>  PRT
<213>  Aspergillus aculeauts

<400>  42

Met Lys Ala Phe His Leu Leu Ala Ala Leu Ala Gly Ala Ala Val Ala
1               5                   10                  15

Gln Gln Ala Gln Leu Cys Asp Gln Tyr Ala Thr Tyr Thr Gly Gly Val
            20                  25                  30

Tyr Thr Ile Asn Asn Asn Leu Trp Gly Lys Asp Ala Gly Ser Gly Ser
        35                  40                  45

Gln Cys Thr Thr Val Asn Ser Ala Ser Ser Ala Gly Thr Ser Trp Ser
    50                  55                  60

Thr Lys Trp Asn Trp Ser Gly Gly Glu Asn Ser Val Lys Ser Tyr Ala
65                  70                  75                  80

Asn Ser Gly Leu Thr Phe Asn Lys Lys Leu Val Ser Gln Ile Ser Gln
                85                  90                  95

Ile Pro Thr Thr Ala Arg Trp Ser Tyr Asp Asn Thr Gly Ile Arg Ala
            100                 105                 110

Asp Val Ala Tyr Asp Leu Phe Thr Ala Ala Asp Ile Asn His Val Thr
        115                 120                 125

Trp Ser Gly Asp Tyr Glu Leu Met Ile Trp Leu Ala Arg Tyr Gly Gly
    130                 135                 140

Val Gln Pro Ile Gly Ser Gln Ile Ala Thr Ala Thr Val Asp Gly Gln

145                 150                 155                 160

Thr Trp Glu Leu Trp Tyr Gly Ala Asn Gly Ser Gln Lys Thr Tyr Ser
                165                 170                 175

Phe Val Ala Pro Thr Pro Ile Thr Ser Phe Gln Gly Asp Val Asn Asp
            180                 185                 190

Phe Phe Lys Tyr Leu Thr Gln Asn His Gly Phe Pro Ala Ser Ser Gln
        195                 200                 205

Tyr Leu Ile Thr Leu Gln Phe Gly Thr Glu Pro Phe Thr Gly Gly Pro
    210                 215                 220

Ala Thr Leu Ser Val Ser Asn Trp Ser Ala Ser Val Gln
225                 230                 235

<210>  43
<211>  895
<212>  PRT
<213>  Clostridium thermocellum

<400>  43

Met Asn Phe Arg Arg Met Leu Cys Ala Ala Ile Val Leu Thr Ile Val
1               5                   10                  15

Leu Ser Ile Met Leu Pro Ser Thr Val Phe Ala Leu Glu Asp Lys Ser
            20                  25                  30

Pro Lys Leu Pro Asp Tyr Lys Asn Asp Leu Leu Tyr Glu Arg Thr Phe
        35                  40                  45

Asp Glu Gly Leu Cys Phe Pro Trp His Thr Cys Glu Asp Ser Gly Gly
    50                  55                  60

Lys Cys Asp Phe Ala Val Val Asp Val Pro Gly Glu Pro Gly Asn Lys
65                  70                  75                  80

Ala Phe Arg Leu Thr Val Ile Asp Lys Gly Gln Asn Lys Trp Ser Val
                85                  90                  95

Gln Met Arg His Arg Gly Ile Thr Leu Glu Gln Gly His Thr Tyr Thr
            100                 105                 110

Val Arg Phe Thr Ile Trp Ser Asp Lys Ser Cys Arg Val Tyr Ala Lys
        115                 120                 125

Ile Gly Gln Met Gly Glu Pro Tyr Thr Glu Tyr Trp Asn Asn Asn Trp
    130                 135                 140

Asn Pro Phe Asn Leu Thr Pro Gly Gln Lys Leu Thr Val Glu Gln Asn
145                 150                 155                 160

Phe Thr Met Asn Tyr Pro Thr Asp Asp Thr Cys Glu Phe Thr Phe His
                165                 170                 175

Leu Gly Gly Glu Leu Ala Ala Gly Thr Pro Tyr Tyr Val Tyr Leu Asp
            180                 185                 190

Asp Val Ser Leu Tyr Asp Pro Arg Phe Val Lys Pro Val Glu Tyr Val
        195                 200                 205

Leu Pro Gln Pro Asp Val Arg Val Asn Gln Val Gly Tyr Leu Pro Phe
    210                 215                 220

Ala Lys Lys Tyr Ala Thr Val Val Ser Ser Ser Thr Ser Pro Leu Lys
225                 230                 235                 240

Trp Gln Leu Leu Asn Ser Ala Asn Gln Val Val Leu Glu Gly Asn Thr
                245                 250                 255

Ile Pro Lys Gly Leu Asp Lys Asp Ser Gln Asp Tyr Val His Trp Ile
            260                 265                 270

Asp Phe Ser Asn Phe Lys Thr Glu Gly Lys Gly Tyr Tyr Phe Lys Leu
        275                 280                 285

Pro Thr Val Asn Ser Asp Thr Asn Tyr Ser His Pro Phe Asp Ile Ser
    290                 295                 300

Ala Asp Ile Tyr Ser Lys Met Lys Phe Asp Ala Leu Ala Phe Phe Tyr
305                 310                 315                 320

His Lys Arg Ser Gly Ile Pro Ile Glu Met Pro Tyr Ala Gly Gly Glu
                325                 330                 335

Gln Trp Thr Arg Pro Ala Gly His Ile Gly Val Ala Pro Asn Lys Gly
            340                 345                 350

Asp Thr Asn Val Pro Thr Trp Pro Gln Asp Asp Glu Tyr Ala Gly Arg
        355                 360                 365

Pro Gln Lys Tyr Tyr Thr Lys Asp Val Thr Gly Gly Trp Tyr Asp Ala
    370                 375                 380

Gly Asp His Gly Lys Tyr Val Val Asn Gly Gly Ile Ala Val Trp Thr
385                 390                 395                 400

Leu Met Asn Met Tyr Glu Arg Ala Lys Ile Arg Gly Ile Ala Asn Gln
                405                 410                 415

Gly Ala Tyr Lys Asp Gly Gly Met Asn Ile Pro Glu Arg Asn Asn Gly
            420                 425                 430

Tyr Pro Asp Ile Leu Asp Glu Ala Arg Trp Glu Ile Glu Phe Phe Lys
        435                 440                 445

Lys Met Gln Val Thr Glu Lys Glu Asp Pro Ser Ile Ala Gly Met Val
    450                 455                 460

His His Lys Ile His Asp Phe Arg Trp Thr Ala Leu Gly Met Leu Pro
465                 470                 475                 480

His Glu Asp Pro Gln Pro Arg Tyr Leu Arg Pro Val Ser Thr Ala Ala
                485                 490                 495

Thr Leu Asn Phe Ala Ala Thr Leu Ala Gln Ser Ala Arg Leu Trp Lys
            500                 505                 510

Asp Tyr Asp Pro Thr Phe Ala Ala Asp Cys Leu Glu Lys Ala Glu Ile
        515                 520                 525

Ala Trp Gln Ala Ala Leu Lys His Pro Asp Ile Tyr Ala Glu Tyr Thr
    530                 535                 540

Pro Gly Ser Gly Gly Pro Gly Gly Gly Pro Tyr Asn Asp Asp Tyr Val
545                 550                 555                 560

Gly Asp Glu Phe Tyr Trp Ala Ala Cys Glu Leu Tyr Val Thr Thr Gly
                565                 570                 575

Lys Asp Glu Tyr Lys Asn Tyr Leu Met Asn Ser Pro His Tyr Leu Glu
            580                 585                 590

Met Pro Ala Lys Met Gly Glu Asn Gly Gly Ala Asn Gly Glu Asp Asn
        595                 600                 605

Gly Leu Trp Gly Cys Phe Thr Trp Gly Thr Thr Gln Gly Leu Gly Thr
    610                 615                 620

Ile Thr Leu Ala Leu Val Glu Asn Gly Leu Pro Ser Ala Asp Ile Gln
625                 630                 635                 640

Lys Ala Arg Asn Asn Ile Ala Lys Ala Ala Asp Lys Trp Leu Glu Asn
                645                 650                 655

Ile Glu Glu Gln Gly Tyr Arg Leu Pro Ile Lys Gln Ala Glu Asp Glu

660                 665                 670

Arg Gly Gly Tyr Pro Trp Gly Ser Asn Ser Phe Ile Leu Asn Gln Met
        675                 680                 685

Ile Val Met Gly Tyr Ala Tyr Asp Phe Thr Gly Asn Ser Lys Tyr Leu
    690                 695                 700

Asp Gly Met Gln Asp Gly Met Ser Tyr Leu Leu Gly Arg Asn Gly Leu
705                 710                 715                 720

Asp Gln Ser Tyr Val Thr Gly Tyr Gly Glu Arg Pro Leu Gln Asn Pro
                725                 730                 735

His Asp Arg Phe Trp Thr Pro Gln Thr Ser Lys Lys Phe Pro Ala Pro
            740                 745                 750

Pro Pro Gly Ile Ile Ala Gly Gly Pro Asn Ser Arg Phe Glu Asp Pro
        755                 760                 765

Thr Ile Thr Ala Ala Val Lys Lys Asp Thr Pro Pro Gln Lys Cys Tyr
    770                 775                 780

Ile Asp His Thr Asp Ser Trp Ser Thr Asn Glu Ile Thr Ile Asn Trp
785                 790                 795                 800

Asn Ala Pro Phe Ala Trp Val Thr Ala Tyr Leu Asp Glu Ile Asp Leu
                805                 810                 815

Ile Thr Pro Pro Gly Gly Val Asp Pro Glu Glu Pro Glu Val Ile Tyr
            820                 825                 830

Gly Asp Cys Asn Gly Asp Gly Lys Val Asn Ser Thr Asp Ala Val Ala
        835                 840                 845

Leu Lys Arg Tyr Ile Leu Arg Ser Gly Ile Ser Ile Asn Thr Asp Asn
    850                 855                 860

Ala Asp Val Asn Ala Asp Gly Arg Val Asn Ser Thr Asp Leu Ala Ile
865                 870                 875                 880

Leu Lys Arg Tyr Ile Leu Lys Glu Ile Asp Val Leu Pro His Lys
                885                 890                 895

<210>  44
<211>  438
<212>  PRT
<213>  Agaricus bisporus

<400>  44

Met Phe Lys Phe Ala Ala Leu Leu Ala Leu Ala Ser Leu Val Pro Gly
1               5                   10                  15

Phe Val Gln Ala Gln Ser Pro Val Trp Gly Gln Cys Gly Gly Asn Gly
            20                  25                  30

Trp Thr Gly Pro Thr Thr Cys Ala Ser Gly Ser Thr Cys Val Lys Gln
        35                  40                  45

Asn Asp Phe Tyr Ser Gln Cys Leu Pro Asn Asn Gln Ala Pro Pro Ser
    50                  55                  60

Thr Thr Thr Gln Pro Gly Thr Thr Pro Pro Ala Thr Thr Thr Ser Gly
65                  70                  75                  80

Gly Thr Gly Pro Thr Ser Gly Ala Gly Asn Pro Tyr Thr Gly Lys Thr
                85                  90                  95

Val Trp Leu Ser Pro Phe Tyr Ala Asp Glu Val Ala Gln Ala Ala Ala
            100                 105                 110

Asp Ile Ser Asn Pro Ser Leu Ala Thr Lys Ala Ala Ser Val Ala Lys
        115                 120                 125

Ile Pro Thr Phe Val Trp Phe Asp Thr Val Ala Lys Val Pro Asp Leu
    130                 135                 140

Gly Gly Tyr Leu Ala Asp Ala Arg Ser Lys Asn Gln Leu Val Gln Ile
145                 150                 155                 160

Val Val Tyr Asp Leu Pro Asp Arg Asp Cys Ala Ala Leu Ala Ser Asn
                165                 170                 175

Gly Glu Phe Ser Leu Ala Asn Asp Gly Leu Asn Lys Tyr Lys Asn Tyr
            180                 185                 190

Val Asp Gln Ile Ala Ala Gln Ile Lys Gln Phe Pro Asp Val Ser Val
        195                 200                 205

Val Ala Val Ile Glu Pro Asp Ser Leu Ala Asn Leu Val Thr Asn Leu
    210                 215                 220

Asn Val Gln Lys Cys Ala Asn Ala Gln Ser Ala Tyr Lys Glu Gly Val
225                 230                 235                 240

Ile Tyr Ala Val Gln Lys Leu Asn Ala Val Gly Val Thr Met Tyr Ile
                245                 250                 255

Asp Ala Gly His Ala Gly Trp Leu Gly Trp Pro Ala Asn Leu Ser Pro
            260                 265                 270

Ala Ala Gln Leu Phe Ala Gln Ile Tyr Arg Asp Ala Gly Ser Pro Arg
        275                 280                 285

Asn Leu Arg Gly Ile Ala Thr Asn Val Ala Asn Phe Asn Ala Leu Arg
    290                 295                 300

Ala Ser Ser Pro Asp Pro Ile Thr Gln Gly Asn Ser Asn Tyr Asp Glu
305                 310                 315                 320

Ile His Tyr Ile Glu Ala Leu Ala Pro Met Leu Ser Asn Ala Gly Phe
                325                 330                 335

Pro Ala His Phe Ile Val Asp Gln Gly Arg Ser Gly Val Gln Asn Ile
            340                 345                 350

Arg Asp Gln Trp Gly Asp Trp Cys Asn Val Lys Gly Ala Gly Phe Gly
        355                 360                 365

Gln Arg Pro Thr Thr Asn Thr Gly Ser Ser Leu Ile Asp Ala Ile Val
    370                 375                 380

Trp Val Lys Pro Gly Gly Glu Cys Asp Gly Thr Ser Asp Asn Ser Ser
385                 390                 395                 400

Pro Arg Phe Asp Ser His Cys Ser Leu Ser Asp Ala His Gln Pro Ala
                405                 410                 415

Pro Glu Ala Gly Thr Trp Phe Gln Ala Tyr Phe Glu Thr Leu Val Ala
            420                 425                 430

Asn Ala Asn Pro Ala Leu
        435

<210> 45
<211> 516
<212> PRT
<213> Phanerochaete chrysosporium

<400> 45

Met Phe Arg Thr Ala Thr Leu Leu Ala Phe Thr Met Ala Ala Met Val
1               5                   10                  15

Phe Gly Gln Gln Val Gly Thr Asn Thr Ala Glu Asn His Arg Thr Leu
            20                  25                  30

Thr Ser Gln Lys Cys Thr Lys Ser Gly Gly Cys Ser Asn Leu Asn Thr
        35                  40                  45

Lys Ile Val Leu Asp Ala Asn Trp Arg Trp Leu His Ser Thr Ser Gly
    50                  55                  60

Tyr Thr Asn Cys Tyr Thr Gly Asn Gln Trp Asp Ala Thr Leu Cys Pro
65                  70                  75                  80

Asp Gly Lys Thr Cys Ala Ala Asn Cys Ala Leu Asp Gly Ala Asp Tyr
                85                  90                  95

Thr Gly Thr Tyr Gly Ile Thr Ala Ser Gly Ser Ser Leu Lys Leu Gln
            100                 105                 110

Phe Val Thr Gly Ser Asn Val Gly Ser Arg Val Tyr Leu Met Ala Asp
        115                 120                 125

Asp Thr His Tyr Gln Met Phe Gln Leu Leu Asn Gln Glu Phe Thr Phe
    130                 135                 140

Asp Val Asp Met Ser Asn Leu Pro Cys Gly Leu Asn Gly Ala Leu Tyr
145                 150                 155                 160

Leu Ser Ala Met Asp Ala Asp Gly Gly Met Ala Lys Tyr Pro Thr Asn
                165                 170                 175

Lys Ala Gly Ala Lys Tyr Gly Thr Gly Tyr Cys Asp Ser Gln Cys Pro
            180                 185                 190

Arg Asp Ile Lys Phe Ile Asn Gly Glu Ala Asn Val Glu Gly Trp Asn
        195                 200                 205

Ala Thr Ser Ala Asn Ala Gly Thr Gly Asn Tyr Gly Thr Cys Cys Thr
    210                 215                 220

Glu Met Asp Ile Trp Glu Ala Asn Asn Asp Ala Ala Ala Tyr Thr Pro
225                 230                 235                 240

His Pro Cys Thr Thr Asn Ala Gln Thr Arg Cys Ser Gly Ser Asp Cys
                245                 250                 255

Thr Arg Asp Thr Gly Leu Cys Asp Ala Asp Gly Cys Asp Phe Asn Ser
            260                 265                 270

Phe Arg Met Gly Asp Gln Thr Phe Leu Gly Lys Gly Leu Thr Val Asp
        275                 280                 285

Thr Ser Lys Pro Phe Thr Val Val Thr Gln Phe Ile Thr Asn Asp Gly
    290                 295                 300

Thr Ser Ala Gly Thr Leu Thr Glu Ile Arg Arg Leu Tyr Val Gln Asn
305                 310                 315                 320

Gly Lys Val Ile Gln Asn Ser Ser Val Lys Ile Pro Gly Ile Asp Pro
                325                 330                 335

Val Asn Ser Ile Thr Asp Asn Phe Cys Ser Gln Gln Lys Thr Ala Phe
            340                 345                 350

Gly Asp Thr Asn Tyr Phe Ala Gln His Gly Gly Leu Lys Gln Val Gly
        355                 360                 365

Glu Ala Leu Arg Thr Gly Met Val Leu Ala Leu Ser Ile Trp Asp Asp
    370                 375                 380

Tyr Ala Ala Asn Met Leu Trp Leu Asp Ser Asn Tyr Pro Thr Asn Lys
385                 390                 395                 400

Asp Pro Ser Thr Pro Gly Val Ala Arg Gly Thr Cys Ala Thr Thr Ser
                405                 410                 415

Gly Val Pro Ala Gln Ile Glu Ala Gln Ser Pro Asn Ala Tyr Val Val
            420                 425                 430

Phe Ser Asn Ile Lys Phe Gly Asp Leu Asn Thr Thr Tyr Thr Gly Thr
        435                 440                 445

Val Ser Ser Ser Ser Val Ser Ser Ser His Ser Ser Thr Ser Thr Ser
    450                 455                 460

Ser Ser His Ser Ser Ser Ser Thr Pro Pro Thr Gln Pro Thr Gly Val
465                 470                 475                 480

Thr Val Pro Gln Trp Gly Gln Cys Gly Gly Ile Gly Tyr Thr Gly Ser
                485                 490                 495

Thr Thr Cys Ala Ser Pro Tyr Thr Cys His Val Leu Asn Pro Tyr Tyr
            500                 505                 510

Ser Gln Cys Tyr
        515

<210>  46
<211>  444
<212>  PRT
<213>  Thermotoga neapolitana

<400>  46

Met Lys Lys Phe Pro Glu Gly Phe Leu Trp Gly Val Ala Thr Ala Ser

1               5                   10                  15

Tyr Gln Ile Glu Gly Ser Pro Leu Ala Asp Gly Ala Gly Met Ser Ile
            20                  25                  30

Trp His Thr Phe Ser His Thr Pro Gly Asn Val Lys Asn Gly Asp Thr
        35                  40                  45

Gly Asp Val Ala Cys Asp His Tyr Asn Arg Trp Lys Glu Asp Ile Glu
    50                  55                  60

Ile Ile Glu Lys Ile Gly Ala Lys Ala Tyr Arg Phe Ser Ile Ser Trp
65                  70                  75                  80

Pro Arg Ile Leu Pro Glu Gly Thr Gly Lys Val Asn Gln Lys Gly Leu
                85                  90                  95

Asp Phe Tyr Asn Arg Ile Ile Asp Thr Leu Leu Glu Lys Asn Ile Thr
            100                 105                 110

Pro Phe Ile Thr Ile Tyr His Trp Asp Leu Pro Phe Ser Leu Gln Leu
        115                 120                 125

Lys Gly Gly Trp Ala Asn Arg Asp Ile Ala Asp Trp Phe Ala Glu Tyr
    130                 135                 140

Ser Arg Val Leu Phe Glu Asn Phe Gly Asp Arg Val Lys His Trp Ile
145                 150                 155                 160

Thr Leu Asn Glu Pro Trp Val Val Ala Ile Val Gly His Leu Tyr Gly
                165                 170                 175

Val His Ala Pro Gly Met Lys Asp Ile Tyr Val Ala Phe His Thr Val
            180                 185                 190

His Asn Leu Leu Arg Ala His Ala Lys Ser Val Lys Val Phe Arg Glu
        195                 200                 205

Thr Val Lys Asp Gly Lys Ile Gly Ile Val Phe Asn Asn Gly Tyr Phe
    210                 215                 220

Glu Pro Ala Ser Glu Arg Glu Glu Asp Ile Arg Ala Ala Arg Phe Met
225                 230                 235                 240

His Gln Phe Asn Asn Tyr Pro Leu Phe Leu Asn Pro Ile Tyr Arg Gly
                245                 250                 255

Glu Tyr Pro Asp Leu Val Leu Glu Phe Ala Arg Glu Tyr Leu Pro Arg
            260                 265                 270

Asn Tyr Glu Asp Asp Met Glu Glu Ile Lys Gln Glu Ile Asp Phe Val
        275                 280                 285

Gly Leu Asn Tyr Tyr Ser Gly His Met Val Lys Tyr Asp Pro Asn Ser
    290                 295                 300

Pro Ala Arg Val Ser Phe Val Glu Arg Asn Leu Pro Lys Thr Ala Met
305                 310                 315                 320

Gly Trp Glu Ile Val Pro Glu Gly Ile Tyr Trp Ile Leu Lys Gly Val
                325                 330                 335

Lys Glu Glu Tyr Asn Pro Gln Glu Val Tyr Ile Thr Glu Asn Gly Ala
            340                 345                 350

Ala Phe Asp Asp Val Val Ser Glu Gly Gly Lys Val His Asp Gln Asn
        355                 360                 365

Arg Ile Asp Tyr Leu Arg Ala His Ile Glu Gln Val Trp Arg Ala Ile
    370                 375                 380

Gln Asp Gly Val Pro Leu Lys Gly Tyr Phe Val Trp Ser Leu Leu Asp
385                 390                 395                 400

Asn Phe Glu Trp Ala Glu Gly Tyr Ser Lys Arg Phe Gly Ile Val Tyr
                405                 410                 415

Val Asp Tyr Asn Thr Gln Lys Arg Ile Ile Lys Asp Ser Gly Tyr Trp
            420                 425                 430

Tyr Ser Asn Gly Ile Lys Asn Asn Gly Leu Thr Asp
        435                 440

<210>  47
<211>  455
<212>  PRT
<213>  Caldocellum saccharolyticum

<400>  47

Met Asp Met Ser Phe Pro Lys Gly Phe Leu Trp Gly Ala Ala Thr Ala
1               5                   10                  15

Ser Tyr Gln Ile Glu Gly Ala Trp Asn Glu Asp Gly Lys Gly Glu Ser
            20                  25                  30

Ile Trp Asp Arg Phe Thr His Gln Lys Arg Asn Ile Leu Tyr Gly His
        35                  40                  45

Asn Gly Asp Val Ala Cys Asp His Tyr His Arg Phe Glu Glu Asp Val
    50                  55                  60

Ser Leu Met Lys Glu Leu Gly Leu Lys Ala Tyr Arg Phe Ser Ile Ala
65                  70                  75                  80

Trp Thr Arg Ile Phe Pro Asp Gly Phe Gly Thr Val Asn Gln Lys Gly
                85                  90                  95

Leu Glu Phe Tyr Asp Arg Leu Ile Asn Lys Leu Val Glu Asn Gly Ile
            100                 105                 110

Glu Pro Val Val Thr Leu Tyr His Trp Asp Leu Pro Gln Lys Leu Gln
        115                 120                 125

Asp Ile Gly Gly Trp Ala Asn Pro Glu Ile Val Asn Tyr Tyr Phe Asp
    130                 135                 140

Tyr Ala Met Leu Val Ile Asn Arg Tyr Lys Asp Lys Val Lys Lys Trp
145                 150                 155                 160

Ile Thr Phe Asn Glu Pro Tyr Cys Ile Ala Phe Leu Gly Tyr Phe His
                165                 170                 175

Gly Ile His Ala Pro Gly Ile Lys Asp Phe Lys Val Ala Met Asp Val
            180                 185                 190

Val His Ser Leu Met Leu Ser His Phe Lys Val Val Lys Ala Val Lys
        195                 200                 205

Glu Asn Asn Ile Asp Val Glu Val Gly Ile Thr Leu Asn Leu Thr Pro
    210                 215                 220

Val Tyr Leu Gln Thr Glu Arg Leu Gly Tyr Lys Val Ser Glu Ile Glu
225                 230                 235                 240

Arg Glu Met Val Ser Leu Ser Ser Gln Leu Asp Asn Gln Leu Phe Leu
                245                 250                 255

Asp Pro Val Leu Lys Gly Ser Tyr Pro Gln Lys Leu Leu Asp Tyr Leu
            260                 265                 270

Val Gln Lys Asp Leu Leu Asp Ser Gln Lys Ala Leu Ser Met Gln Gln
        275                 280                 285

Glu Val Lys Glu Asn Phe Ile Phe Pro Asp Phe Leu Gly Ile Asn Tyr
    290                 295                 300

Tyr Thr Arg Ala Val Arg Leu Tyr Asp Glu Asn Ser Ser Trp Ile Phe

305                 310                 315                 320

Pro Ile Arg Trp Glu His Pro Ala Gly Glu Tyr Thr Glu Met Gly Trp
                325                 330                 335

Glu Val Phe Pro Gln Gly Leu Phe Asp Leu Leu Ile Trp Ile Lys Glu
            340                 345                 350

Ser Tyr Pro Gln Ile Pro Ile Tyr Ile Thr Glu Asn Gly Ala Ala Tyr
        355                 360                 365

Asn Asp Ile Val Thr Glu Asp Gly Lys Val His Asp Ser Lys Arg Ile
    370                 375                 380

Glu Tyr Leu Lys Gln His Phe Glu Ala Ala Arg Lys Ala Ile Glu Asn
385                 390                 395                 400

Gly Val Asp Leu Arg Gly Tyr Phe Val Trp Ser Leu Met Asp Asn Phe
                405                 410                 415

Glu Trp Ala Met Gly Tyr Thr Lys Arg Phe Gly Ile Ile Tyr Val Asp
            420                 425                 430

Tyr Glu Thr Gln Lys Arg Ile Lys Lys Asp Ser Phe Tyr Phe Tyr Gln
        435                 440                 445

Gln Tyr Ile Lys Glu Asn Ser
    450                 455